# EUROPEAN PATENT APPLICATION

(11) **EP 4 840 859 A1**
(43) Date of publication of application: **18.11.2026**
(21) Application number: 25738631.8
(22) Date of filing: 10.01.2025
(51) Int. Cl.: C07D 487/04, A61K 31/53, A61P 35/00

(54) **NOVEL PYRROLOTRIAZINE DERIVATIVE AND USE THEREOF**

(30) Priority: 11.01.2024 KR 20240004716
(71) Applicant: Pelemed Co., Ltd., Gwangju 61005 (KR)
(72) Inventor: PARK, Jin Hee, Seoul 05698 (KR); JANG, Soo Yeon, Seoul 06065 (KR); LEE, Jae Seon, Yangju-si, Gyeonggi-do 11479 (KR); PARK, Min Ji, Seoul 05537 (KR); KIM, Soo Jin, Yongin-si, Gyeonggi-do 16824 (KR); KO, Bong Ki, Seoul 05569 (KR)
(74) Representative: Meissner Bolte Partnerschaft mbB
(86) International application number: PCT/KR2025/000684
(87) International publication number: WO 2025/151010

(57) **Abstract**

The present invention relates to novel pyrrolotriazine derivatives and the uses thereof as inhibitors of the activities of YES1 kinase and Transcriptional Enhanced Associated Domain (TEAD). The compound of the present invention effectively inhibits the activity of YES1 kinase and TEAD, and thus may be useful for preventing or treating YES1 kinase- associated diseases and TEAD-associated diseases, particularly solid cancer.

## Description

### FIELD OF THE INVENTION

The present invention relates to a novel pyrrolotriazine derivative and the use thereof as an inhibitor of YES1 (YES proto-oncogene 1), a non-receptor protein kinase. More specifically, it relates to the use thereof as an anticancer efficacy substance based on inhibition of TEAD activity by a YES1 inhibition mechanism.

### BACKGROUND OF THE INVENTION

Protein kinase is an enzyme that catalyzes the phosphorylation of hydroxyl groups located on tyrosine, serine, and threonine residues of proteins, and plays an important role in growth factor signal transduction that induces cell growth, differentiation, and proliferation.

Among protein kinases, tyrosine protein kinases are known to be divided into 58 types of receptor tyrosine kinases (RTK) and 32 types of non-receptor tyrosine kinases (NRTK). Receptor tyrosine kinases have been considered as the main target for drug development, but non-receptor tyrosine kinases have recently been considered as effective target enzymes for cancer treatment.

The SRC family of kinases (SFK) is a family of non-receptor tyrosine kinases including nine members: SRC, YES, FYN, FGR, LCK, HCK, BLK, LYN, and FRK. Among them, YES1 regulates cancer signaling pathways, and is the only member of the SRC kinase family that shows gene amplification in primary tumors of untreated patients, and is known to be overexpressed in cancer thereby promoting cell proliferation, survival, and invasiveness. High-level expression of YES1 is associated with poor prognosis, and its role as a target and prognostic biomarker in cancer was studied (Garmendia et al., Mol Cancer Ther., 2022, 21, 1371-1380).

Recently, various reports have described the association between YES1 and tumorigenicity. For example, downregulation of YES1 by short hairpin RNA (shRNA) has been shown to significantly inhibit cell growth in several malignant tumors, including colorectal carcinoma, rhabdomyosarcoma, and basal-like breast cancer (Sancier et al., PloS One., 2011, 6, e17237; Yeung et al., Oncogene, 2013, 32, 5429-38; Bilal et al., Genes Cancer, 2010, 1, 1063-73). In addition, some miRNAs have been shown to regulate tumor progression through YES1 modulation (Fang et al., Mol Cancer, 2017, 16, 139).

In addition, overexpression of YES1 is known to promote tyrosine phosphorylation of YES-associated protein (YAP), a mechanism causing malignant tumor development, which leads to transcription factor activation through binding to the transcriptional enhanced associated domain (TEAD) following its translocation into the nucleus.

The YAP-TEAD complex induces the transcription of genes related to cell growth, such as connective tissue growth factor (CTGF), and is an important anticancer target that functions at the lowest level of the Hippo pathway. It is overexpressed in various cancers, including prostate, gastric, and rectal cancers. Recently, the Hippo pathway has become a subject of interest in relation to hyperproliferative disorders and diseases, particularly in cancer treatment (J. Med. Chem. 2018, 61, 12, 5057-5072).

### DISCLOSURE OF THE INVENTION

### PROBLEMS TO BE SOLVED

As a result of research efforts to derive novel compounds having inhibitory activity against YES1 kinase and TEAD, the inventors completed the present invention by identifying that novel pyrrolotriazine derivative compounds can effectively inhibit YES1 kinase and also have excellent inhibitory activity against TEAD.

An object of the present invention is to provide a novel pyrrolotriazine derivative compound having inhibitory activity against YES1 kinase and TEAD and a pharmaceutically acceptable salt thereof.

Another object of the present invention is to provide a pharmaceutical composition for preventing or treating YES1 kinase and TEAD-associated diseases, comprising the novel pyrrolotriazine derivative compound. The present invention also provides an use of the novel compound for preventing or treating YES1 kinase and TEAD- associated diseases, and a method for preventing or treating YES1 kinase and TEAD- associated diseases, comprising administering the novel compound to a subject in need thereof.

However, the objects of the present invention are not limited thereto, and other objects and advantages of the present invention which are not specifically described can be understood by the following description, and will be more clearly understood by the embodiments of the present invention. In addition, the objects and advantages of the present invention can be easily realized by the means and combinations thereof set forth in the claims

### MEANS FOR SOLVING THE PROBLEMS

According to one embodiment of the present invention, a compound represented by the following formula 1 or a pharmaceutically acceptable salt thereof is provided. 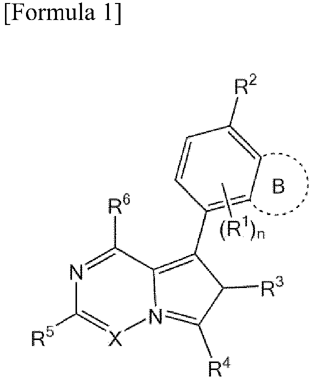 wherein in Formula 1,
X is C or N,
Ring B is absent or is (3-10 membered)cycloalkyl or (3-10 membered)heterocyclyl,
R¹ is each independently selected from the group consisting of hydrogen, halogen, C₁₋₁₀ alkyl, C₁₋₁₀ haloalkyl, C₁₋₁₀ alkoxy, C₁₋₆ haloalkoxy, (3-10 membered)cycloalkyl, C₁₋₁₀ alkylene-(3-10 membered)cycloalkyl, (3-10 membered)heterocyclyl, C₁₋₁₀ alkylene-(3-10 membered)heterocyclyl, (6-14 membered)aryl, C₁₋₁₀ alkylene-(6-14 membered)aryl, (5-12 membered)heteroaryl, C₁₋₁₀ alkylene-(5-12 membered)heteroaryl, NRₐR_{b}, O-C₁₋₁₀ alkylene-NRₐR_{b}, C₁₋₁₀ alkylene-O-NRₐR_{b}, C(O)-NRₐR_{b}, C₁₋₁₀ alkylene-C(O)-NRₐR_{b} and C(O)O-NRₐR_{b},
wherein (3-10 membered)cycloalkyl, (3-10 membered)heterocyclyl, (6-14 membered)aryl and (5-12 membered)heteroaryl are each independently unsubstituted or optionally substituted with a substituent selected from the group consisting of C₁₋₆ alkyl, C₁₋₆ alkoxy, halogen, C₁₋₆ haloalkyl, NH₂, NO₂, OH and CN,
n is an integer from 1 to 4,
R² is selected from the group consisting of hydrogen, halogen, C₁₋₅ alkyl, C₁₋₅ haloalkyl, C₁₋₅ alkoxy, C₁₋₅ haloalkoxy, C(O)-C₁₋₅ alkyl, C(O)-C₁₋₅ haloalkyl, C(O)O-C₁₋₅ alkyl, C(O)O-C₁₋₅ haloalkyl, CC(O)OC₁₋₅ alkyl, CC(O)O-C₁₋₅ haloalkyl, NR_{c}R_{d}, O-(6-14 membered) aryl, and O-(5-12 membered) heteroaryl,
wherein R_{c} and R_{d} are each independently optionally substituted with a substituent selected from the group consisting of hydrogen, C₁₋₅ alkyl, C₁₋₅ haloalkyl, C₁₋₅ alkoxy, C₁₋₅ haloalkoxy, C₁₋₃ aryl, C₁₋₃ heteroaryl, C(O)O-C₁₋₅alkyl, C(O)O-C₁₋₅haloalkyl, C(O)O-(3-10 membered) cycloalkyl, C(O)C₁₋₃ aryl, C(O)-(5-12 membered) heteroaryl, and C(O)NHC₁₋₅alkyl,
wherein (3-10 membered)cycloalkyl and (5-12 membered) heteroaryl are each independently unsubstituted or optionally substituted with a substituent selected from the group consisting of C₁₋₆alkyl, C₁₋₆alkoxy, halogen and C₁₋6haloalkyl,
R³ is selected from the group consisting of hydrogen, halogen, C₁₋₆alkyl, C₁₋₆haloalkyl, C₁₋₆alkoxy, NH₂, OH, CN, COOH, NO₂, C(O)C₁₋₆alkyl, C(O)OC₁₋₆alkyl and C₁₋₆alkylamino,
R⁴ is selected from the group consisting of (3-10 membered) cycloalkyl, (3-14 membered) heterocyclyl, (3-14 membered) N-heterocyclyl, (6-14 membered) aryl, (5-12 membered) heteroaryl, C₁₋₆ alkylene-(3-10 membered)cycloalkyl, C₁₋₆ alkylene-(3-14 membered) heterocyclyl, C₁₋₆ alkylene-(3-10 membered) N-heterocyclyl, C₁₋₆ alkylene-(6-14 membered) aryl, C₁₋₆ alkylene-(5-12 membered) heteroaryl, NRₒRₚ and 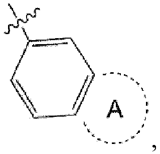
wherein the ring A is (3-10 membered) cycloalkyl or (3-14 membered) heterocyclyl,
wherein R⁴ is optionally substituted with one or more R⁷,
wherein R⁷ is each independently selected from the group consisting of halogen, C₁₋₆ alkyl, C₁₋₆ haloalkyl, C₁₋₆ alkoxy, C₁₋₆ haloalkoxy, C₁₋₁₀ alkylene-C₁₋₆ alkoxy, C₁₋₁₀ alkylene-C₁₋₆ haloalkoxy, (3-10 membered) cycloalkyl, C₁₋₁₀ alkylene-(3-10 membered) cycloalkyl, (6-14 membered) aryl, C₁₋₁₀ alkylene-(6-14 membered) aryl, (5-12 membered) heteroaryl, C₁₋₁₀ alkylene-(5-12 membered) heteroaryl, NRₑR_{f}, C₁₋₆ alkyl-NRₑR_{f}, C₁₋₁₀ alkylene-NRₑR_{f}, oxo, C(O)-NRₑR_{f}, C₁₋₁₀ alkylene-C(O)-NRₑR_{f}, C(O)O-NRₑR_{f}, CN, OH, NO₂, C(O)Rₑ, C(O)ORₑ, C₁₋₆ alkylene-C(O)ORₑ and C₁₋₆ alkylene-OH,
R⁵ is selected from the group consisting of hydrogen, halogen, C₁₋₆ alkyl, C₁₋₆ haloalkyl, C₁₋₆ alkoxy, NH₂, OH, CN, COOH, NO₂, C(O)C₁₋₆ alkyl, C(O)OC₁₋₆ alkyl and C₁₋₆ alkylamino,
R⁶ is NR_{g}Rₕ,
Rₐ, R_{b}, Rₑ, R_{f}, R_{g} and Rₕ are each independently selected from the group consisting of hydrogen, halogen, C₁₋₆ alkyl, C₁₋₆ haloalkyl, C(O)C₁₋₆ alkyl, C(O)C₁₋₆ haloalkyl, C(O)OC₁₋₆ alkyl and C(O)OC₁₋₆ haloalkyl,
Rₒ and Rₚ are each independently selected from the group consisting of hydrogen, halogen, C₁₋₆ alkyl, C₁₋₆ haloalkyl, (3-10 membered)cycloalkyl, C₁₋₁₀ alkylene-(3-10 membered)cycloalkyl, (3-10 membered) heterocyclyl and C₁₋₁₀ alkylene-(3-10 membered) heterocyclyl,
wherein (3-10 membered)cycloalkyl, C₁₋₁₀ alkylene-(3-10 membered)cycloalkyl, (3-10 membered)heterocyclyl or C₁₋₁₀ alkylene-(3-10 membered)heterocyclyl are optionally substituted with one or more R⁸, wherein R⁸ is each independently selected from the group consisting halogen, C₁₋₆ alkyl, C₁₋₆ haloalkyl, C₁₋₆ alkoxy, NR_{q}Rᵣ, C₁₋₁₀ alkylene-NR_{q}Rᵣ, oxo, C(O)-NR_{q}Rᵣ, C₁₋₁₀ alkylene-C(O)-NR_{q}Rᵣ, C(O)O-NR_{q}Rᵣ, CN, OH, NO₂, C(O)R_{q}, C(O)OR_{q}, C₁₋₆ alkylene-C(O)OR_{q} and C₁₋₆ alkylene-OH,
R_{q} and Rᵣ are each independently selected from the group consisting of hydrogen, halogen, C₁₋₆ alkyl and C₁₋₆ haloalkyl.

The compounds disclosed herein have high inhibitory activity against YES1 kinase and transcriptional enhanced associated domain.

The compounds and pharmaceutical compositions disclosed herein are useful for preventing or treating YES1 kinase associated diseases or transcriptional enhanced associated domain associated diseases by administering the compounds or compositions to a subject.

The compounds and pharmaceutical compositions disclosed herein are useful for preventing or treating solid tumors by administering the compounds or compositions to a subject.

### ADVANTAGE OF THE INVENTION

The pyrrolotriazine derivative compound of the present invention is a novel compound that has not been previously known, which effectively inhibits the activities of YES1 kinase and TEAD, and thus may be useful for the preventing or treating of YES1 kinase- and TEAD-associated diseases, especially solid cancer.

### DETAILED DESCRIPTION OF THE INVENTION

### Definition of Terms

Unless defined otherwise, all technical terms used herein have the same meaning as commonly understood by a person of ordinary skill in the art to which this invention belongs. Also, numerical values recited herein are intended to include the meaning of "about" unless explicitly stated otherwise.

Definitions of moieties and substituents as used herein are provided below. Unless otherwise specified, each moiety is defined as follows, and is used with the same meaning as commonly understood by those skilled in the art.

The substituents, as used herein, are intended to include both further substituted and unsubstituted instances.

As used herein, the terms "halo" or "halogen" refer to bromo, chloro, fluoro, or iodo.

As used herein, the term "alkyl" is a hydrocarbon having primary, secondary, tertiary, and/or quaternary carbon atoms, including saturated aliphatic groups, which may be straight-chain, branched, cyclic, or combinations thereof. For example, an alkyl group may have 1 to 8 carbon atoms (i.e., C₁-C₈ alkyl), 1 to 6 carbon atoms (i.e., C₁-C₆ alkyl), or 1 to 3 carbon atoms (i.e., C₁-C₃ alkyl). Examples of suitable alkyl groups may include methyl (Me, -CH₃), ethyl (Et, -C₂H₅), 1-propyl (n-Pr, -CH₂CH₂CH₃), 2-propyl (i-Pr, -CH(CH₃)₂), 1-butyl (n-Bu, - CH₂CH₂CH₂CH₃), 2-methyl-1-propyl (i-Bu, -CH₂CH(CH₃)₂), 2-butyl (s-Bu, - CH(CH₃)CH₂CH₃), 2-methyl-2-propyl (t-Bu, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-CH(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃), but is not limited thereto.

As used herein, the term "alkoxy" refers to an -O-(alkyl) group, including -OCH₃, - OCH₂CH₃, -O(CH₂)₂CH₃, -O(CH₂)₃CH₃, -O(CH₂)₄CH₃, -O(CH₂)₅CH₃, and the like, but is not limited thereto, wherein "alkyl" is as defined above.

As used herein, the terms "haloalkyl" and "haloalkoxy" refer to alkyl and alkoxy groups, in which one or more hydrogen atoms are each replaced by a halogen atom. For example, haloalkyl includes -CF₃, -CHF₂, -CH₂F, -CBr₃, -CHBr₂, -CH₂Br, -CCl₃, -CHCl₂, - CH₂Cl, -CI₃, -CHI₂, -CH₂I, -CH₂-CF₃, -CH₂-CHF₃, -CH₂-CH₂F, -CH₂-CBr₃, -CH₂-CHBr₂, - CH₂-CH₂Br, -CH₂-CCl₃, -CH₂-CHCl₂, -CH₂-CH₂Cl, -CH₂-CI₃, -CH₂-CHI₂, -CH₂-CH₂I and the like, but is not limited thereto. Herein, alkyl and halogen are each as defined above.

As used herein, the term "amine" or "amino" refers to a substituted or unsubstituted amine group represented by the form -NRR', wherein R and R' are each independently selected from hydrogen, alkyl, alkenyl, alkynyl, aryl, arylalkyl, cycloalkyl, heteroaryl, and heterocyclyl groups.

The term "amino" also includes a corresponding quaternary ammonium salt of any amino group, such as -[N(R1)(Rm)(Rn)]⁺. Examples of amino groups include aminoalkyl groups in which at least one of R1, Rm, or Rn is an alkyl group. In certain embodiments, R1, Rm, and Rn are selected from hydrogen and alkyl.

As used herein, the term "alkylamino" refers to a concept that includes both mono-alkylamino and di-alkylamino groups.

As used herein, the term "mono-alkylamino" refers to an -NH(alkyl) group, including -NHCH₃, -NHCH₂CH₃, -NH(CH₂)₂CH₃, -NH(CH₂)₃CH₃, -NH(CH₂)₄CH₃, -NH(CH₂)₅CH₃, and the like, wherein "alkyl" is as defined above.

As used herein, the term "di-alkylamino" refers to an -N(alkyl)(alkyl) group, including -N(CH₃)₂, -N(CH₂CH₃)₂, -N((CH₂)₂CH₃)₂, -N(CH₃)(CH₂CH₃), and the like, wherein each "alkyl" is independently as defined above.

As used herein, the term "cycloalkyl" refers to a monocyclic or polycyclic saturated ring having carbon and hydrogen atoms and no carbon-carbon multiple bonds. The cycloalkyl group may contain from 3 to 10 carbon atoms in a monocyclic ring, from 7 to 12 carbon atoms in a bicyclic ring, or up to about 20 carbon atoms in a polycyclic ring. Preferably, the cycloalkyl group may be monocyclic. Examples of cycloalkyl groups include (C₃-C₂₀) cycloalkyl or (C₃-C₁₀) cycloalkyl, and preferably (C₃-C₇) cycloalkyl (e.g., cyclopropyl, cyclobutyl, cyclopentyl, cyclohexyl, and cycloheptyl) but are not limited thereto. In addition, the cycloalkyl group may be optionally substituted.

As used herein, the terms "heterocyclyl(hetero ring)" refer to a monovalent or divalent, saturated or partially saturated non-aromatic ring structure, preferably a 3- to 10-membered ring, more preferably a 3- to 7-membered ring, in which the ring structure contains at least 1 heteroatom, preferably 1 to 4 heteroatoms, more preferably 1 to 2 heteroatom(s). Suitable examples of the heteroatom include oxygen, sulfur, and nitrogen, but are not limited thereto. The term "heterocyclyl" also include polycyclic ring systems having two or more cyclic rings in which one or more carbons are common to two adjacent rings, wherein at least one of the rings is heterocyclic and the other cyclic ring may be, for example, cycloalkyl, cycloalkenyl, cycloalkynyl, aryl, heteroaryl, and/or heterocyclyl. Bicyclic and polycyclic/heterocyclic ring systems may be fused, bridged, or spiro ring systems. Suitable examples of the heterocycloalkyl group include azetidinyl, dihydropyridyl, dihydroindolyl, tetrahydropyridyl (piperidinyl), tetrahydrothiophenyl, sulfur-oxidized tetrahydrothiophenyl, indolenyl, piperidinyl, 4- piperidinyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, 6H-1,2,5-thiadiazinyl, pyranyl, chromenyl, xanthenyl, phenoxatinyl, 2H-pyrrolyl, 3H-indolyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, quinuclidinyl, morpholinyl, and oxazolidinyl(each of which may be substituted or unsubstituted), but are not limited thereto.

As used herein, the term "alkylene" may refer to an alkylene group having 1 to 20 carbon atoms, 1 to 16 carbon atoms, 1 to 12 carbon atoms, 1 to 10 carbon atoms, or 1 to 4 carbon atoms, unless otherwise specified. The alkylene group may be a straight-chain, branched, or cyclic alkylene group, and may optionally be substituted with one or more substituents.

As used herein, the term "aryl" includes substituted or unsubstituted monovalent or divalent aromatic hydrocarbon groups, which may be monocyclic, bicyclic or polycyclic, in which each atom of the ring is carbon. Preferably, the aryl ring is a 6- to 20-membered ring, a 6- to 14-membered ring, a 6- to 10-membered ring, or more preferably a 6-membered ring. An aryl group may be a polycyclic ring system having two or more cyclic rings in which two or more carbons are common to two adjacent rings, wherein at least one of the rings may be aromatic, and the other cyclic ring may be, for example, a cycloalkyl, cycloalkenyl, cycloalkynyl, aryl, heteroaryl, and/or heterocycloalkyl. Aryl groups include benzene, naphthalene, phenanthrene, anthracene, indene, indane, phenol, and aniline, but are not limited thereto.

As used herein, the term "heteroaryl" refers to a substituted or unsubstituted monovalent or divalent aromatic group, which is monocyclic, bicyclic or polycyclic, containing one or more heteroatoms in the ring. Examples of suitable heteroatoms that may be contained in the aromatic ring include oxygen, sulfur and nitrogen, but are not limited thereto. In polycyclic heteroaryl ring systems, the ring system has at least two cyclic rings in which at least one carbon atom is common to two adjacent rings, wherein at least one of the rings is heteroaromatic and the other cyclic rings may be, for example, cycloalkyl, cycloalkenyl, cycloalkynyl, aryl, heteroaryl, and/or heterocyclyl. Bicyclic and polycyclic/heteroaryl ring systems may be fused, bridged, or spiro ring systems. Representative examples of heteroaryl groups include triazolyl, tetrazolyl, oxadiazolyl, pyridyl, furyl, benzofuranyl, quinolinyl, pyrrolyl, indolyl, oxazolyl, benzoxazolyl, imidazolyl, benzimidazolyl, isoxazolyl, pyrazolyl, pyridazinyl, pyrimidinyl, pyrazinyl, triazinyl, cinnolinyl, phthalazinyl, quinazolinyl, pyrimidyl, oxetanyl, azepinyl, piperazinyl, morpholinyl, dioxanyl, and oxazolyl (each of which may be unsubstituted or substituted), but are not limited thereto.

The term "substituted" used in the present invention for alkyl, alkoxy, alkoxyalkyl, alkylamino, aryl, heteroaryl, cycloalkyl or heterocyclyl refers to alkyl, alkoxy, alkoxyalkyl, alkylamino, aryl, heteroaryl, heterocyclyl or cycloalkyl, in which one or more hydrogen atoms are each independently replaced by a non-hydrogen substituent. A substituent may include any of the substituents described herein, for example, halogen, hydroxyl, alkyl, amino, nitroalkyl, alkoxy, carbonyl (e.g., carboxyl, alkoxycarbonyl, formyl, or acyl), phosphoryl, phosphate, phosphonate, amido, amidin, imine, cyano, azido, sulfate, sulfonate, sulfamoyl, sulfonamido, sulfonyl, heterocyclyl, aralkyl, or aromatic or heteroaromatic moieties, but are not limited thereto. It will be understood by those skilled in the art that a substituted moiety on the hydrocarbon chain may itself be optionally substituted. Unless otherwise specified, the site of attachment of a substituent as used herein may be at any suitable position of the substituent.

As used herein, the terms "treat", "treating", "treatment", "improve" and "improving" refer to any objective or subjective parameter, e.g., decline; remission; reducing symptoms or making the symptom, injury, pathology or condition more tolerable by a patient; reducing the frequency or duration of a symptom or condition; or, in some circumstances, any indication of success in treating or ameliorating an injury, pathology, condition, or symptom (e.g., pain), including preventing the onset of the symptom or condition. Treatment or improvement of symptoms may be based on any objective or subjective parameter, for example, the results of a physical examination.

As used herein, the term "prevention" collectively refers to partially or completely delaying or inhibiting the onset or recurrence of a disease, disorder, or a symptom associated therewith; preventing the acquisition or reacquisition of the disease or disorder; or reducing the risk of acquiring the disease or disorder. The prevention includes any act of suppressing or delaying the onset of cancer through the composition according to one embodiment of the present invention.

The phrase "pharmaceutically acceptable" is an expression used in the art to indicate that a substance or composition must be chemically and/or toxicologically compatible with the other components of the formulation and/or the mammal to be treated therewith.

"Pharmaceutically acceptable salt" or "salt" are used herein to refer to an acid or base addition salt that is suitable for or compatible with the treatment of patient. An exemplary inorganic acid that forms a suitable salt may be hydrochloric acid, hydrobromic acid, sulfuric acid and phosphoric acid, as well as a metal salt, for example sodium monohydrogen orthophosphate and potassium hydrogen sulfate. An exemplary organic acid that forms a suitable salt may be mono-, di- and tricarboxylic acids, for example glycolic acid, lactic acid, pyruvic acid, malonic acid, succinic acid, glutaric acid, fumaric acid, malic acid, tartaric acid, citric acid, ascorbic acid, maleic acid, benzoic acid, phenylacetic acid, cinnamic acid and salicylic acid, as well as sulfonic acid, for example p-toluene sulfonic acid and methanesulfonic acid. Mono- or di-acid salts may be formed, and such salts may exist in hydrated, solvated or substantially anhydrous form. In general, acid addition salts of the compounds of the present invention are more soluble in water and various hydrophilic organic solvents compared to free base forms thereof, and generally exhibit higher melting points. The selection of suitable salts is known to those skilled in the art. Other non-pharmaceutically acceptable salts, for example, oxalates may be used in the isolation of the compounds of the present invention, for example for laboratory use or for subsequent conversion into pharmaceutically acceptable acid addition salts. An exemplary inorganic base that form a suitable salt may be lithium, sodium, potassium, calcium, magnesium, or barium hydroxides. An exemplary organic base that form a suitable salt may be aliphatic, cycloaliphatic or aromatic organic amines, for example, methylamine, trimethylamine and picoline or ammonia. Thus, in some embodiments, contemplated salts of the present invention may be alkyl, dialkyl, trialkyl or tetra-alkyl ammonium salts. In certain embodiments, contemplated salts of the present invention may be L-arginine, benenthamine, benzathine, betaine, calcium hydroxide, choline, deanol, diethanolamine, diethylamine, 2-(diethylamino)ethanol, ethanolamine, ethylenediamine, N-methylglucamine, hydrabamine, 1H-imidazole, lithium, L-lysine, magnesium, 4-(2-hydroxyethyl)morpholine, piperazine, potassium, 1-(2- hydroxyethyl)pyrrolidine, sodium, triethanolamine, tromethamine, and zinc salts, but is not limited thereto. In certain embodiments, contemplated salts of the present invention may be Na, Ca, K, Mg, Zn or other metal salts, but is not limited thereto.

The selection of appropriate salts is known to those skilled in the art.

Pharmaceutically acceptable acid addition salts may also exist as various solvates, for example, solvates with water, methanol, ethanol, dimethylformamide, and the like. Mixtures of these solvates may also be prepared. The source of such solvates may be from the solvent used for crystallization, inherently present in the solvent for manufacture or crystallization, or accidentally introduced through such a solvent.

As used herein, the term "IC₅₀" refers to the concentration of an inhibitor or compound that achieves 50% inhibition of a target protein.

As used herein, the term "GI₅₀" refers to the concentration of an inhibitor or compound that produces 50% inhibition relative to the maximum inhibition of cell proliferation.

As used herein, the terms "subject", and "patient" refer to a warm-blooded animal, for example, pig, cow, chicken, horse, guinea pig, mouse, rat, gerbil, cat, rabbit, dog, monkey, chimpanzee and human.

### Pyrrolotriazine derivative compound

The present invention provides novel pyrrolotriazine derivative compound.

In one embodiment, the present invention relates to a novel pyrrolotriazine derivative compound represented by the following formula 1 or a pharmaceutically acceptable salt thereof. 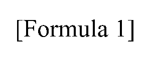 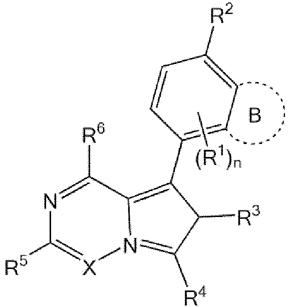 wherein in Formula 1,
X is C or N,
Ring B is absent or is (3-10 membered)cycloalkyl or (3-10 membered)heterocyclyl,
R¹ is each independently selected from the group consisting of hydrogen, halogen, C₁₋₁₀ alkyl, C₁₋₁₀ haloalkyl, C₁₋₁₀ alkoxy, C₁₋₆ haloalkoxy, (3-10 membered)cycloalkyl, C₁₋₁₀ alkylene-(3-10 membered)cycloalkyl, (3-10 membered)heterocyclyl, C₁₋₁₀ alkylene-(3-10 membered)heterocyclyl, (6-14 membered)aryl, C₁₋₁₀ alkylene-(6-14 membered)aryl, (5-12 membered)heteroaryl, C₁₋₁₀ alkylene-(5-12 membered)heteroaryl, NRₐR_{b}, O-C₁₋₁₀ alkylene-NRₐR_{b}, C₁₋₁₀ alkylene-O-NRₐR_{b}, C(O)-NRₐR_{b}, C₁₋₁₀ alkylene-C(O)-NRₐR_{b} and C(O)O-NRₐR_{b},
wherein (3-10 membered)cycloalkyl, (3-10 membered)heterocyclyl, (6-14 membered)aryl and (5-12 membered)heteroaryl are each independently unsubstituted or optionally substituted with a substituent selected from the group consisting of C₁₋₆ alkyl, C₁₋₆ alkoxy, halogen, C₁₋₆ haloalkyl, NH₂, NO₂, OH and CN,
n is an integer from 1 to 4,
R² is selected from the group consisting of hydrogen, halogen, C₁₋₅ alkyl, C₁₋₅ haloalkyl, C₁₋₅ alkoxy, C₁₋₅ haloalkoxy, C(O)-C₁₋₅ alkyl, C(O)-C₁₋₅ haloalkyl, C(O)O-C₁₋₅ alkyl, C(O)O-C₁₋₅ haloalkyl, CC(O)OC₁₋₅ alkyl, CC(O)O-C₁₋₅ haloalkyl, NR_{c}R_{d}, O-(6-14 membered) aryl, and O-(5-12 membered) heteroaryl,
wherein R_{c} and R_{d} are each independently optionally substituted with a substituent selected from the group consisting of hydrogen, C₁₋₅ alkyl, C₁₋₅ haloalkyl, C₁₋₅ alkoxy, C₁₋₅ haloalkoxy, C₁₋₃ aryl, C₁₋₃ heteroaryl, C(O)O-C₁₋₅alkyl, C(O)O-C₁₋₅haloalkyl, C(O)O-(3-10 membered) cycloalkyl, C(O)C₁₋₃ aryl, C(O)-(5-12 membered) heteroaryl, and C(O)NHC₁₋₅alkyl,
wherein (3-10 membered)cycloalkyl and (5-12 membered) heteroaryl are each independently unsubstituted or optionally substituted with a substituent selected from the group consisting of C₁₋₆alkyl, C₁₋₆alkoxy, halogen and C₁₋₆haloalkyl,
R³ is selected from the group consisting of hydrogen, halogen, C₁₋₆alkyl, C₁₋₆haloalkyl, C₁₋₆alkoxy, NH₂, OH, CN, COOH, NO₂, C(O)C₁₋₆alkyl, C(O)OC₁₋₆alkyl and C₁₋₆alkylamino,
R⁴ is selected from the group consisting of (3-10 membered) cycloalkyl, (3-14 membered) heterocyclyl, (3-14 membered) N-heterocyclyl, (6-14 membered) aryl, (5-12 membered) heteroaryl, C₁₋₆ alkylene-(3-10 membered)cycloalkyl, C₁₋₆ alkylene-(3-14 membered) heterocyclyl, C₁₋₆ alkylene-(3-10 membered) N-heterocyclyl, C₁₋₆ alkylene-(6-14 membered) aryl, C₁₋₆ alkylene-(5-12 membered) heteroaryl, NRₒRₚ and 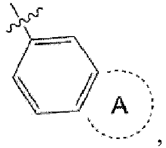
wherein ring A is (3-10 membered) cycloalkyl or (3-14 membered) heterocyclyl,
wherein R⁴ is optionally substituted with one or more R⁷,
wherein R⁷ is each independently selected from the group consisting of halogen, C₁₋₆ alkyl, C₁₋₆ haloalkyl, C₁₋₆ alkoxy, C₁₋₆ haloalkoxy, C₁₋₁₀ alkylene-C₁₋₆ alkoxy, C₁₋₁₀ alkylene-C₁₋₆ haloalkoxy, (3-10 membered) cycloalkyl, C₁₋₁₀ alkylene-(3-10 membered) cycloalkyl, (6-14 membered) aryl, C₁₋₁₀ alkylene-(6-14 membered) aryl, (5-12 membered) heteroaryl, C₁₋₁₀ alkylene-(5-12 membered) heteroaryl, NRₑR_{f}, C₁₋₁₀ alkyl-NRₑR_{f}, C₁₋₁₀ alkylene-NRₑR_{f}, oxo, C(O)-NRₑR_{f}, C₁₋₁₀ alkylene-C(O)-NRₑR_{f}, C(O)O-NRₑR_{f}, CN, OH, NO₂, C(O)Rₑ, C(O)ORₑ, C₁₋₆ alkylene-C(O)ORₑ and C₁₋₆ alkylene-OH,
R⁵ is selected from the group consisting of hydrogen, halogen, C₁₋₆ alkyl, C₁₋₆ haloalkyl, C₁₋₆ alkoxy, NH₂, OH, CN, COOH, NO₂, C(O)C₁₋₆ alkyl, C(O)OC₁₋₆ alkyl and C₁₋₆ alkylamino,
R⁶ is NR_{g}Rₕ,
Rₐ, R_{b}, Rₑ, R_{f}, R_{g} and Rₕ are each independently selected from the group consisting of hydrogen, halogen, C₁₋₆ alkyl, C₁₋₆ haloalkyl, C(O)C₁₋₆ alkyl, C(O)C₁₋₆ haloalkyl, C(O)OC₁₋₆ alkyl and C(O)OC₁₋₆ haloalkyl,
Rₒ and Rₚ are each independently selected from the group consisting of hydrogen, halogen, C₁₋₆ alkyl, C₁₋₆ haloalkyl, (3-10 membered)cycloalkyl, C₁₋₁₀ alkylene-(3-10 membered)cycloalkyl, (3-10 membered) heterocyclyl and C₁₋₁₀ alkylene-(3-10 membered) heterocyclyl,
wherein (3-10 membered)cycloalkyl, C₁₋₁₀ alkylene-(3-10 membered)cycloalkyl, (3-10 membered)heterocyclyl or C₁₋₁₀ alkylene-(3-10 membered)heterocyclyl are optionally substituted with one or more R⁸, wherein R⁸ is each independently selected from the group consisting halogen, C₁₋₆ alkyl, C₁₋₆ haloalkyl, C₁₋₆ alkoxy, NR_{q}Rᵣ, C₁₋₁₀ alkylene-NR_{q}Rᵣ, oxo, C(O)-NR_{q}Rᵣ, C₁₋₁₀ alkylene-C(O)-NR_{q}Rᵣ, C(O)O-NR_{q}Rᵣ, CN, OH, NO₂, C(O)R_{q}, C(O)OR_{q}, C₁₋₆ alkylene-C(O)OR_{q} and C₁₋₆ alkylene-OH,
R_{q} and Rᵣ may be each independently selected from the group consisting of hydrogen, halogen, C₁₋₆ alkyl and C₁₋₆ haloalkyl.

In one specific embodiment of the present invention, R¹ is selected from the group consisting of hydrogen, halogen, C₁₋₁₀ alkyl, C₁₋₁₀ haloalkyl, C₁₋₁₀ alkoxy, C₁₋₆ haloalkoxy, NRₐR_{b}, O-C₁₋₁₀ alkylene-NRₐR_{b}, C₁₋₁₀ alkylene-O-NRₐR_{b}, C(O)-NRₐR_{b}, C₁₋₁₀ alkylene-C(O)-NRₐR_{b} and C(O)O-NRₐR_{b},
n is an integer from 1 to 4,
R² is selected from the group consisting of hydrogen, halogen, C₁₋₅ alkyl, C₁₋₅ haloalkyl, C₁₋₅ alkoxy, C₁₋₅ haloalkoxy, NR_{c}R_{d}, O-(6-14 membered) aryl and O-(5-12 membered) heteroaryl,
wherein R_{c} and R_{d} are each independently optionally substituted with at least one substituent selected from the group consisting of hydrogen, C₁₋₅ alkyl, C₁₋₅ haloalkyl, C₁₋₅ alkoxy, C₁₋₅ haloalkoxy, C(O)O-C₁₋₅ alkyl, C(O)O-C₁₋₅ haloalkyl, C(O)O-(3-10 membered)cycloalkyl and C(O)-(5-12 membered)heteroaryl,
wherein (3-10 membered) cycloalkyl and (5-12 membered) heteroaryl are each independently unsubstituted or optionally substituted with C₁₋₆ alkyl or C₁₋₆ haloalkyl,
R³ may be selected from the group consisting of hydrogen, halogen, C₁₋₆ alkyl and C₁₋₆ haloalkyl.

In one specific embodiment of the present invention,
R⁴ is selected from the group consisting of (3-10 membered) cycloalkyl, (3-14 membered) heterocyclyl, (3-14 membered) N-heterocyclyl, (6-14 membered) aryl, (5-12 membered) heteroaryl, C₁₋₆ alkylene-(3-10 membered) cycloalkyl, C₁₋₆ alkylene-(3-14 membered) heterocyclyl, C₁₋₆ alkylene-(3-10 membered) N-heterocyclyl, C₁₋₆ alkylene-(6-14 membered) aryl, C₁₋₆ alkylene-(5-12 membered) heteroaryl, NRₒRₚ and 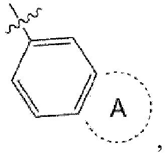
wherein ring A is (3-10 membered) cycloalkyl or (3-14 membered) heterocyclyl,
wherein R⁴ is optionally substituted with one or more R⁷, wherein R⁷ is selected from the group consisting of halogen, C₁₋₆ alkyl, C₁₋₆ haloalkyl, C₁₋₆ alkoxy, C₁₋₆ haloalkoxy, C₁₋₁₀ alkylene-C₁₋₆ alkoxy, C₁₋₁₀ alkylene-C₁₋₆ haloalkoxy, (3-10 membered) cycloalkyl, (6-14 membered) aryl, (5-12 membered) heteroaryl, NRₑR_{f}, C₁₋₁₀ alkylene-NRₑR_{f}, C₁₋₁₀ alkylene-NRₑR_{f}, oxo, C(O)-NRₑR_{f}, C₁₋₁₀ alkylene-C(O)-NRₑR_{f}, C(O)O-NRₑR_{f}, CN, OH, NO₂, C(O)Rₑ, C(O)ORₑ, C₁₋₆ alkylene-C(O)ORₑ, and C₁₋₆ alkylene-OH,
Rₒ and Rₚ are each independently selected from the group consisting of hydrogen, halogen, C₁₋₆ alkyl, C₁₋₆ haloalkyl, (3-10 membered) cycloalkyl, C₁₋₁₀ alkylene-(3-10 membered) cycloalkyl, (3-10 membered) heterocyclyl and C₁₋₁₀ alkylene-(3-10 membered) heterocyclyl,
wherein (3-10 membered) cycloalkyl, C₁₋₁₀ alkylene-(3-10 membered) cycloalkyl, (3-10 membered) heterocyclyl or C₁₋₁₀ alkylene-(3-10 membered) heterocyclyl are optionally substituted with one or more R⁸, wherein R⁸ is halogen, C₁₋₆ alkyl, C₁₋₆ haloalkyl, C₁₋₆ alkoxy, NR_{q}Rᵣ, C₁₋₁₀ alkylene-NR_{q}Rᵣ, oxo, C(O)-NR_{q}Rᵣ, C₁₋₁₀ alkylene-C(O)-NR_{q}Rᵣ, C(O)O-NR_{q}Rᵣ, CN, OH, NO₂, C(O)R_{q}, C(O)OR_{q}, C₁₋₆ alkylene-C(O)OR_{q} and C₁₋₆ alkylene-OH,
R_{q} and Rᵣ may each be independently selected from the group consisting of hydrogen, halogen, C₁₋₆ alkyl and C₁₋₆ haloalkyl.

In one specific embodiment of the present invention, R⁵ is selected from the group consisting of hydrogen, halogen, C₁₋₆ alkyl, C₁₋₆ haloalkyl, C₁₋₆ alkoxy, NH₂, OH, CN, COOH, NO₂, C(O)C₁₋₆ alkyl, C(O)OC₁₋₆ alkyl, and C₁₋₆ alkylamino,
R⁶ is NR_{g}Rₕ,
R_{g} and Rₕ may each be independently selected from the group consisting of hydrogen, C₁₋₆ alkyl, C₁₋₆ haloalkyl, and C(O)C₁₋₆ alkyl.

In one specific embodiment of the present invention, R¹ is selected from the group consisting of hydrogen, halogen, C₁₋₁₀ alkyl, C₁₋₁₀ haloalkyl, C₁₋₁₀ alkoxy, and C₁₋₆ haloalkoxy,
R² is NR_{c}R_{d}, O-(6-14 membered) aryl or O-(5-12 membered) heteroaryl,
wherein R_{c} and R_{d} are each independently hydrogen, C(O)O-C₁₋₅ alkyl, C(O)O-C₁₋₅ haloalkyl, C(O)O-(3-10 membered) cycloalkyl and C(O)-(5-12 membered) heteroaryl,
wherein (3-10 membered) cycloalkyl and (5-12 membered) heteroaryl are each independently unsubstituted or optionally substituted with a substituent selected from the group consisting of C₁₋₆ alkyl, C₁₋₆ alkoxy, halogen, and C₁₋₆ haloalkyl, R³ is selected from the group consisting of hydrogen, halogen, C₁₋₆ alkyl and C₁₋₆ haloalkyl,
R⁴ is selected from the group consisting of monocyclic (3-7 membered) cycloalkyl, monocyclic (3-6 membered) heterocyclyl, spirocyclic (7-14 membered) heterocyclyl, fused cyclic (7-14 membered) heterocyclyl, (6-10 membered) aryl, (5-10 membered) heteroaryl, C₁₋₆ alkylene-(3-10 membered) N-heterocyclyl, NRₒRₚ and 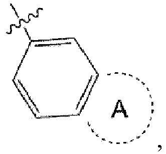
wherein ring A is (3-10 membered) cycloalkyl or (3-14 membered) heterocyclyl,
wherein R⁴ is optionally substituted with one or more R⁷, wherein R⁷ is selected from the group consisting of halogen, C₁₋₆ alkyl, C₁₋₆ haloalkyl, C₁₋₆ alkoxy, C₁₋₆ haloalkoxy, C₁₋₁₀ alkylene-C₁₋₆ alkoxy, (3-10 membered) cycloalkyl, (6-14 membered) aryl, (5-12 membered) heteroaryl, NRₑR_{f}, C₁₋₆ alkyl-NRₑR_{f}, C₁₋₁₀ alkylene-NRₑR_{f}, oxo, CN, OH, NO₂, C(O)ORₑ, C₁₋₆ alkylene-C(O)ORₑ and C₁₋₆ alkylene-OH,
R⁵ is hydrogen or halogen,
R⁶ is NR_{g}Rₕ,
Rₑ, R_{f}, R_{g} and Rₕ are each independently selected from the group consisting of hydrogen, C₁₋₆ alkyl, C₁₋₆ haloalkyl and C(O)C₁₋₆ alkyl,
Rₒ and Rₚ are each independently selected from the group consisting of hydrogen, halogen, C₁₋₆ alkyl, C₁₋₆ haloalkyl, (3-10 membered) cycloalkyl, C₁₋₁₀ alkylene-(3-10 membered) cycloalkyl, (3-10 membered) heterocyclyl and C₁₋₁₀ alkylene-(3-10 membered) heterocyclyl,
wherein (3-10 membered) cycloalkyl, C₁₋₁₀ alkylene-(3-10 membered) cycloalkyl, (3-10 membered) heterocyclyl or C₁₋₁₀ alkylene-(3-10 membered) heterocyclyl is optionally substituted with one or more R⁸, wherein R⁸ is selected from the group consisting of halogen, C₁₋₆ alkyl, C₁₋₆ haloalkyl, C₁₋₆ alkoxy, NR_{q}Rᵣ, C₁₋₁₀ alkylene-NR_{q}Rᵣ, oxo, C(O)-NR_{q}Rᵣ, C₁₋₁₀ alkylene-C(O)-NR_{q}Rᵣ, C(O)O-NR_{q}Rᵣ, CN, OH, NO₂, C(O)R_{q}, C(O)OR_{q}, C₁₋₆ alkylene-C(O)OR_{q} and C₁₋₆ alkylene-OH,
R_{q} and Rᵣ may each be independently selected from the group consisting of hydrogen, halogen, C₁₋₆ alkyl and C₁₋₆ haloalkyl.

In one specific embodiment of the present invention,
wherein ring B is absent or is (3-10 membered) heterocyclyl,
wherein R¹ is hydrogen or methoxy,
wherein R² is selected from the group consisting of 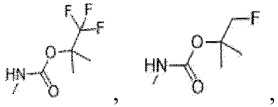 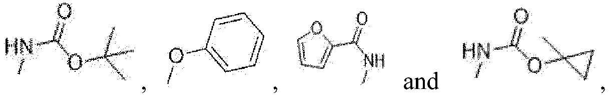
wherein R³ may be selected from the group consisting of hydrogen, halogen, C₁₋₃ alkyl and C₁₋₃ haloalkyl.

In one specific embodiment of the present invention, wherein R⁴ may be selected from the group consisting of

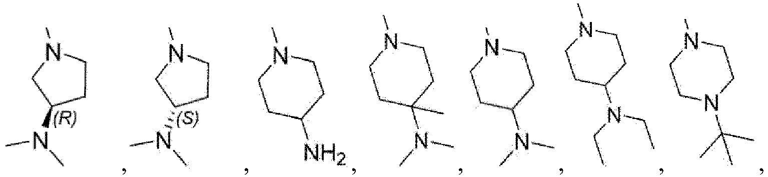

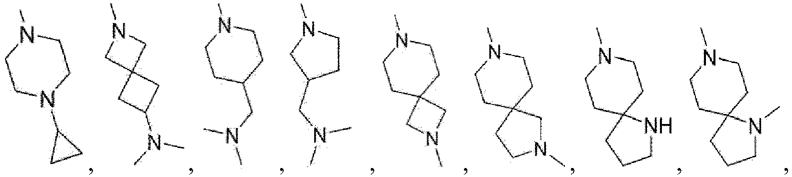

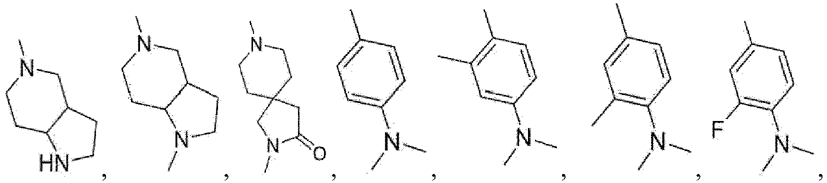

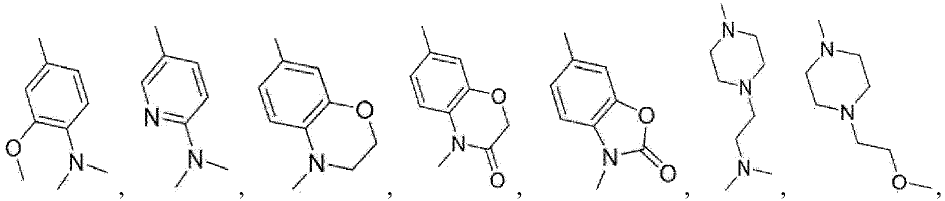

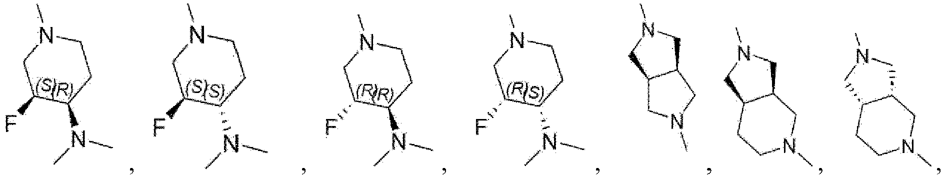

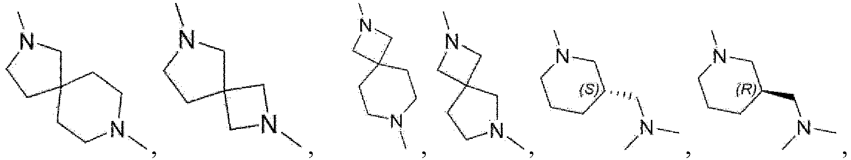

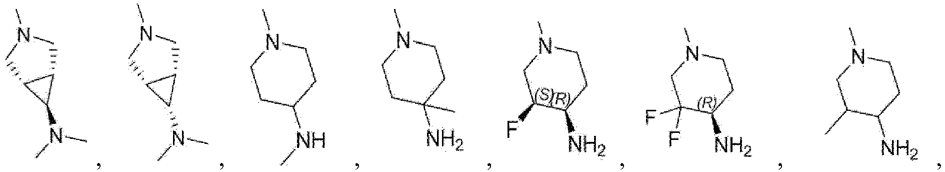

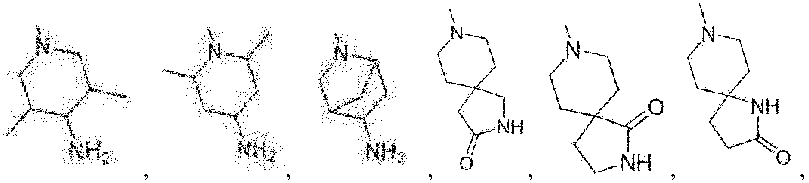

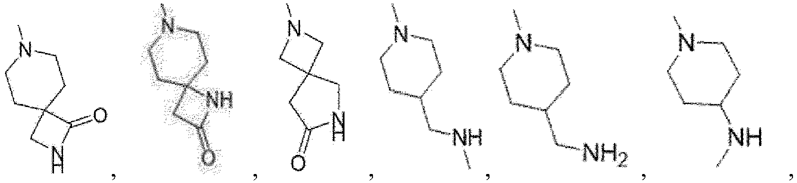

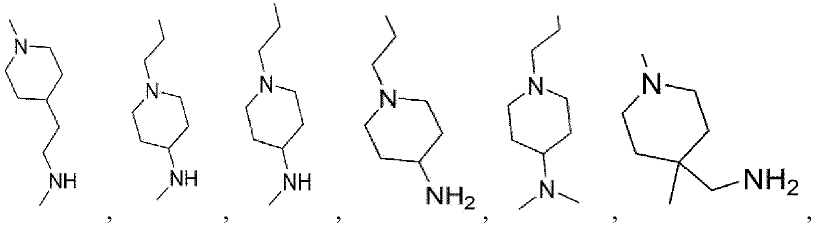

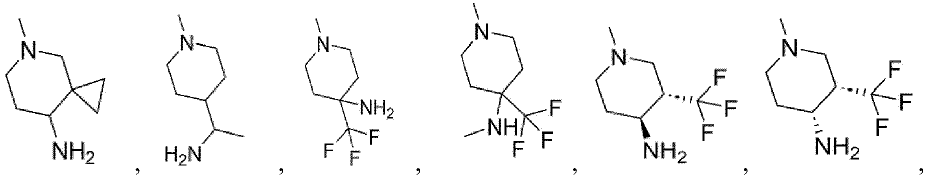

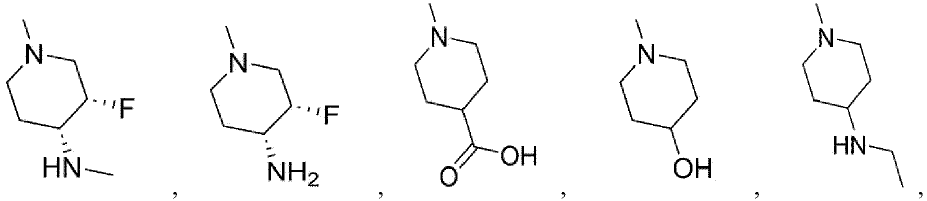

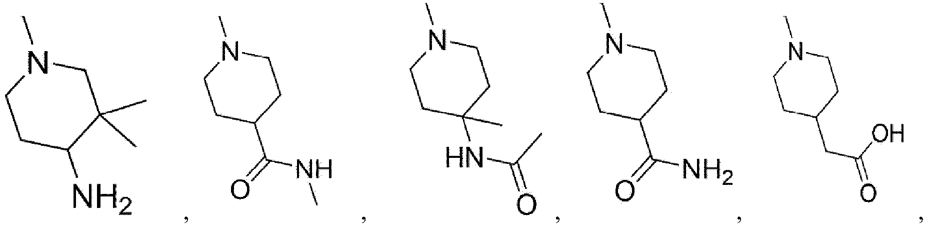

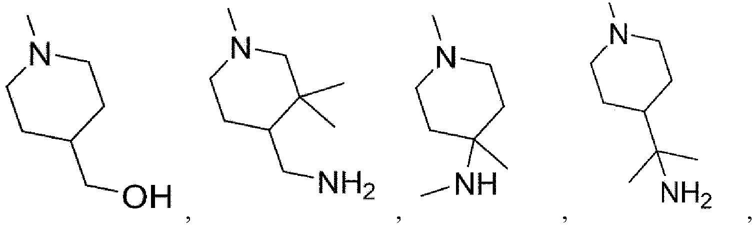

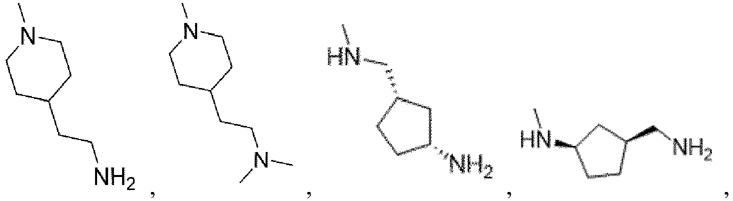

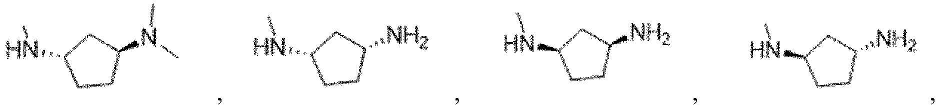

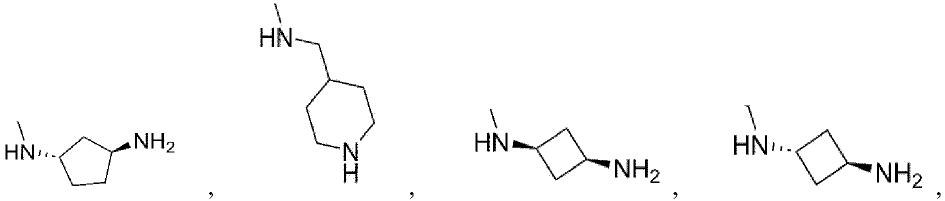

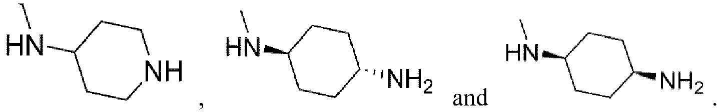

In one embodiment of the present invention,
R⁵ is hydrogen or halogen,
R⁶ is NR_{g}Rₕ,
R_{g} and Rₕ may each be independently hydrogen or C₁₋₆ alkyl.

In one specific embodiment of the present invention, wherein 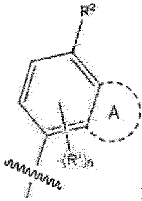 may be selected from the group consisting of

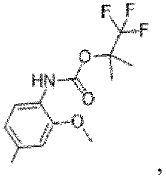

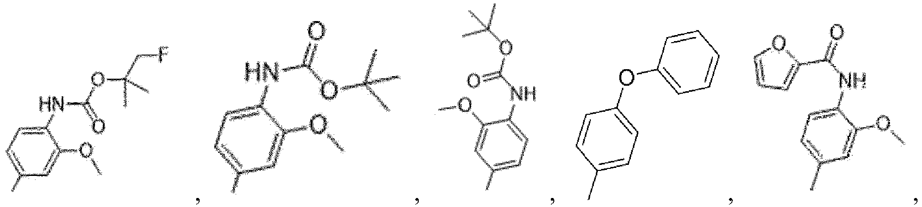

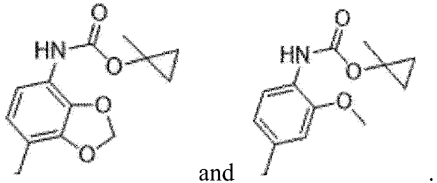

In one specific embodiment of the present invention, the representative compound of formula 1 comprises a compound selected from the group consisting of compounds 1 to 138 below, but is not limited thereto.

| No. | Compound name |
|---|---|
| 1 | tert-butyl (4-(4-amino-7-(4-(dimethylamino)phenyl)pyrrolo[2,1-f][1,2,4]triazin-5-yl)-2-methoxyphenyl)carbamate |
| 2 | tert-butyl (4-(4-amino-7-(4-(dimethylamino)-2-methylphenyl)pyrrolo[2,1-f][1,2,4]triazin-5-yl)-2-methoxyphenyl)carbamate |
| 3 | tert-butyl (4-(4-amino-7-(4-(dimethylamino)-3-methylphenyl)pyrrolo[2,1-f][1,2,4]triazin-5-yl)-2-methoxyphenyl)carbamate |
| 4 | tert-butyl (4-(4-amino-7-(4-(dimethylamino)-3-fluorophenyl)pyrrolo[2,1-f][1,2,4]triazin-5-yl)-2-methoxyphenyl)carbamate |
| 5 | tert-butyl (4-(4-amino-7-(4-(dimethylamino)-3-methoxyphenyl)pyrrolo[2,1-f][1,2,4]triazin-5-yl)-2-methoxyphenyl)carbamate |
| 6 | tert-butyl (4-(4-amino-7-(6-(dimethylamino)pyridin-3-yl)pyrrolo[2,1-f][1,2,4]triazin-5-yl)-2-methoxyphenyl)carbamate |
| 7 | tert-butyl (4-(4-amino-7-(4-methyl-3,4-dihydro-2H-benzo[b][1,4]oxazin-7-yl)pyrrolo[2,1-f][1,2,4]triazin-5-yl)-2-methoxyphenyl)carbamate |
| 8 | tert-butyl (4-(4-amino-7-(4-methyl-3-oxo-3,4-dihydro-2H-benzo[b][1,4]oxazin-7-yl)pyrrolo[2,1-f][1,2,4]triazin-5-yl)-2-methoxyphenyl)carbamate |
| 9 | tert-butyl (4-(4-amino-7-(3-methyl-2-oxo-2,3-dihydrobenzo[d]oxazol-6-yl)pyrrolo[2,1-f][1,2,4]triazin-5-yl)-2-methoxyphenyl)carbamate |
| 10 | tert-butyl (R)-(4-(4-amino-7-(3-(dimethylamino)pyrrolidin-1-yl)pyrrolo[2,1-f][1,2,4]triazin-5-yl)-2-methoxyphenyl)carbamate |
| 11 | tert-butyl (S)-(4-(4-amino-7-(3-(dimethylamino)pyrrolidin-1-yl)pyrrolo[2,1-f][1,2,4]triazin-5-yl)-2-methoxyphenyl)carbamate |
| 12 | tert-butyl (4-(4-amino-7-(4-aminopiperidin-1-yl)pyrrolo[2,1-f][1,2,4]triazin-5-yl)-2-methoxyphenyl)carbamate |
| 13 | tert-butyl (4-(4-amino-7-(4-(dimethylamino)-4-methylpiperidin-1-yl)pyrrolo[2,1-f][1,2,4]triazin-5-yl)-2-methoxyphenyl)carbamate |
| 14 | tert-butyl (4-(4-amino-7-(4-(diethylamino)piperidin-1-yl)pyrrolo[2,1-f][1,2,4]triazin-5-yl)-2-methoxyphenyl)carbamate |
| 15 | tert-butyl (4-(4-amino-7-(4-(dimethylamino)piperidin-1-yl)pyrrolo[2,1-f][1,2,4]triazin-5-yl)-2-methoxyphenyl)carbamate |
| 16 | tert-butyl (4-(4-amino-7-(4-(tert-butyl)piperazin-1-yl)pyrrolo[2,1-f][1,2,4]triazin-5-yl)-2-methoxyphenyl)carbamate |
| 17 | tert-butyl (4-(4-amino-7-(4-cyclopropylpiperazin-1-yl)pyrrolo[2,1-f][1,2,4]triazin-5-yl)-2-methoxyphenyl)carbamate |
| 18 | tert-butyl (4-(4-amino-7-(6-(dimethylamino)-2-azaspiro[3.3]heptan-2-yl)pyrrolo[2,1-f][1,2,4]triazin-5-yl)-2-methoxyphenyl)carbamate |
| 19 | tert-butyl (4-(4-amino-7-(4-((dimethylamino)methyl)piperidin-1-yl)pyrrolo[2,1-f][1,2,4]triazin-5-yl)-2-methoxyphenyl)carbamate |
| 20 | tert-butyl (4-(4-amino-7-(3-((dimethylamino)methyl)pyrrolidin-1-yl)pyrrolo[2,1-f][1,2,4]triazin-5-yl)-2-methoxyphenyl)carbamate |
| 21 | tert-butyl (4-(4-amino-7-(2-methyl-2,7-diazaspiro[3.5]nonan-7-yl)pyrrolo[2,1-f][1,2,4]triazin-5-yl)-2-methoxyphenyl)carbamate |
| 22 | tert-butyl (4-(4-amino-7-(2-methyl-2,8-diazaspiro[4.5]decan-8-yl)pyrrolo[2,1-f][1,2,4]triazin-5-yl)-2-methoxyphenyl)carbamate |
| 23 | tert-butyl (4-(4-amino-7-(1,8-diazaspiro[4.5]decan-8-yl)pyrrolo[2,1-f][1,2,4]triazin-5-yl)-2-methoxyphenyl)carbamate |
| 24 | tert-butyl (4-(4-amino-7-(1-methyl-1,8-diazaspiro[4.5]decan-8-yl)pyrrolo[2,1-f][1,2,4]triazin-5-yl)-2-methoxyphenyl)carbamate |
| 25 | tert-butyl (4-(4-amino-7-(octahydro-5H-pyrrolo[3,2-c]pyridin-5-yl)pyrrolo[2,1-f][1,2,4]triazin-5-yl)-2-methoxyphenyl)carbamate |
| 26 | tert-butyl (4-(4-amino-7-(1-methyloctahydro-5H-pyrrolo[3,2-c]pyridin-5-yl)pyrrolo[2,1-f][1,2,4]triazin-5-yl)-2-methoxyphenyl)carbamate |
| 27 | tert-butyl (4-(4-amino-7-(2-methyl-3-oxo-2,8-diazaspiro[4.5]decan-8-yl)pyrrolo[2,1-f][1,2,4]triazin-5-yl)-2-methoxyphenyl)carbamate |
| 28 | tert-butyl (4-(4-amino-7-(4-(2-(dimethylamino)ethyl)piperazin-1-yl)pyrrolo[2,1-f][1,2,4]triazin-5-yl)-2-methoxyphenyl)carbamate |
| 29 | tert-butyl (4-(4-amino-7-(4-(2-methoxyethyl)piperazin-1-yl)pyrrolo[2,1-f][1,2,4]triazin-5-yl)-2-methoxyphenyl)carbamate |
| 30 | tert-butyl (4-(4-amino-7-((3S,4R)-4-(dimethylamino)-3-fluoropiperidin-1-yl)pyrrolo[2,1-f][1,2,4]triazin-5-yl)-2-methoxyphenyl)carbamate |
| 31 | tert-butyl (4-(4-amino-7-((3S,4S)-4-(dimethylamino)-3-fluoropiperidin-1-yl)pyrrolo[2,1-f][1,2,4]triazin-5-yl)-2-methoxyphenyl)carbamate |
| 32 | tert-butyl (4-(4-amino-7-((3R,4R)-4-(dimethylamino)-3-fluoropiperidin-1-yl)pyrrolo[2,1-f][1,2,4]triazin-5-yl)-2-methoxyphenyl)carbamate |
| 33 | tert-butyl (4-(4-amino-7-((3R,4S)-4-(dimethylamino)-3-fluoropiperidin-1-yl)pyrrolo[2,1-f][1,2,4]triazin-5-yl)-2-methoxyphenyl)carbamate |
| 34 | tert-butyl (4-(4-amino-7-((3aR,6aS)-5-methylhexahydropyrrolo[3,4-c]pyrrol-2(1H)-yl)pyrrolo[2,1-f][1,2,4]triazin-5-yl)-2-methoxyphenyl)carbamate |
| 35A | tert-butyl (4-(4-amino-7-((3aR,7aR)-5-methyloctahydro-2H-pyrrolo[3,4-c]pyridin-2-yl)pyrrolo[2,1-f][1,2,4]triazin-5-yl)-2-methoxyphenyl)carbamate |
| 35B | tert-butyl (4-(4-amino-7-((3aS,7aS)-5-methyloctahydro-2H-pyrrolo[3,4-c]pyridin-2-yl)pyrrolo[2,1-f][1,2,4]triazin-5-yl)-2-methoxyphenyl)carbamate |
| 36 | tert-butyl (4-(4-amino-7-(8-methyl-2,8-diazaspiro[4.5]decan-2-yl)pyrrolo[2,1-f][1,2,4]triazin-5-yl)-2-methoxyphenyl)carbamate |
| 37 | tert-butyl (4-(4-amino-7-(2-methyl-2,6-diazaspiro[3.4]octan-6-yl)pyrrolo[2,1-f][1,2,4]triazin-5-yl)-2-methoxyphenyl)carbamate |
| 38 | tert-butyl (4-(4-amino-7-(7-methyl-2,7-diazaspiro[3.5]nonan-2-yl)pyrrolo[2,1-f][1,2,4]triazin-5-yl)-2-methoxyphenyl)carbamate |
| 39 | tert-butyl (4-(4-amino-7-(6-methyl-2,6-diazaspiro[3.4]octan-2-yl)pyrrolo[2,1-f][1,2,4]triazin-5-yl)-2-methoxyphenyl)carbamate |
| 40 | tert-butyl (S)-(4-(4-amino-7-(3-((dimethylamino)methyl)piperidin-1-yl)pyrrolo[2,1-f][1,2,4]triazin-5-yl)-2-methoxyphenyl)carbamate |
| 41 | tert-butyl (R)-(4-(4-amino-7-(3-((dimethylamino)methyl)piperidin-1-yl)pyrrolo[2,1-f][1,2,4]triazin-5-yl)-2-methoxyphenyl)carbamate |
| 42 | tert-butyl (4-(4-amino-7-((1R,5S,6S)-6-(dimethylamino)-3-azabicyclo[3.1.0]hexan-3-yl)pyrrolo[2,1-f][1,2,4]triazin-5-yl)-2-methoxyphenyl)carbamate |
| 44 | tert-butyl (4-(4-amino-7-(4-(methylamino)piperidin-1-yl)pyrrolo[2,1-f][1,2,4]triazin-5-yl)-2-methoxyphenyl)carbamate |
| 45 | tert-butyl (4-(4-amino-7-(4-amino-4-methylpiperidin-1-yl)pyrrolo[2,1-f][1,2,4]triazin-5-yl)-2-methoxyphenyl)carbamate |
| 46 | tert-butyl (4-(4-amino-7-((3S,4R)-4-amino-3-fluoropiperidin-1-yl)pyrrolo[2,1-f][1,2,4]triazin-5-yl)-2-methoxyphenyl)carbamate |
| 47 | tert-butyl (R)-(4-(4-amino-7-(4-amino-3,3-difluoropiperidin-1-yl)pyrrolo[2,1-f][1,2,4]triazin-5-yl)-2-methoxyphenyl)carbamate |
| 48 | tert-butyl (4-(4-amino-7-(4-amino-3-methylpiperidin-1-yl)pyrrolo[2,1-f][1,2,4]triazin-5-yl)-2-methoxyphenyl)carbamate |
| 49 | tert-butyl (4-(4-amino-7-(4-amino-3,5-dimethylpiperidin-1-yl)pyrrolo[2,1-f][1,2,4]triazin-5-yl)-2-methoxyphenyl)carbamate |
| 50 | tert-butyl (4-(4-amino-7-(4-amino-2,6-dimethylpiperidin-1-yl)pyrrolo[2,1-f][1,2,4]triazin-5-yl)-2-methoxyphenyl)carbamate |
| 51 | tert-butyl (4-(4-amino-7-(5-amino-2-azabicyclo[2.2.1]hepten-2-yl)pyrrolo[2,1-f][1,2,4]triazin-5-yl)-2-methoxyphenyl)carbamate |
| 52 | tert-butyl (4-(4-amino-7-(3-oxo-2,8-diazaspiro[4.5]decan-8-yl)pyrrolo[2,1-f][1,2,4]triazin-5-yl)-2-methoxyphenyl)carbamate |
| 53 | tert-butyl (4-(4-amino-7-(1-oxo-2,8-diazaspiro[4.5]decan-8-yl)pyrrolo[2,1-f][1,2,4]triazin-5-yl)-2-methoxyphenyl)carbamate |
| 54 | tert-butyl (4-(4-amino-7-(2-oxo-1,8-diazaspiro[4.5]decan-8-yl)pyrrolo[2,1-f][1,2,4]triazin-5-yl)-2-methoxyphenyl)carbamate |
| 55 | tert-butyl (4-(4-amino-7-(1-oxo-2,7-diazaspiro[3.5]nonan-7-yl)pyrrolo[2,1-f][1,2,4]triazin-5-yl)-2-methoxyphenyl)carbamate |
| 56 | tert-butyl (4-(4-amino-7-(2-oxo-1,7-diazaspiro[3.5]nonan-7-yl)pyrrolo[2,1-f][1,2,4]triazin-5-yl)-2-methoxyphenyl)carbamate |
| 57 | tert-butyl (4-(4-amino-7-(7-oxo-2,6-diazaspiro[3.4]octen-2-yl)pyrrolo[2,1-f][1,2,4]triazin-5-yl)-2-methoxyphenyl)carbamate |
| 58 | tert-butyl (4-(4-amino-7-(4-(methylamino)piperidin-1-yl)pyrrolo[2,1-f][1,2,4]triazin-5-yl)-2-methoxyphenyl)carbamate |
| 59 | tert-butyl (4-(4-amino-7-(4-(aminomethyl)piperidin-1-yl)pyrrolo[2,1-f][1,2,4]triazin-5-yl)-2-methoxyphenyl)carbamate |
| 60 | 1-fluoro-2-methylpropan-2-yl (4-(4-amino-7-(4-aminopiperidin-1-yl)-6-methylpyrrolo[2,1-f][1,2,4]triazin-5-yl)-2-methoxyphenyl)carbamate |
| 61 | 1-fluoro-2-methylpropan-2-yl (4-(4-amino-6-methyl-7-(4-(methylamino)piperidin-1-yl)pyrrolo[2,1-f][1,2,4]triazin-5-yl)-2-methoxyphenyl)carbamate |
| 62 | 1-fluoro-2-methylpropan-2-yl (4-(4-amino-7-(4-(dimethylamino)piperidin-1-yl)-6-methylpyrrolo[2,1-f][1,2,4]triazin-5-yl)-2-methylmethoxy)carbamate |
| 63 | 1-fluoro-2-methylpropan-2-yl (4-(4-amino-6-methyl-7-(4-(2-(methylamino)ethyl)piperidin-1-yl)pyrrolo[2,1-f][1,2,4]triazin-5-yl)-2-methoxyphenyl)carbamate |
| 64 | 1-fluoro-2-methylpropan-2-yl (4-(4-amino-6-methyl-7-(2-(4-(methylamino)piperidin-1-yl)ethyl)pyrrolo[2,1-f][1,2,4]triazin-5-yl)-2-methoxyphenyl)carbamate |
| 65 | 1-fluoro-2-methylpropan-2-yl (4-(4-amino-7-(4-aminopiperidin-1-yl)pyrrolo[2,1-f][1,2,4]triazin-5-yl)-2-methoxyphenyl)carbamate |
| 66 | 1-fluoro-2-methylpropan-2-yl (4-(4-amino-7-(4-(2-(methylamino)ethyl)piperidin-1-yl)pyrrolo[2,1-f][1,2,4]triazin-5-yl)-2-methoxyphenyl)carbamate |
| 67 | 1-fluoro-2-methylpropan-2-yl (4-(4-amino-7-(2-(4-(methylamino)piperidin-1-yl)ethyl)pyrrolo[2,1-f][1,2,4]triazin-5-yl)-2-methoxyphenyl)carbamate |
| 68 | 1-fluoro-2-methylpropan-2-yl (4-(4-amino-7-(2-(4-aminopiperidin-1-yl)ethyl)pyrrolo[2,1-f][1,2,4]triazin-5-yl)-2-methoxyphenyl)carbamate |
| 69 | 1-fluoro-2-methylpropan-2-yl (4-(4-amino-7-(2-(4-(dimethylamino)piperidin-1-yl)ethyl)pyrrolo[2,1-f][1,2,4]triazin-5-yl)-2-methoxyphenyl)carbamate |
| 70 | 1-methylcyclopropyl (7-(4-amino-7-(4-aminopiperidin-1-yl)pyrrolo[2,1-f][1,2,4]triazin-5-yl)benzo[d][1,3]dioxo-4-yl)carbamate |
| 71 | 1-methylcyclopropyl (4-(4-amino-7-(4-aminopiperidin-1-yl)pyrrolo[2,1-f][1,2,4]triazin-5-yl)-2-methoxyphenyl)carbamate |
| 72 | 1,1,1-trifluoro-2-methylpropan-2-yl (4-(4-amino-7-(4-aminopiperidin-1-yl)pyrrolo[2,1-f][1,2,4]triazin-5-yl)-2-methoxyphenyl)carbamate |
| 73 | tert-butyl (7-(4-amino-7-(4-aminopiperidin-1-yl)pyrrolo[2,1-f][1,2,4]triazin-5-yl)benzo[d][1,3]dioxo-4-yl)carbamate |
| 74 | tert-butyl (4-(4-amino-7-(4-aminopiperidin-1-yl)-6-methylpyrrolo[2,1-f][1,2,4]triazin-5-yl)-2-methoxyphenyl)carbamate |
| 75 | tert-butyl (4-(4-amino-6-methyl-7-(4-(methylamino)piperidin-1-yl)pyrrolo[2,1-f][1,2,4]triazin-5-yl)-2-methoxyphenyl)carbamate |
| 76 | tert-butyl (4-(4-amino-7-(4-(dimethylamino)piperidin-1-yl)-6-methylpyrrolo[2,1-f][1,2,4]triazin-5-yl)-2-methoxyphenyl)carbamate |
| 77 | tert-butyl (4-(4-amino-6-methyl-7-(4-(2-(methylamino)ethyl)piperidin-1-yl)pyrrolo[2,1-f][1,2,4]triazin-5-yl)-2-methoxyphenyl)carbamate |
| 78 | tert-butyl (4-(4-amino-7-(4-aminopiperidin-1-yl)-6-methylpyrrolo[2,1-f][1,2,4]triazin-5-yl)-2-methoxyphenyl)carbamate |
| 79 | tert-butyl (4-(4-amino-7-(4-(aminomethyl)-4-methylpiperidin-1-yl)pyrrolo[2,1-f][1,2,4]triazin-5-yl)-2-methoxyphenyl)carbamate |
| 80 | tert-butyl (4-(4-amino-7-(8-amino-5-azaspiro[2.5]octen-5-yl)pyrrolo[2,1-f][1,2,4]triazin-5-yl)-2-methoxyphenyl)carbamate |
| 81 | tert-butyl (4-(4-amino-7-(4-(1-aminoethyl)piperidin-1-yl)pyrrolo[2,1-f][1,2,4]triazin-5-yl)-2-methoxyphenyl)carbamate |
| 82 | tert-butyl (4-(4-amino-7-(4-amino-4-ethylpiperidin-1-yl)pyrrolo[2,1-f][1,2,4]triazin-5-yl)-2-methoxyphenyl)carbamate |
| 83 | tert-butyl (4-(4-amino-7-(4-amino-4-(trifluoromethyl)piperidin-1-yl)pyrrolo[2,1-f][1,2,4]triazin-5-yl)-2-methoxyphenyl)carbamate |
| 84 | tert-butyl (4-(4-amino-7-(4-(methylamino)-4-(trifluoromethyl)piperidin-1-yl)pyrrolo[2,1-f][1,2,4]triazin-5-yl)-2-methoxyphenyl)carbamate |
| 85 | tert-butyl (4-(4-amino-7-((3S,4S)-4-amino-3-(trifluoromethyl)piperidin-1-yl)pyrrolo[2,1-f][1,2,4]triazin-5-yl)-2-methoxyphenyl)carbamate |
| 86 | tert-butyl (4-(4-amino-7-((3S,4R)-4-amino-3-(trifluoromethyl)piperidin-1-yl)pyrrolo[2,1-f][1,2,4]triazin-5-yl)-2-methoxyphenyl)carbamate |
| 87 | tert-butyl (4-(4-amino-7-((3S,4R)-3-fluoro-4-(methylamino)piperidin-1-yl)pyrrolo[2,1-f][1,2,4]triazin-5-yl)-2-methoxyphenyl)carbamate |
| 89 | 1-(4-amino-5-(4-((tert-butoxycarbonyl)amino)-3-methoxyphenyl)pyrrolo[2,1-f][1,2,4]triazin-7-yl)piperidine-4-carboxylic acid |
| 90 | tert-butyl (4-(4-amino-7-(4-hydroxypiperidin-1-yl)pyrrolo[2,1-f][1,2,4]triazin-5-yl)-2-methoxyphenyl)carbamate |
| 91 | tert-butyl (4-(4-amino-7-(4-amino-3,5-dimethylpiperidin-1-yl)pyrrolo[2,1-f][1,2,4]triazin-5-yl)-2-methoxyphenyl)carbamate |
| 92 | tert-butyl (4-(4-amino-7-(4-(ethylamino)piperidin-1-yl)pyrrolo[2,1-f][1,2,4]triazin-5-yl)-2-methoxyphenyl)carbamate |
| 93 | tert-butyl (4-(4-amino-7-(6-amino-3-azabicyclo[3.1.1]hepten-3-yl)pyrrolo[2,1-f][1,2,4]triazin-5-yl)-2-methoxyphenyl)carbamate |
| 94 | tert-butyl (4-(4-amino-7-(4-amino-3,3-dimethylpiperidin-1-yl)pyrrolo[2,1-f][1,2,4]triazin-5-yl)-2-methoxyphenyl)carbamate |
| 95 | tert-butyl (4-(4-amino-7-(4-(methylcarbamoyl)piperidin-1-yl)pyrrolo[2,1-f][1,2,4]triazin-5-yl)-2-methoxyphenyl) |
| 96 | tert-butyl (4-(7-(4-acetamido-4-methylpiperidin-yl)-4-aminopyrrolo[2,1-f][1,2,4]triazin-5-yl)-2-methoxyphenyl)carbamate |
| 97 | tert-butyl (4-(4-amino-7-(4-carbamoylpiperidin-1-yl)pyrrolo[2,1-f][1,2,4]triazin-5-yl)-2-methoxyphenyl)carbamate |
| 98 | tert-butyl (4-(4-amino-7-(2-(4-(dimethylamino)piperidin-1-yl)ethyl)pyrrolo[2,1-f][1,2,4]triazin-5-yl)-2-methoxyphenyl)carbamate |
| 99 | tert-butyl (4-(4-amino-7-(2-(4-(methylamino)piperidin-1-yl)ethyl)pyrrolo[2,1-f][1,2,4]triazin-5-yl)-2-methoxyphenyl)carbamate |
| 100 | tert-butyl (4-(4-amino-7-(2-(4-aminopiperidin-1-yl)ethyl)pyrrolo[2,1-f][1,2,4]triazin-5-yl)-2-methoxyphenyl)carbamate |
| 101 | 2-(1-(4-amino-5-(4-((tert-butoxycarbonyl)amino)-3-methoxyphenyl)pyrrolo[2,1-f][1,2,4]triazin-7-yl)piperidin-4-yl)acetic acid |
| 102 | tert-butyl (4-(4-amino-7-(4-(hydroxymethyl)piperidin-1-yl)pyrrolo[2,1-f][1,2,4]triazin-5-yl)-2-methoxyphenyl)carbamate |
| 103 | tert-butyl (4-(7-(4-acetamidopiperidin-1-yl)-4-aminopyrrolo[2,1-f][1,2,4]triazin-5-yl)-2-methoxyphenyl)carbamate |
| 104 | tert-butyl (4-(4-amino-7-(4-(aminomethyl)-3,3-dimethylpiperidin-1-yl)pyrrolo[2,1-f][1,2,4]triazin-5-yl)-2-methoxyphenyl)carbamate |
| 105 | tert-butyl (4-(4-amino-7-(4-methyl-4-(methylamino)piperidin-1-yl)pyrrolo[2,1-f][1,2,4]triazin-5-yl)-2-methoxyphenyl)carbamate |
| 106 | tert-butyl (4-(4-amino-7-(4-(2-aminopropan-2-yl)piperidin-1-yl)pyrrolo[2,1-f][1,2,4]triazin-5-yl)-2-methoxyphenyl)carbamate |
| 107 | tert-butyl (4-(4-amino-7-(4-(2-aminoethyl)piperidin-1-yl)pyrrolo[2,1-f][1,2,4]triazin-5-yl)-2-methoxyphenyl)carbamate |
| 108 | tert-butyl (4-(4-amino-7-(4-(2-(methylamino)ethyl)piperidin-1-yl)pyrrolo[2,1-f][1,2,4]triazin-5-yl)-2-methoxyphenyl)carbamate |
| 109 | tert-butyl (4-(4-amino-7-(4-(2-(dimethylamino)ethyl)piperidin-1-yl)pyrrolo[2,1-f][1,2,4]triazin-5-yl)-2-methoxyphenyl)carbamate |
| 110 | tert-butyl (4-(4-amino-7-((((1S,3R)-3-aminocyclopentyl)methyl)amino)pyrrolo[2,1-f][1,2,4]triazin-5-yl)-2-methoxyphenyl)carbamate |
| 111 | tert-butyl (4-(4-amino-7-(((1R,3S)-3-(aminomethyl)cyclopentyl)amino)pyrrolo[2,1-f][1,2,4]triazin-5-yl)-2-methoxyphenyl)carbamate |
| 112 | tert-butyl (4-(4-amino-7-(((1S,3S)-3-(dimethylamino)cyclopentyl)amino)pyrrolo[2,1-f][1,2,4]triazin-5-yl)-2-methoxyphenyl)carbamate |
| 113 | tert-butyl (4-(4-amino-7-(((1S,3R)-3-aminocyclopentyl)amino)pyrrolo[2,1-f][1,2,4]triazin-5-yl)-2-methoxyphenyl)carbamate |
| 114 | tert-butyl (4-(4-amino-7-(((1R,3S)-3-aminocyclopentyl)amino)pyrrolo[2,1-f][1,2,4]triazin-5-yl)-2-methoxyphenyl)carbamate |
| 115 | tert-butyl (4-(4-amino-7-(((1R,3R)-3-aminocyclopentyl)amino)pyrrolo[2,1-f][1,2,4]triazin-5-yl)-2-methoxyphenyl)carbamate |
| 116 | tert-butyl (4-(4-amino-7-(((1S,3S)-3-aminocyclopentyl)amino)pyrrolo[2,1-f][1,2,4]triazin-5-yl)-2-methoxyphenyl)carbamate |
| 117 | tert-butyl (4-(4-amino-7-((piperidin-4-ylmethyl)amino)pyrrolo[2,1-f][1,2,4]triazin-5-yl)-2-methoxyphenyl)carbamate |
| 118 | tert-butyl (4-(4-amino-7-(((1S,3S)-3-aminocyclobutyl)amino)pyrrolo[2,1-f][1,2,4]triazin-5-yl)-2-methoxyphenyl)carbamate |
| 119 | tert-butyl (4-(4-amino-7-(((1R,3R)-3-aminocyclobutyl)amino)pyrrolo[2,1-f][1,2,4]triazin-5-yl)-2-methoxyphenyl)carbamate |
| 120 | tert-butyl (4-(4-amino-7-(piperidin-4-ylamino)pyrrolo[2,1-f][1,2,4]triazin-5-yl)-2-methoxyphenyl)carbamate |
| 121 | tert-butyl (4-(4-amino-7-(((1S,4S)-4-aminocyclohexyl)amino)pyrrolo[2,1-f][1,2,4]triazin-5-yl)-2-methoxyphenyl)carbamate |
| 122 | tert-butyl (4-(4-amino-7-(((1S,4S)-4-aminocyclohexyl)amino)pyrrolo[2,1-f][1,2,4]triazin-5-yl)-2-methoxyphenyl)carbamate |
| 123 | tert-butyl (4-(4-amino-7-(4-(aminomethyl)-4-methylpiperidin-1-yl)pyrrolo[2,1-f][1,2,4]triazin-5-yl)-2-methoxyphenyl)carbamate |
| 124 | tert-butyl (4-(4-amino-6-chloro-7-(4-(dimethylamino)piperidin-1-yl)pyrrolo[2,1-f][1,2,4]triazin-5-yl)-2-methoxyphenyl)carbamate |
| 125 | tert-butyl (4-(4-amino-7-(4-aminopiperidin-1-yl)-6-(trifluoromethyl)pyrrolo[2,1-f][1,2,4]triazin-5-yl)-2-methoxyphenyl)carbamate |
| 126 | tert-butyl (4-(4-amino-7-(4-aminopiperidin-1-yl)-6-iodopyrrolo[2,1-f][1,2,4]triazin-5-yl)-2-methoxyphenyl)carbamate |
| 127 | tert-butyl (4-(4-amino-7-(4-aminopiperidin-1-yl)-6-fluoropyrrolo[2,1-f][1,2,4]triazin-5-yl)-2-methoxyphenyl)carbamate |
| 128 | tert-butyl (4-(4-amino-7-(4-(dimethylamino)piperidin-1-yl)-6-fluoropyrrolo[2,1-f][1,2,4]triazin-5-yl)-2-methoxyphenyl)carbamate |
| 129 | tert-butyl (4-(4-amino-6-fluoro-7-(4-(methylamino)piperidin-1-yl)pyrrolo[2,1-f][1,2,4]triazin-5-yl)-2-methoxyphenyl)carbamate |
| 130 | tert-butyl (4-(4-amino-6-fluoro-7-(2-(4-(methylamino)piperidin-1-yl)ethyl)pyrrolo[2,1-f][1,2,4]triazin-5-yl)-2-methoxyphenyl)carbamate |
| 131 | tert-butyl (4-(4-amino-6-fluoro-7-(4-(2-(methylamino)ethyl)piperidin-1-yl)pyrrolo[2,1-f][1,2,4]triazin-5-yl)-2-methoxyphenyl)carbamate |
| 132 | tert-butyl (4-(1-amino-6-(4-aminopiperidin-1-yl)pyrrolo[1,2-a]pyrazin-8-yl)-2-methoxyphenyl)carbamate |
| 133 | tert-butyl (4-(1-amino-6-(4-(methylamino)piperidin-1-yl)pyrrolo[1,2-a]pyrazin-8-yl)-2-methoxyphenyl)carbamate |
| 134 | tert-butyl (4-(1-amino-6-(4-(dimethylamino)piperidin-1-yl)pyrrolo[1,2-a]pyrazin-8-yl)-2-methoxyphenyl)carbamate |
| 135 | 7-(4-aminopiperidin-1-yl)-5-(4-phenoxyphenyl)pyrrolo[2,1-f][1,2,4]triazin-4-amine |
| 136 | 7-(4-(dimethylamino)piperidin-1-yl)-5-(4-phenoxyphenyl)pyrrolo[2,1-f][1,2,4]triazin-4-amine |
| 137 | N-(4-(4-amino-7-(4-aminopiperidin-1-yl)pyrrolo[2,1-f][1,2,4]triazin-5-yl)-2-methoxyphenyl)furan-2-carboxamide |
| 138 | N-(4-(4-amino-7-(4-aminopiperidin-1-yl)pyrrolo[2,1-f][1,2,4]triazin-5-yl)-2-methoxyphenyl)furan-3-carboxamide |

In one embodiment of the present invention, the present invention may provide a pharmaceutical composition for preventing or treating YES1-associated disease, comprising the pyrrolotriazine derivative compound or a pharmaceutically acceptable salt thereof.

In one embodiment of the present invention, the present invention may provide a pharmaceutical composition for preventing or treating Transcriptional Enhanced Associated Domain (TEAD)-associated disease, comprising the pyrrolotriazine derivative compound or a pharmaceutically acceptable salt thereof.

In one embodiment of the present invention, the YES1-associated disease and transcriptional enhanced associated domain-associated disease comprise malignant cells that express the YES1 kinase and hyperproliferative malignant cells in which cell growth is not suppressed due to the activation of the transcriptional enhanced associated domain, such as cancer. In some embodiments, the cancer comprise a solid tumor.

In one embodiment of the present invention, the present invention may provide a pharmaceutical composition for preventing or treating solid cancer, comprising the pyrrolotriazine derivative compound or a pharmaceutically acceptable salt thereof.

In one embodiment of the present invention, the present invention may provide a pharmaceutical composition for preventing or treating solid cancer, wherein the solid cancer is selected from the group consisting of thyroid cancer, bladder cancer, breast cancer, colorectal cancer, small intestine cancer, endometrial cancer, cervical cancer, bone cancer, gastric cancer, lymphoma, bronchial cancer, laryngeal cancer, head and neck cancer, liver cancer, prostate cancer, pancreatic cancer, ovarian cancer, esophageal cancer, kidney cancer, lung cancer, oral cancer, brain cancer, brain tumor, glioma, gallbladder cancer, cholangiocarcinoma, skin cancer, skin melanoma, rectal cancer, colon cancer, uterine cancer, uterine sarcoma, and malignant mesothelioma.

In the present invention, the term "pharmaceutical composition" may refer to a composition comprising a molecule or compound that provides several beneficial pharmaceutical effects when administered to a subject. The beneficial pharmaceutical effects may comprise enabling diagnostic determinations; improving a disease, symptom, disorder or condition; reducing or preventing the onset of a disease, symptom, disorder or condition; and generally responding to a disease, symptom, disorder or condition.

The pharmaceutical composition may be formulated into any one preparation form selected from the group consisting of tablets, soft or hard capsules, pills, powders, suspensions, syrups, injections and granules.

The pharmaceutical composition may be intended for oral or parenteral administration.

The concentration of the active ingredient in the pharmaceutical composition may be from 0.00001 to 9 wt% based on the total weight of the composition. For example, 0.00001 wt% or more, 0.0001 wt% or more, 0.001 wt% or more, 0.01 wt% or more, and 9 wt% or less, 8 wt% or less, 7 wt% or less, 6 wt% or less, 5 wt% or less, 4 wt% or less, 3 wt% or less, 2 wt% or less, or 1 wt% or less. In one embodiment of the present specification, the concentration of the active ingredient may be preferably from 0.0001 to 5 wt%, more preferably from 0.001 to 3 wt%.

The pharmaceutical composition may comprise conventional fillers, bulking agents, binders, disintegrants, anticoagulants, lubricants, wetting agents, pH regulators, nutrients, vitamins, electrolytes, alginic acid and its salts, pectic acid and its salts, protective colloids, glycerin, flavoring agents, emulsifiers, or preservatives. The formulation of the pharmaceutical composition may vary depending on the method of use, and may be formulated using methods well known in the art to which the present invention pertains so as to provide rapid, sustained, or delayed release of the active ingredient after administration to a mammal.

One embodiment of the present invention provides a method for preventing, improving, or treating a condition of a subject, comprising administering the pharmaceutical composition to the subject. The administration may be administered by methods known in the art. The administration may be administered directly to a subject by any means, for example, intravenously, intramuscularly, orally, transdermally, mucosally, intranasally, intratracheally, or subcutaneously. The administration may be systemic or local.

The composition according to one embodiment may be administered to a subject in an amount of 0.00001 mg to 1,000 mg per day, for example, from 0.00001 mg to 500 mg, from 0.00001 mg to 100 mg, from 0.00001 mg to 50 mg, from 0.00001 mg to 25 mg, from 1 mg to 1,000 mg, from 1 mg to 500 mg, from 1 mg to 100 mg, from 1 mg to 50 mg, from 1 mg to 25 mg, from 5 mg to 1,000 mg, from 5 mg to 500 mg, from 5 mg to 100 mg, from 5 mg to 50 mg, from 5 mg to 25 mg, from 10 mg to 1,000 mg, from 10 mg to 500 mg, from 10 mg to 100 mg, from 10 mg to 50 mg, or from 10 mg to 25 mg. However, the dosage may be prescribed in various ways depending on factors such as the formulation method, administration method, patient's age, weight, sex, pathological condition, food, administration time, administration route, excretion rate, and response sensitivity. A person skilled in the art may appropriately adjust the dosage in consideration of these factors. The frequency of administration may be once a day or twice or more times a day within the range of clinically acceptable side effects. The site of administration may be one or two or more sites. The total number of administration days may range from 1 to 30 days for one treatment, daily or at intervals of 2 to 5 days. If necessary, the same treatment may be repeated after an appropriate period. For non-human animals, the same dosage per kg as for humans may be administered, or a dosage converted from the above dosage based on the volume ratio (e.g., average value) of specific organs (e.g., the heart) between the target animal and a human may be administered.

### Use for preventing or treating

The novel pyrrolotriazine derivative compound or a pharmaceutically acceptable salt thereof, according to the present invention, inhibits the activity of YES1 kinase and transcriptional enhanced associated domain (TEAD), and thus exhibits a preventive or therapeutic effect against cancers related to these activities.

In one embodiment of the present invention, the compound of the present invention may be used to prevent or treat YES1 kinase-associated diseases and transcriptional enhanced associated domain (TEAD)-associated diseases.

In one embodiment of the present invention, the YES1-associated diseases and transcriptional enhanced associated domain-associated diseases comprise malignant cells that express YES1 kinase and hyperproliferative malignant cells in which cell growth is not suppressed due to activation of the transcriptional enhanced associated domain, such as cancer.

In one embodiment of the present invention, a use of the compound or a pharmaceutically acceptable salt thereof, according to the present invention, for preventing or treating solid cancer is provided. In a specific embodiment, the solid cancer may be thyroid cancer, bladder cancer, breast cancer, colon cancer, small intestine cancer, endometrial cancer, cervical cancer, bone cancer, gastric cancer, lymphoma, bronchial cancer, laryngeal cancer, head and neck cancer, liver cancer, prostate cancer, pancreatic cancer, ovarian cancer, esophageal cancer, kidney cancer, lung cancer, oral cancer, brain cancer, brain tumor, glioma, gallbladder cancer, cholangiocarcinoma, skin cancer, skin melanoma, rectal cancer, colon cancer, uterine cancer, uterine sarcoma, and malignant mesothelioma, but is not limited thereto.

Hereinafter, the present invention will be described in more detail through examples. These examples are only intended to more specifically explain the present invention, and it will be apparent to those skilled in the art that the scope of the present invention is not limited by these examples according to the gist of the present invention.

### Preparation Example of Intermediate

### Intermediate 1: Preparation of 2-fluoro-N,N-dimethyl-4-(4,4,5,5-tetramethyl-1,3,2-dioxaborolan-2-yl)aniline

The title compound was prepared according to the method shown in Reaction Formula 1 below.

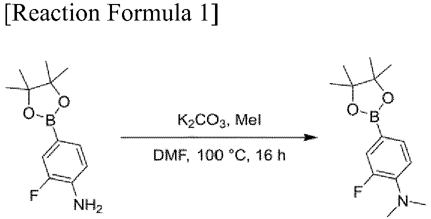

2-fluoro-4-(4,4,5,5-tetramethyl-1,3,2-dioxaborolan-2-yl)aniline (1.0 eq), K₂CO₃ (3.0 eq) and MeI (2.5 eq) were dissolved in DMF, and the mixture was stirred at 100 °C for 16 hours. The reaction mixture was cooled to room temperature, followed by addition of water and extraction of the organic compounds with ethyl acetate. The organic layer was washed with brine, the residual water in the organic layer was removed with Na₂SO_{4,} followed by concentration. The concentrate was purified by silica gel column (Petroleum ether/Ethyl acetate (B: 0-5% concentration gradient)). The target compound was obtained as a colorless oil and was actually used in the preparation of Example 4 ₍yield: 76.0%, LCMS: m/z (M+H)+= 266.1).

### Intermediate 2: Preparation of 2-methoxy-N,N-di(methyl)-4-[4,4,5,5-tetra(methyl)-1,3,2-dioxaborolan-2-yl]aniline

The title compound was prepared according to the method shown in Reaction Formula 2 below.

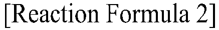

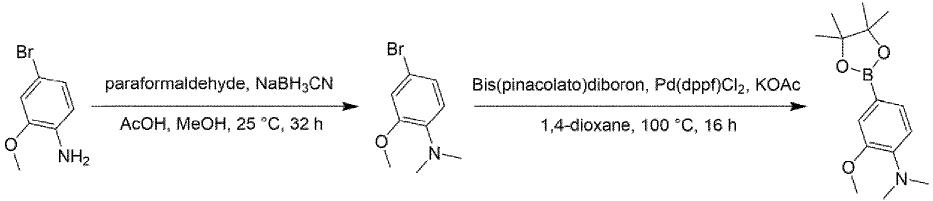

### Step 1: Preparation of 4-bromo-2-methoxy-NN-dimethylaniline

4-bromo-2-methoxyaniline (1.0 eq) and 37% paraformaldehyde (3.0 eq) were dissolved in methanol, followed by addition of acetic acid (0.1 eq). The mixture was stirred at room temperature under nitrogen for 2 hours. NaBH₃CN (1.5 eq) was added portionwise to the reaction mixture, and the mixture was stirred at room temperature for 32 hours. After completion of the reaction, sat. aqueous NaHCO₃ was added and the organic compounds were extracted with ethyl acetate. The organic layer was washed with brine, and the residual water in the organic layer was removed with Na₂SO₄, followed by concentration. The concentrate was purified by silica gel column (Petroleum ether/Ethyl acetate (B: 1-5% concentration gradient)). The target compound 4-bromo-2-methoxy-N,N-dimethylaniline was obtained as a colorless oil (yield: 79.0%, LCMS: m/z (M+H)+= 229.9).

### Step 2: Preparation of 2-methoxy-N,N-dimethyl-4-(4,4,5,5-tetramethyl-1,3,2-dioxaborolan-2-yl)aniline

4-bromo-2-methoxy-N,N-dimethylaniline (1.0 eq), prepared in step 1, was dissolved in 1,4-dioxane, followed by addition of B2pin2 (1.2 eq), Pd(dppf)Cl₂ (0.1 eq), and KOAc (2.0 eq). The mixture was stirred at 100°C under nitrogen for 16 hours. After completion of the reaction, the mixture was concentrated under reduced pressure, and the concentrate was purified by silica gel column (Petroleum ether/Ethyl acetate (B: 1-6% concentration gradient)). The target compound was obtained as a yellow oil and was actually used in the preparation of Example 6 (yield: 83.0%, LCMS: m/z (M+H)+= 278.2).

### Intermediate 3: Preparation of N,N,4-trimethylpiperidin-4-amine

The title compound was prepared according to the method shown in Reaction Formula 3 below.

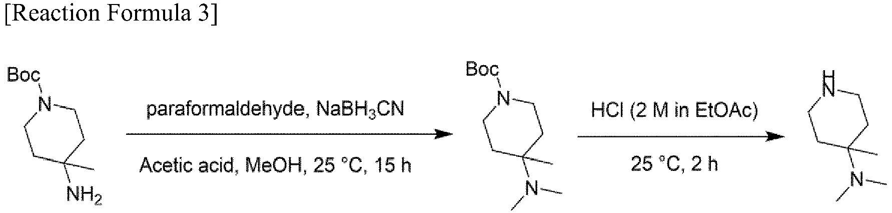

### Step 1: Preparation of tert-butyl 4-(dimethylamino)-4-methylpiperidin-1-carboxylate

tert-butyl 4-amino-4-methylpiperidin-1-carboxylate (1.0 eq) and 37% paraformaldehyde (3.0 eq) were dissolved in acetic acid (0.1 eq) and methanol, and then the mixture was stirred for 1 hour. The reaction mixture was cooled to 0°C, and NaBH₃CN (2.0 eq) was added. The mixture was stirred at room temperature for 15 hours. After completion of the reaction, sat. aqueous NaHCO₃ was added, and the organic compounds were extracted with ethyl acetate. The organic layer was washed with brine, and the residual water in the organic layer was removed with Na₂SO₄, followed by concentration. The concentrate was purified by silica gel column (Petroleum ether/Ethyl acetate (B: 60-85% concentration gradient)). The target compound tert-butyl 4-(dimethylamino)-4-methylpiperidin-1-carboxylate was obtained as a colorless oil (yield: 32.4%, LCMS: m/z (M+H)+= 243.2).

### Step 2: Preparation of N,N,4-trimethylpiperidin-4-amine

HCl(2M in dioxane) was added to tert-butyl 4-(dimethylamino)-4-methylpiperidin-1-carboxylate (1.0 eq), prepared in step 1, and the mixture was stirred at room temperature for 2 hours. The reaction mixture was concentrated under reduced pressure to obtain a yellow solid without additional purification, which was actually used in the preparation of Example 13 (yield: 100%, LCMS: m/z (M+H)+= 143.3).

In the case of Examples 18, 30, and 32, the intermediates were also prepared using the same method.

### Preparation of Example

### Example 1: Preparation of tert-butyl (4-(4-amino-7-(4-(dimethylamino)phenyl)pyrrolo[2,1-f][1,2,4]triazin-5-yl)-2-methoxyphenyl)carbamate

The title compound was prepared according to the method shown in Reaction Formula 4 below.

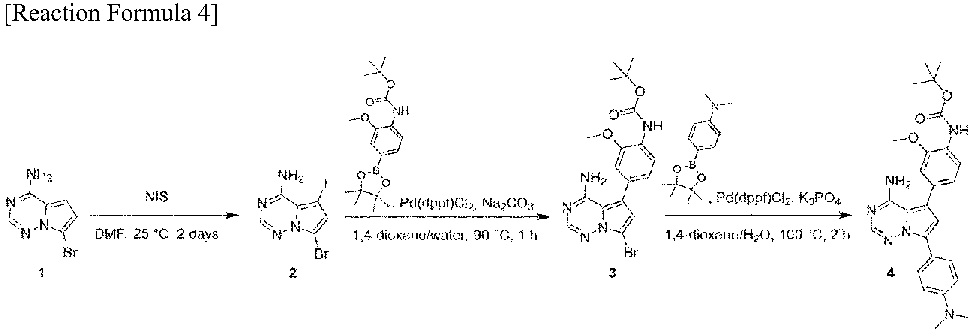

### Step 1: Preparation of 7-bromanyl-5-iodanyl-pyrrolo[2,1-f][1,2,4]triazin-4-amine

7-bromanylpyrrolo[2,1-f][1,2,4]triazin-4-amine (1.0 eq) was dissolved in DMF, followed by addition of NIS (1.4 eq). The mixture was stirred at room temperature for two days. After completion of the reaction, the precipitate was filtered and washed with ACN to obtain the target compound 7-bromanyl-5-iodanyl-pyrrolo[2,1-f][1,2,4]triazin-4-amine as a white solid without additional purification (yield: 80.7%, LCMS: m/z (M+H)+= 338.9).

### Step 2: Preparation of 1,1-di(methyl)ethyl N-[4-(4-azanyl-7-bromanyl-pyrrolo[2,1-f][1,2,4]triazin-5-yl)-2-methoxy-phenyl]carbamate

7-bromanyl-5-iodanyl-pyrrolo[2,1-f][1,2,4]triazin-4-amine (1.0 eq), prepared in step 1, and 4-(tert-butoxycarbonylamino)-3-methoxyphenylboronic acid pinacol ester (1.1 eq) were dissolved in 1,4-dioxane and water (5:1). Pd(dppf)Cl₂ (0.1 eq), followed by addtion of Na₂CO₃ (2.0 eq).The mixture was stirred at 90°C under nitrogen for 1 hour. After completion of the reaction, the reaction mixture was concentrated under reduced pressure, and the concentrate was purified by silica gel column (Petroleum ether/Ethyl acetate (B: 15-50% concentration gradient)). The target compound 1,1-di(methyl)ethyl N-[4-(4-azanyl-7-bromanyl-pyrrolo[2,1-f][1,2,4]triazin-5-yl)-2-methoxy-phenyl]carbamate was obtained as a yellow solid (yield: 74.1%, LCMS: m/z (M+H)⁺= 434.1).

### Step 3: Preparation of tert-butyl (4-(4-amino-7-(4-(dimethylamino)phenyl)pyrrolo[2,1-f][1,2,4]triazin-5-yl)-2-methoxyphenyl)carbamate

1,1-di(methyl)ethyl N-[4-(4-azanyl-7-bromanyl-pyrrolo[2,1-f][1,2,4]triazin-5-yl)-2-methoxy-phenyl]carbamate (1.0 eq), prepared in step 2, and 4-(N,N-dimethylamino)phenylboronic acid pinacol ester (1.2 eq) were dissolved in 1,4-dioxane and water (5:1). Pd(dppf)Cl₂ (0.1 eq) and K₂PO₄ (3.0 eq) were added, and the mixture was stirred at 100°C under nitrogen for 2 hours. After completion of the reaction, water was added to the reaction mixture, and the organic compounds were extracted with ethyl acetate. The organic layer was washed with brine, and the residual water in the organic layer was removed with Na₂SO_{4,} followed by concentration. After filtering, the filtrate was concentrated, and the concentrate was purified by silica gel column (Petroleum ether/Ethyl acetate (1:1)). The target compound tert-butyl (4-(4-amino-7-(4-(dimethylamino)phenyl)pyrrolo[2,1-f][1,2,4]triazin-5-yl)-2-methoxyphenyl)carbamate was obtained as a yellow solid (yield: 28.3%, LCMS: m/z (M+H)⁺= 475.2).

### Examples 2 to 9: Preparation of compounds 2 to 9

The compounds of Examples 2 to 9 according to the present invention were prepared using a method similar to that of Example 1.

### Example 10: Preparation of tert-butyl(R)-(4-(4-amino-7-(3-(dimethylamino)pyrrolidin-1-yl)pyrrolo[2,1-f][1,2,4]triazin-5-yl)-2-methoxyphenyl)carbamate

The title compound was prepared according to the method shown in Reaction Formula 5 below.

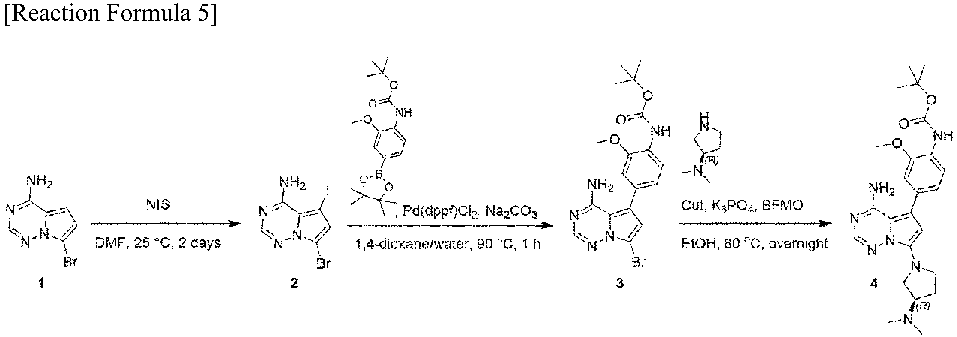

**Steps 1 and 2 were prepared by the same method as those of Example 1.**

### Step 3: Preparation of tert-butyl(R)-(4-(4-amino-7-(3-(dimethylamino)pyrrolidin-1-yl)pyrrolo[2,1-f][1,2,4]triazin-5-yl)-2-methoxyphenyl)carbamate

1,1-di(methyl)ethyl N-[4-(4-azanyl-7-bromanyl-pyrrolo[2,1-f][1,2,4]triazin-5-yl)-2-methoxy-phenyl]carbamate (1.0 eq), prepared in step 2, and (3R)-*N,N*-dimethylpyrrolidin-3-amine (3.0 eq) were dissolved in ethanol, followed by addition of CuI (0.2 eq), K₃PO₄ (2.0 eq), and *N,N'*-bis(2-furylmethyl)oxamide (0.2 eq). The mixture was stirred at 80°C under nitrogen for 24 hours. After completion of the reaction, the reaction mixture was concentrated under reduced pressure, and the concentrate was purified by silica gel column (Ethyl acetate/Methanol (B: 5-20% concentration gradient)). The target compound tert-butyl(R)-(4-(4-amino-7-(3-(dimethylamino)pyrrolidin-1-yl)pyrrolo[2,1-f][1,2,4]triazin-5-yl)-2-methoxyphenyl)carbamate was obtained as a yellow solid (yield: 3.35%, LCMS: m/z (M+H)⁺= 468.3).

### Preparation of Examples 11, 13, 17, 19, 21 and 22

The compounds of Example 11, 13, 17, 19, 21 and 22 according to the present invention were prepared using a method similar to that of Example 10.

### Example 12: Preparation of tert-butyl (4-(4-amino-7-(4-aminopiperidin-1-yl)pyrrolo[2,1-f][1,2,4]triazin-5-yl)-2-methoxyphenyl)carbamate

The title compound was prepared according to the method shown in Reaction Formula 6 below.

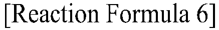

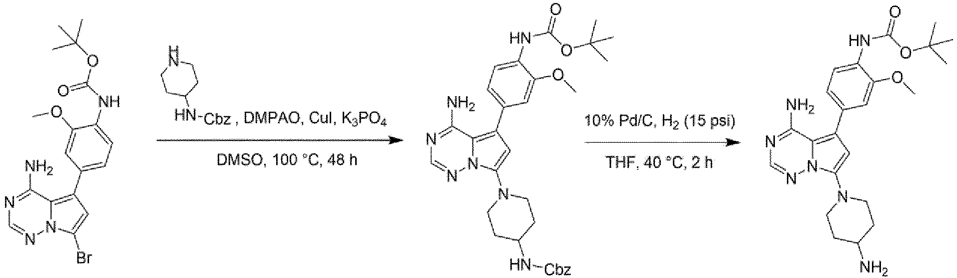

### Step 1: Preparation of tert-butyl (4-(4-amino-7-(4-(((benzyloxy)carbonyl)amino)piperidin-1-yl)pyrrolo[2,1-f][1,2,4]triazin-5-yl)-2-methoxyphenyl)carbamate

tert-butyl (4-(4-amino-7-bromopyrrolo[2,1-f][1,2,4]triazin-5-yl)-2-methoxyphenyl)carbamate (1.0 eq) and benzyl piperidin-4-ylcarbamate (1.0 eq) were dissolved in DMSO, followed by addition of 2-((2,6-dimethylphenyl)amino)-2-oxoacetic acid (0.2 eq), CuI (0.2 eq), and K₃PO₄ (2.0 eq). The reaction mixture was stirred at 100°C under nitrogen for 48 hours. After completion of the reaction, sat. aqueous NaHCO₃ was added and the organic compounds were extracted with ethyl acetate. The organic layer was washed with brine, and the residual water was removed with Na₂SO₄, followed by concentration. The concentrate was purified by silica gel column (Ethyl acetate/Methanol (B: 5-20% concentration gradient)). The target compound tert-butyl (4-(4-amino-7-(4-(((benzyloxy)carbonyl)amino)piperidin-1-yl)pyrrolo[2,1-f][1,2,4]triazin-5-yl)-2-methoxyphenyl)carbamate was obtained as a yellow solid (yield: 13.6%, LCMS: m/z (M+H)+= 588.3).

### Step 2: Preparation of tert-butyl (4-(4-amino-7-(4-aminopiperidin-1-yl)pyrrolo[2,1-f][1,2,4]triazin-5-yl)-2-methoxyphenyl)carbamate

tert-butyl (4-(4-amino-7-(4-(((benzyloxy)carbonyl)amino)piperidin-1-yl)pyrrolo[2,1-f][1,2,4]triazin-5-yl)-2-methoxyphenyl)carbamate (1.0 eq), prepared in step 1, was dissolved in THF, followed by addition of 10% Pd/C. The reaction mixture was stirred at 40°C under hydrogen (15 psi) for 2 hours. The mixture was filtered, concentrated, and purified by prep-HPLC (0.1% FA in water/ACN (B: 5-35% concentration gradient)). The target compound tert-butyl (4-(4-amino-7-(4-aminopiperidin-l-yl)pyrrolo[2,1-f][1,2,4]triazin-5-yl)-2-methoxyphenyl)carbamate was obtained as a yellow solid (yield: 39.9%, LCMS: m/z (M+H)+= 454.3).

### Example 14: preparation of tert-butyl (4-(4-amino-7-(4-(diethylamino)piperidin-1-yl)pyrrolo[2,1-f][1,2,4]triazin-5-yl)-2-methoxyphenyl)carbamate

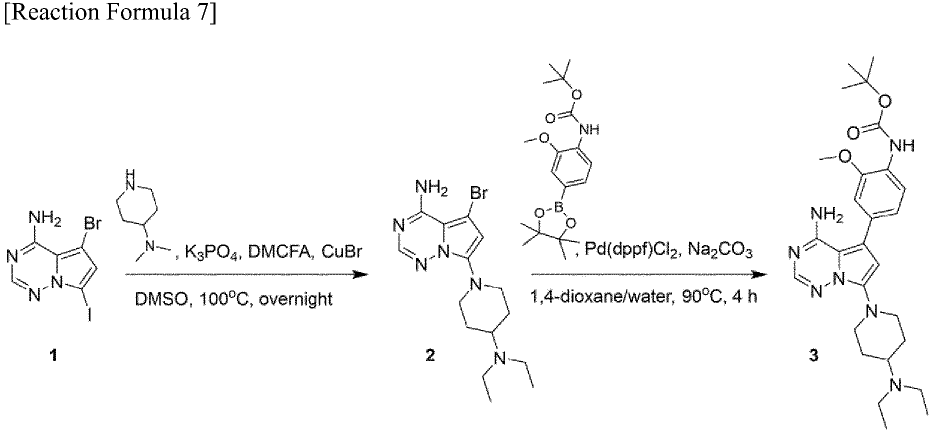

### Step 1: Preparation of 5-bromanyl-7-[4-[di(ethyl)aminol-1-piperidyl]pyrrolo[2,1-f][1,2,4]triazin-4-amine

5-bromanyl-7-iodanyl-pyrrolo[2,1-f][1,2,4]triazin-4-amine (1.0 eq), 4-diethylamino-piperidine (1.0 eq), and K₃PO₄(2.0 eq) were dissolved in DMSO, followed by addition of [(2,6-dimethylphenyl)carbamoyl]formic acid (0.2 eq) and CuBr (0.2 eq). The mixture was stirred at 100°C for 16 hours. After completion of the reaction, the reaction mixture was filtered, and DMSO was removed under reduced pressure. The concentrate was purified by silica gel column (Ethyl acetate/Methanol(B: 5-50% concentration gradient)). The target compound 5-bromanyl-7-[4-[di(ethyl)amino]-1-piperidyl]pyrrolo[2,1-f][1,2,4]triazin-4-amine was obtained as a yellow solid (yield: 30.8%, LCMS: m/z (M+H)⁺= 367.1).

### Step 2: Preparation of tert-butyl (4-(4-amino-7-(4-(diethylamino)piperidin-1-yl)pyrrolo[2,1-f][1,2,4]triazin-5-yl)-2-methoxyphenyl)carbamate

5-bromanyl-7-[4-[di(ethyl)amino]-1-piperidyl]pyrrolo[2,1-f][1,2,4]triazin-4-amine (1.0 eq), prepared in step 1, and 4-(tert-butoxycarbonylamino)-3-methoxyphenylboronic acid pinacol ester (1.0 eq) were dissolved in 1,4-dioxane and water (5:1), followed by addition of Pd(dppf)Cl₂ (0.1 eq) and Na₂CO₃ (2.0 eq). The mixture was stirred at 90°C under nitrogen for 4 hours. After completion of the reaction, the reaction mixture was concentrated under reduced pressure, and the concentrate was purified by prep-HPLC (0.1% FA in water/ACN (B: 8-45% concentration gradient)). The target compound tert-butyl (4-(4-amino-7-(4-(diethylamino)piperidin-1-yl)pyrrolo[2,1-f][1,2,4]triazin-5-yl)-2-methoxyphenyl)carbamate was obtained as a brown solid (yield: 10.5%, LCMS: m/z (M+H)+= 510.3).

### Preparation of Examples 15, 16, 18, 20, 24, 26 to 48 and 50 to 57

The compounds of Example 15, 16, 18, 20, 24, 26 to 48 and 50 to 57 according to the present invention were prepared using a method similar to that of Example 14.

### Example 23: Preparation of tert-butyl (4-(4-amino-7-(1,8-diazaspiro[4.5]decan-8-yl)pyrrolo[2,1-f][1,2,4]triazin-5-yl)-2-methoxyphenyl)carbamate (Example 25 was also prepared by the same method)

The title compound was prepared according to the method shown in Reaction Formula 8 below.

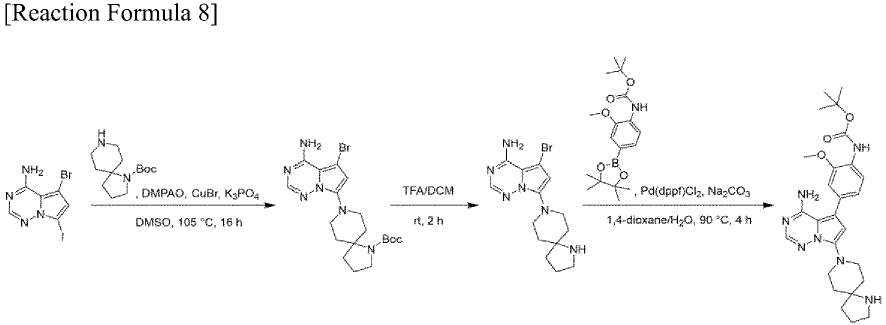

### Step 1: Preparation of tert-butyl 8-(4-amino-5-bromopyrrolo[2,1-f][1,2,4]triazin-7-yl)-1,8-diazaspiro[4.5]decan-1-carboxylate

5-bromanyl-7-iodanyl-pyrrolo[2,1-f][1,2,4]triazin-4-amine (1.0 eq) and tert-butyl 1,8-diazaspiro[4.5]decan-1-carboxylate (1.0 eq) were dissolved in DMSO, followed by addition of DMPAO (2-((2,6-Dimethylphenyl)amino)-2-oxoacetic Acid) (0.2 eq), CuI (0.2 eq), and K₃PO₄ (2.0 eq). The reaction mixture was stirred at 105°C under nitrogen for 16 hours. After completion of the reaction, the mixture was concentrated under reduced pressure, and the concentrate was purified by silica gel column (Petroleum ether/Ethyl acetate (B: 5-50% concentration gradient)). The target compound tert-butyl 8-(4-amino-5-bromopyrrolo[2,1-f][1,2,4]triazin-7-yl)-1,8-diazaspiro[4.5]decan-1-carboxylate was obtained as a yellow solid (yield: 30.0%, LCMS: m/z (M+H)+= 451.2).

### Step 2: Preparation of 5-bromo-7-(1,8-diazaspiro[4.5]decan-8-yl)pyrrolo[2,1-f][1,2,4]triazin-4-amine

tert-butyl 8-(4-amino-5-bromopyrrolo[2,1-f][1,2,4]triazin-7-yl)-1,8-diazaspiro[4.5]decan-1-carboxylate, prepared in step 1, was dissolved in DCM, followed by addition of TFA (1:1 ratio). The reaction mixture was stirred at room temperature for 2 hours and concentrated under reduced pressure. The target compound 5-bromo-7-(1,8-diazaspiro[4.5]decan-8-yl)pyrrolo[2,1-f][1,2,4]triazin-4-amine was obtained as a yellow oil without additional purification (LCMS: m/z (M+H)+= 351.2).

### Step 3: Preparation of tert-butyl (4-(4-amino-7-(1,8-diazaspiro[4.5]decan-8-yl)pyrrolo[2,1-f][1,2,4]triazin-5-yl)-2-methoxyphenyl)carbamate

5-bromo-7-(1,8-diazaspiro[4.5]decan-8-yl)pyrrolo[2,1-f][1,2,4]triazin-4-amine (1.0 eq), prepared in step 2, and 4-(tert-butoxycarbonylamino)-3-methoxyphenylboronic acid pinacol ester (1.1 eq) were dissolved in 1,4-dioxane and water (5:1). Pd(dppf)Cl₂ (0.1 eq) and Na₂CO₃ (3.0 eq) were added, and the mixture was stirred at 90°C under nitrogen for 4 hours. After completion of the reaction, the reaction mixture was concentrated under reduced pressure, and the concentrate was purified by prep-HPLC (0.1% FA in water/ACN (B: 10-40% concentration gradient)). The target compound tert-butyl (4-(4-amino-7-(1,8-diazaspiro[4.5]decan-8-yl)pyrrolo[2,1-f][1,2,4]triazin-5-yl)-2-methoxyphenyl)carbamate was obtained as a yellow solid (yield: 24.5%, LCMS: m/z (M+H)+= 494.2).

### Preparation of Examples 25, 49, 58, 59, 65, 66, 70, 71, 72, 73, 79 to 87, 89, 90, 91 to 97, 101 to 109 and 135 to 138

The compounds of Example 25, 49, 58, 59, 65, 66, 70, 71, 72, 73, 79 to 87, 89, 90, 91 to 97, 101 to 109 and 135 to 138 according to the present invention were prepared using a method similar to that of Example 23.

### Preparation of Examples 75 and 76

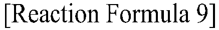

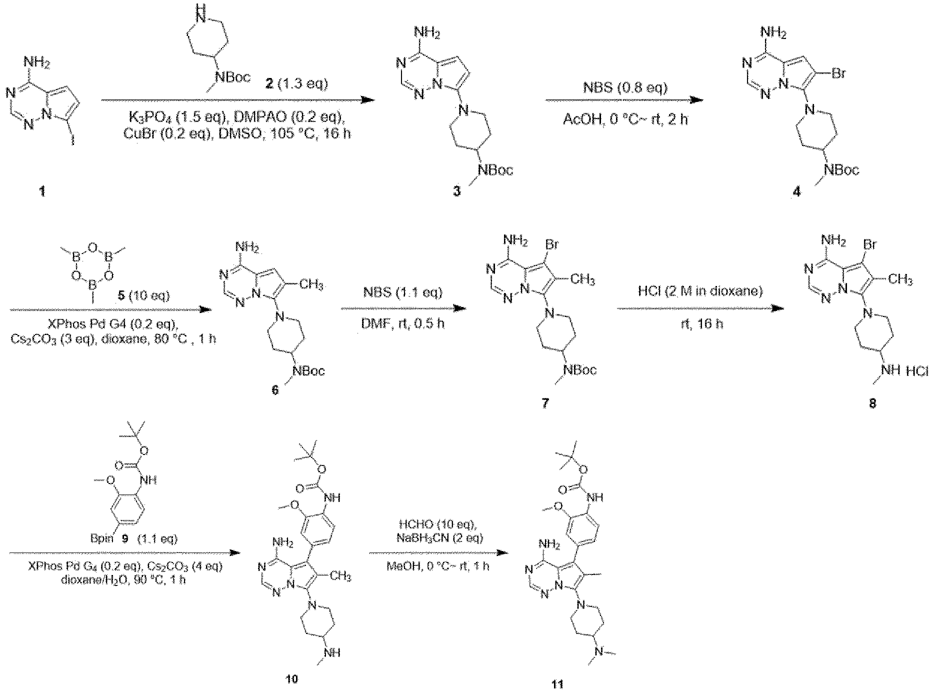

**Step 1:** 7-iodopyrrol[2,1-f][1,2,4]triazin-4-amine (25 g, 96.14 mmol), tert-butyl methyl(piperidin-4-yl)carbamate (1.3eq), K₃PO₄ (1.5eq) are dissolved in DMSO, followed by addition of 2-((2,6-dimethylphenyl)amino)-2-oxacetic acid (0.2) and CuBr (0.2eq). The reaction was carried out at 105°C for 16 hours. The reaction solution was concentrated under reduced pressure and purified by silica column chromatography (5-60% ethyl acetate in petroleum ether). The target compound tert-butyl (1-(4-aminopyrrolo[2,1-f][1,2,4]triazin-7-yl)piperidin-4-yl)(methyl)carbamate (13.51% yield) was obtained as a yellow solid. LCMS: m/z (M+H)⁺= 374.2.

**Step 2** : tert-butyl (1-(4-aminopyrrolo[2,1-f][1,2,4]triazin-7-yl)piperidin-4-yl)(methyl)carbamate (1eq) was dissolved in DMF and AcOH, followed by addition of NBS (0.8eq) at 0°C. The reaction solution was allowed to react at room temperature for 2 hours, followed by addition of water to quench the reaction. NaHCO₃ was added to adjust pH = 8-9, followed by extraction of the reaction mixture with ethyl acetate. The organic layer was evaporated under reduced pressure and the target compound tert-butyl (1-(4-amino-6-bromopyrrolo[2,1-f][1,2,4]triazin-7-yl)piperidin-4-yl)(methyl)carbamate (54.91% yield) was obtained as a yellow solid by column chromatography (5-60% ethyl acetate in petroleum ether). LCMS: m/z (M+H)⁺ = 425.0.

**Step 3:** tert-butyl (1-(4-amino-6-bromopyrrolo[2,1-f][1,2,4]triazin-7-yl)piperidin-4-yl)(methyl)carbamate (1eq), 2,4,6-trimethyl-1,3,5,2,4,6-trioxatriborinane (50% in THF) (10eq), Cs₂CO₃ (3eq) and XPhos Pd G4 (0.2eq) were dissolved in Dioxane. The reaction was carried out at 80°C for 1 hour. The reaction solution was concentrated under reduced pressure and the target compound tert-butyl (1-(4-amino-6-methylpyrrolo[2,1-f][1,2,4]triazin-7-yl)piperidin-4-yl)(methyl)carbamate (68.00% yield) was obtained as a yellow solid by column chromatography (5-80% ethyl acetate in petroleum ether). LCMS: m/z (M+H)⁺ = 361.1.

**Step 4 :** tert-butyl (1-(4-amino-6-methylpyrrolo[2,1-f][1,2,4]triazin-7-yl)piperidin-4-yl)(methyl)carbamate (1eq) was dissolved in DMF, followed by addition of NBS(1.1eq) at 0°C. The reaction was carried out at 25°C for 0.5 hours, followed by addition of sat. aq. NaHCO₃ and sat. aq. Na₂SO₃ to quench the reaction. Then the mixture was extracted with ethyl acetate. The organic layer was evaporated under reduced pressure, and the target compound tert-butyl (1-(4-amino-5-bromo-6-methylpyrazole[2,1-f][1,2,4]triazin-7-yl)piperidin-4-yl)(methyl)carbamate (91.69% yield) was obtained as a yellow solid by column chromatography (5-100% ethyl acetate in petroleum ether). LCMS: m/z (M+H)⁺ = 439.0.

**Step 5** : HCl (2M in dioxane, 10eq) was added to tert-butyl (1-(4-amino-5-bromo-6-methylpyrazole[2,1-f][1,2,4]triazin-7-yl)piperidin-4-yl)(methyl)carbamate (1eq), and the reaction was carried out at 25°C for 16 hours. The reaction mixture was evaporated under reduced pressure to obtain the target compound 5-bromo-6-methyl-7-(4-(methylamino)piperidin-1-yl)pyrrolo[2,1-f][1,2,4]triazin-4-amine (98.48% yield) as a yellow solid. LCMS: m/z (M+H)⁺ = 339.0.

**Step 6**: 5-bromo-6-methyl-7-(4-(methylamino)piperidin-1-yl)pyrrolo[2,1-f][1,2,4]triazin-4-amine (1eq), tert-butyl N-[2-methoxy-4-(4,4,5,5-tetramethyl-1,3,2-dioxaborolan-2-yl)phenyl]carbamate (1.1eq), Cs₂CO₃ (4eq) and XPhos Pd G4 (0.2eq) were dissolved in dioxane and water. The reaction was carried out at 90°C for 1 hour. Reaction mixture was evaporated under reduced pressure, and purified by column chromatography (5-50% methanol in ethyl acetate). The target compound tert-butyl (4-(4-amino-6-methyl-7-(4-(methylamino)piperidin-1-yl) pyrrolo[2,1-f][1,2,4]trizin-5-yl)-2-methoxyphenyl)carbamate was obtained as a yellow solid. LCMS: m/z (M+H)⁺ = 482.3.

**Step 7:** tert-butyl (4-(4-amino-6-methyl-7-(4-(methylamino)piperidin-1-yl)pyrrolo[2,1-f][1,2,4]triazin-5-yl)-2-methoxyphenyl)carbamate (1eq) and 37% aq. HCHO (10eq) was dissolved in MeOH, followed by addition of NaBH₃CN (2eq) at 0°C. The reaction mixture was allowed to react at 25°C for 1 hour. After completion of the reaction, the mixture was evaporated under reduced pressure, and purified by column chromatography (Eluent of 40% MeOH /Ethyl acetate) to obtain the target compound tert-butyl (4-(4-amino-7-(4-(dimethylamino)piperidin-1-yl)-6-methoxypyrrolo[2,1-f][1,2,4]triazin-5-yl)-2-methoxyphenyl)carbamate (57.26% yield) as a yellow solid. LCMS: m/z (M+H)⁺ = 496.3.

The compounds of Examples 60 to 63, 74 and 77 according to the present invention were prepared using a method similar to that of Example 76.

### Preparation of Example 110

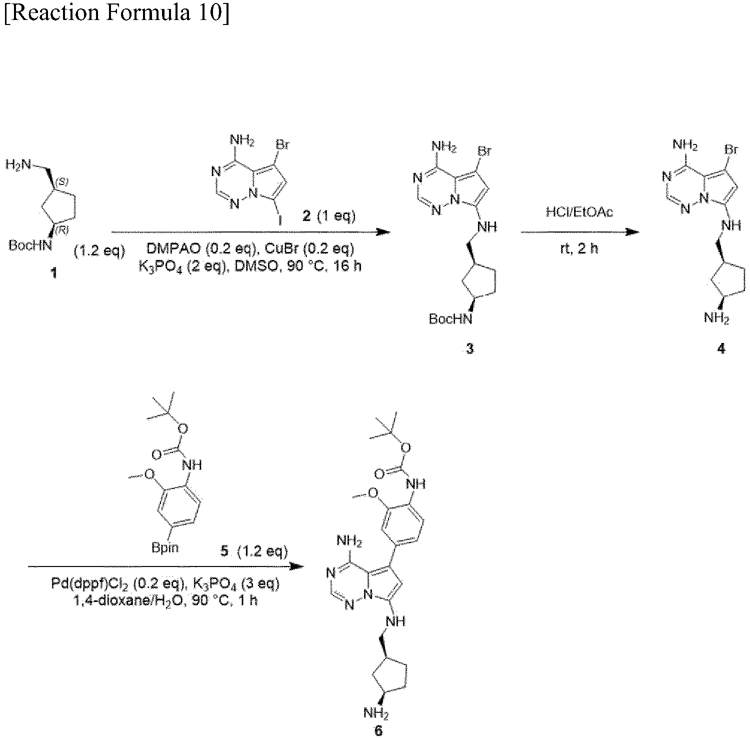

**Step 1** : 5-bromo-7-iodopyrrolo[2,1-f][1,2,4]triazin-4-amine (leq), tert-butyl ((1R,3S)-3-(aminomethyl)cyclopentyl)carbamate (1.2eq) and K₃PO₄ (2eq) were dissolved in DMSO,followed by addition of CuBr (0.2eq) and DMPAO (0.2eq). The reaction mixture was allowed to react at 90°C for 16 hours. The mixture was evaporated under reduced pressure and the target compound tert-butyl ((1R,3S)-3-(((4-amino-5-bromopyrrolo[2,1-f][1,2,4]triazin-7-yl)amino)methyl)cyclopentyl)carbamate (5.36% yield) was obtained as a yellow oil by column chromatography (Petroleum ether/Ethyl acetate = 1:2). LCMS: m/z (M+H)⁺ = 424.9.

**Step 2** : HCl (2.0 M in ethyl acetate, 10eq) was added to tert-butyl ((1R,3S)-3-(((4-amino-5-bromopyrrolo[2,1-f][1,2,4]triazin-7-yl)amino)methyl)cyclopentyl)carbamate (1eq) and the reaction was carried out at 25°C for 2 hours. The mixture was evaporated under reduced pressure to obtain the target compound N7-(((1S,3R)-3-aminocyclopentyl)methyl)-5-bromopyrrolo[2,1-f][1,2,4]triazin-4,7-diamine as a yellow solid. LCMS: m/z (M+H)⁺ = 325.0.

**Step 3** : N7-(((1S,3R)-3-aminocyclopentyl)methyl)-5-bromopyrrolo[2,1-f][1,2,4]triazin-4,7-diamine (1eq) and K₃PO₄ (3eq) were dissolved in dioxane and H₂O. Then tert-butyl N-[2-methoxy-4-(4,4,5,5-tetramethyl-1,3,2-dioxaborolan-2-yl)phenyl]carbamate (1.2eq) and Pd(dppf)Cl₂ (0.2eq) were added. The reaction solution was allowed to react at 90°C for 1 hour and evaporated under reduced pressure. Then the mixture was purified by column chromatography to obtain the target compound tert-butyl (4-(4-amino-7-((((1S,3R)-3-aminocyclopentyl)methyl)amino)pyrrolo[2,1-f][1,2,4]triazin-5-yl)-2-methoxyphenyl)carbamate (18.8% yield) as a yellow solid.

### Preparation of Examples 111, 112, 113, 114, 115, 116, 117, 118, 119, 120, 121 and 122

The compounds of Examples 111, 112, 113, 114, 115, 116, 117, 118, 119, 120, 121 and 122 according to the present invention were prepared using a method similar to that of Example 110.

### Preparation of Example 132

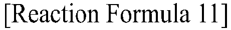

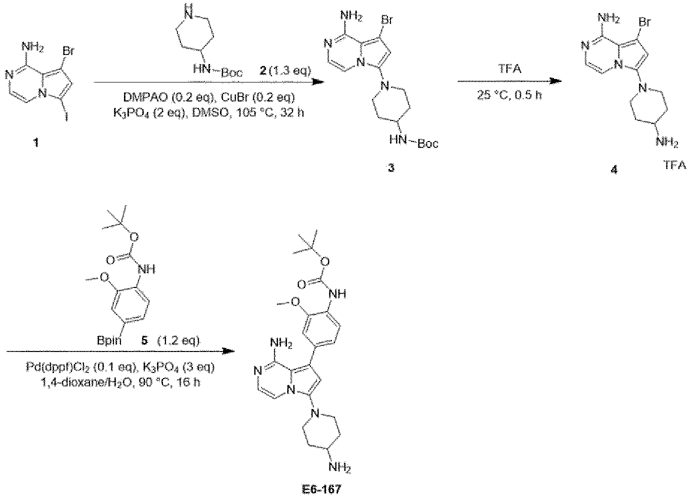

**Step 1** : 8-bromo-6-iodopyrrolo[1,2-a]pyrazin-1-amine (1eq), tert-butyl piperidin-4-ylcarbamate (1.3eq) and K₃PO₄ (2eq) were dissolved in DMSO, followed by addtion of 2-((2,6-dimethylphenyl)amino)-2-oxoacetic acid (0.2eq) and CuBr (0.2eq). The reaction mixture was allowed to react at 105°C for 32 hours. The reaction mixture was evaporated under reduced pressure, and the target compound tert-butyl (1-(1-amino-8-bromopyrrolo[1,2-a]pyrazin-6-yl)piperidin-4-yl)carbamate (14.21% yield) was obtained as a yellow solid by column chromatography (5-50% ethyl acetate in petroleum ether). LCMS: m/z (M+H)⁺ = 410.0.

**Step 2 :** tert-butyl (1-(1-amino-8-bromopyrrolo[1,2-a]pyrazin-6-yl)piperidin-4-yl)carbamate (1eq) was dissolved in TFA and DCM, and the mixture was allowed to react at 25°C for 0.5 hours. The reaction mixture was evaporated under reduced pressure, and the target compound 6-(4-aminopiperidin-1-yl)-8-bromopyrrolo[1,2-a]pyrazin-1-amine was obtained as a yellow solid. MS: m/z (M+H)⁺ = 309.9.

**Step 3:** 6-(4-amino-1-piperidyl)-8-bromo-pyrrolo[1,2-a]pyrazin-1-amine, tert-butyl (2-methoxy-4-(4,4,5,5-tetramethyl-1,3,2-dioxaborolan-2-yl)phenyl)carbamate (1.2eq), Pd(dppf)Cl₂ (0.1eq) and K₃PO₄ (3eq) were dissolved in dioxane and water. The mixture was allowed to react at 90°C for 16 hours. The reaction mixture was dried under reduced pressure, and the target compound tert-butyl (4-(1-amino-6-(4-aminopiperidin-1-yl)pyrrolo[1,2-a]pyrazin-8-yl)-2-methoxyphenyl)carbamate (8.24% yield) was obtained as a yellow solid by column chromatography(5-20% methanol in ethyl acetate). LCMS: m/z (M+H)⁺ = 453.3.

The compounds of Examples 133, 134 according to the present invention were prepared using a method similar to that of Example 132.

### Preparation of Example 98

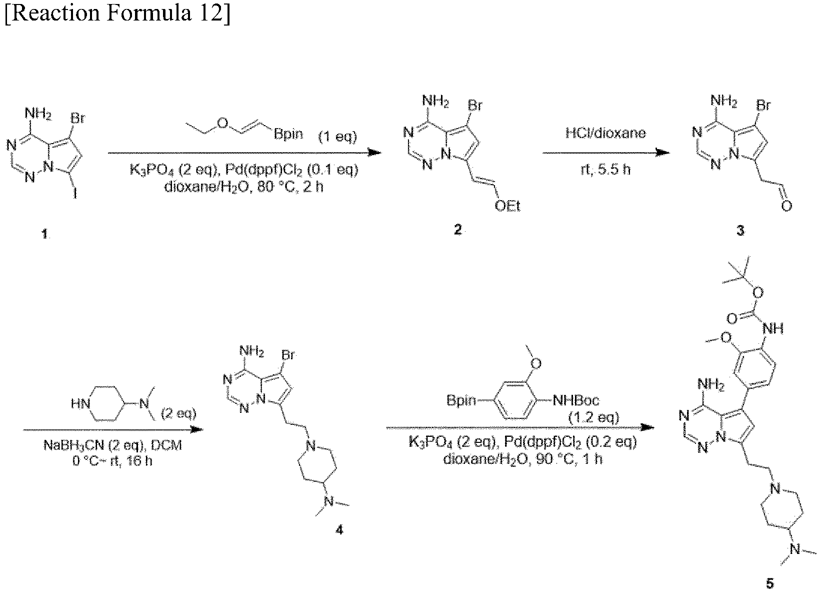

**Step 1**: 5-bromo-7-iodopyrrolo[2,1-f][1,2,4]triazin-4-amine (1eq) and (E)-2-(2-ethoxyvinyl)-4,4,5,5-tetramethyl-1,3,2-dioxaborolane (1eq) were dissolved in dioxane and H₂O, followed by addtion of K₃PO₄ (2eq) and Pd(dppf)Cl₂ (0.1eq) were added. The reaction solution was allowed to react at 80°C for 2 hours. H₂O was added to the reaction solution, and the mixture was extracted with EtOAc. The organic layer was evaporated under reduced pressure, and purified by column chromatography (Petroleum ether/Ethyl acetate = 1:1 to 0:1) to obtain the target compound (E)-5-bromo-7-(2-ethoxyvinyl)pyrrolo[2,1-f][1,2,4]triazin-4-amine as a yellow solid. LCMS: m/z (M+H)⁺ = 282.9.

**Step 2** : HCl (2.0 M in 1,4-dioxane, 10eq) was added to (E)-5-bromo-7-(2-ethoxyvinyl)pyrrolo[2,1-f][1,2,4]triazin-4-amine (1eq), and the mixture was allowed to react at 25°C for 5.5 hours. The reaction solution was evaporated under reduced pressure to obtain the target compound 2-(4-amino-5-bromopyrrolo[2,1-f][1,2,4]triazin-7-yl)acetaldehyde as a yellow solid. LCMS: m/z (M+H)⁺ =254.9.

**Step 3** : 2-(4-amino-5-bromo-pyrrolo[2,1-f][1,2,4]triazin-7-yl)acetaldehyde (1eq) and N,N-dimethylpiperidin-4-amine (2eq) were dissolved in DCM, followed by addition of NaBH₃CN (2eq) at 0°C. The reaction solution was allowed to react at 25°C for 16 hours. Water was added to the reaction solution and the mixture was extracted with EtOAc. The organic layer was evaporated under reduced pressure, and the target compound 5-bromo-7-(2-(4-(dimethylamino)piperidin-1-yl)ethyl)pyrrolo[2,1-f][1,2,4]triazin-4-amine (41.01% yield) was obtained as a yellow solid by column chromatography (Ethyl acetate/Methanol= 0:1). LCMS: m/z (M+H)⁺ =367.0.

**Step 4** : 5-bromo-7-(2-(4-(dimethylamino)piperidin-1-yl)ethyl)pyrrolo[2,1-f][1,2,4]triazin-4-amine (1eq) and tert-butyl N-[2-methoxy-4-(4,4,5,5-tetramethyl-1,3,2-dioxaborolan-2-yl)phenyl]carbamate (1.2eq) were dissolved in dioxane and H₂O, followed by addtion of K₃PO₄ (2eq) and Pd(dppf)Cl₂ (0.2eq). The reaction solution was allowed to react at 90°C for 1 hour. The reaction solution was evaporated under reduced pressure and purified by column chromatography (Ethyl acetate/Methanol= 0:1) to obtain the target compound ert-butyl (4-(4-amino-7-(2-(4-(dimethylamino)piperidin-1-yl)ethyl)pyrrolo[2,1-f][1,2,4]triazin-5-yl)-2-methoxyphenyl)carbamate (21.90% yield) as a yellow solid.

The compounds of Examples 64, 67 to 69, 78, 99, 100, 131 according to the present invention were prepared using a method similar to that of Example 98.

### Preparation of Example 123

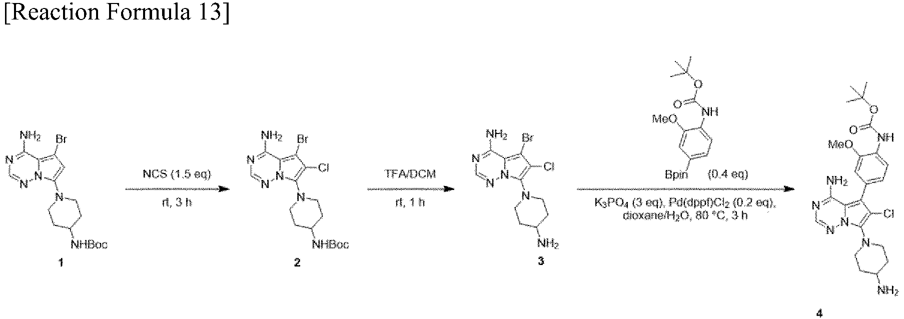

**Step 1 :** tert-butyl (1-(4-amino-5-bromopyrrolo[2,1-f][1,2,4]triazin-7-yl)piperidin-4-yl)carbamate (1eq) was dissolved in DMF, followed by addtion of NCS(1.5eq) at 0°C. The reaction solution was stirred at 25°C for 3 hours. Saturated aqueous Na₂SO₃ and saturated aqueous NaHCO₃ were added to the reaction mixture and the reaction mixture was extracted with EtOAc. The organic layer was evaporated under reduced pressure, and purified by column chromatography (Petroleum ether/Ethyl acetate = 1:1) to obtain the target compound 1-(4-amino-5-bromo-6-chloropyrrolo[2,1-f][1,2,4]triazin-7-yl)piperidin-4-yl)carbamate (75.66% yield) as a yellow solid. LCMS: m/z (M+H)⁺ = 445.0.

**Step 2** : tert-butyl (1-(4-amino-5-bromo-6-chloropyrrolo[2,1-f][1,2,4]triazin-7-yl)piperidin-4-yl)carbamate was dissolved in DCM, followed by addtion of TFA. The mixture was stirred at 25°C for 1 hour. After completion of the reaction, the mixture was evaporated under reduced pressure to obtain the target compound 7-(4-aminopiperidin-1-yl)-5-bromo-6-chloropyrrolo[2,1-f][1,2,4]triazin-4-amine as a yellow solid. LCMS: m/z (M+H)⁺=344.9.

**Step 3** : 7-(4-aminopiperidin-1-yl)-5-bromo-6-chloropyrrolo[2,1-f][1,2,4]triazin-4-amine (1eq) and K₃PO₄ (3eq) are dissolved in dioxane and H₂O, followed by addtion of tert-butyl N-[2-methoxy-4-(4,4,5,5-tetramethyl-1,3,2-dioxaborolan-2-yl)phenyl]carbamate(0.4eq) and Pd(dppf)Cl₂ (0.2eq). The reaction solution was stirred at 80°C for 3 hours. The mixture was evaporated under reduced pressure, and purified by column chromatography (Petroleum ether/Ethyl acetate= 1:1) to obtain the target compound tert-butyl (4-(4-amino-7-(4-aminopiperidin-1-yl)-6-chloropyrrolo[2,1-f][1,2,4]triazin-5-yl)-2-methoxyphenyl)carbamate (3.9% yield) as a white solid.

The compound of Example 124 according to the present invention was prepared using a method similar to that of Example 123.

### Preparation of Example 127

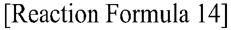

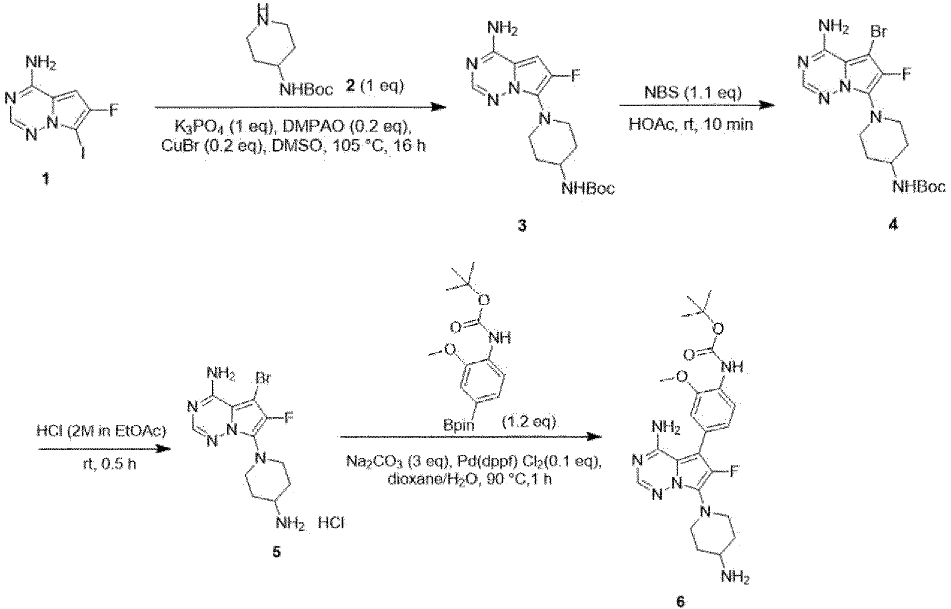

**Step** 1: 6-fluoro-7-iodo-pyrrolo[2,1-f][1,2,4]triazin-4-amine (1eq), tert-butyl N-(4-piperidyl)carbamate (1eq) and K₃PO₄ (1eq) were dissolved in DMSO, followed by addition of 2-((2,6-dimethylphenyl)amino)-2-oxoacetic acid (0.2eq) and CuBr (0.2eq). The reaction solution was stirred at 105°C for 16 hours. Water was added to the reaction solution and the mixture was extracted with EtOAc. The organic layer was evaporated under reduced pressure to obtain the target compound tert-butyl (1-(4-amino-6-fluoropyrrolo[2,1-f][1,2,4]triazin-7-yl)piperidin-4-yl)carbamate (9.5% yield) as a yellow solid by column chromatography (5-30% ethyl acetate in petroleum ether). LCMS: m/z (M+H)⁺ = 351.0.

**Step** 2 : tert-butyl (1-(4-amino-6-fluoropyrrolo[2,1-f][1,2,4]triazin-7-yl)piperidin-4-yl)carbamate (1eq) was dissolved in AcOH, followed by addition of NBS (1.1eq). The reaction solution was stirred at 25°C for 10 minutes. After completion of the reaction, the pH was adjusted to 7-8 with sat. aq. NaHCO₃, followed by extraction of the mixture with EtOAc. The organic layer was evaporated under reduced pressure to obtain the target compound tert-butyl (1-(4-amino-5-bromo-6-fluoropyrrolo[2,1-f][1,2,4]triazin-7-yl)piperidin-4-yl)carbamate as a yellow solid. LCMS: m/z (M+H)⁺ = 438.9.

**Step 3** : tert-butyl (1-(4-amino-5-bromo-6-fluoropyrrolo[2,1-f][1,2,4]triazin-7-yl)piperidin-4-yl)carbamate (1eq) was dissolved in HCl (2M in EtOAc), and the mixture was stirred at 25°C for 0.5 hours. The reaction solution was evaporated under reduced pressure to obtain the target compound 7-(4-aminopiperidin-1-yl)-5-bromo-6-fluoropyrrolo[2,1-f][1,2,4]triazin-4-amine as a yellow solid. LCMS: m/z (M+H)⁺ = 328.9.

**Step 4**: 7-(4-amino-1-piperidyl)-5-bromo-6-fluoro-pyrrolo[2,1-f][1,2,4]triazin-4-amine (1eq), tert-butyl (2-methoxy-4-(4,4,5,5-tetramethyl-1,3,2-dioxaborolan-2-yl)phenyl)carbamate (1.2eq), Pd(dppf)Cl₂ (0.1eq) and Na₂CO₃ (3eq) were dissolved in dioxane and water, and the mixture was stirred at 90°C for 1 hour. The mixture was evaporated under reduced pressure, and purified by column chromatography (5-20% methanol in ethyl acetate) to obtain the target tert-butyl N-[4-[4-amino-7-(4-amino-1-piperidyl)-6-fluoro-pyrrolo[2,1-f][1,2,4]triazin-5-yl]-2-methoxy-phenyl]carbamate(19.15 % yield) as a yellow solid. LCMS: m/z (M+H)⁺ = 472.1.

The compounds of Examples 128, 129, 130 according to the present invention were prepared using a method similar to that of Example 127.

### Preparation of Example 125

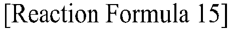

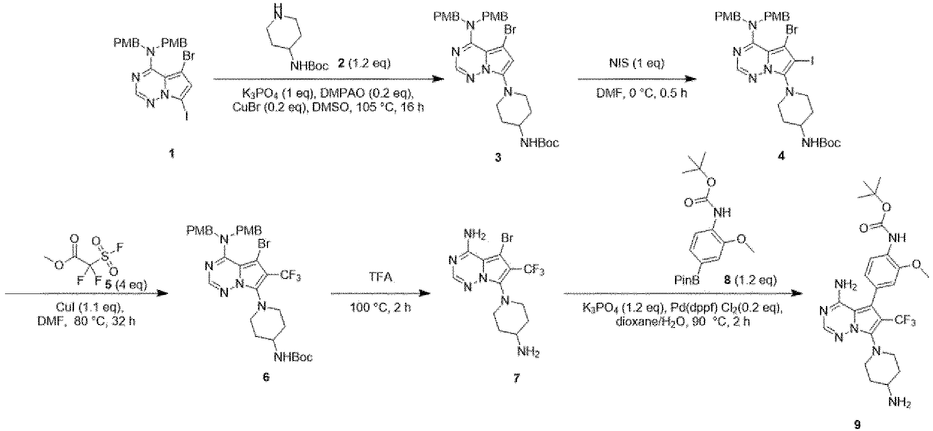

**Step 1** : tert-butyl piperidin-4-ylcarbamate (1.2eq) and 5-bromo-7-iodo-N,N-bis(4-methoxybenzyl)pyrrolo[2,1-f][1,2,4]triazin-4-amine (1eq) were dissolved in DMSO, followed by addirion of DMPAO (0.2eq), CuBr (0.2eq) and K₃PO₄ (1eq). The reaction solution was stirred at 105°C for 16 hours. After completion of the reaction, water was added and the mixture was extracted with EtOAc. The organic layer was evaporated under reduced pressure, and purified by column chromatography (Eluent of 20~35% Ethyl acetate/Petroleum ether) to obtain the target tert-butyl (1-(4-(bis(4-methoxybenzyl)amino)-5-bromopyrrolo[2,1-f][1,2,4]triazin-7-yl)piperidin-4-yl)carbamate (40.54% yield) as a yellow solid. LCMS: m/z (M+H)⁺ = 653.1
**Step 2:** tert-butyl (1-(4-(bis(4-methoxybenzyl)amino)-5-bromopyrrolo[2,1-f][1,2,4]triazin-7-yl)piperidin-4-yl)carbamate (leq) was dissolved in DMF, followed by addition of NIS (1eq) at 0°C. The reaction solution was stirred at 0°C for 0.5 hours. The reaction solution was poured into Na₂SO₃ solution and the mixture was extracted with EtOAc. The organic layer was evaporated under reduced pressure, and purified by column chromatography (Eluent of 35~ 45% Ethyl acetate/Petroleum ether) to obtain the target compound tert-butyl (1-(4-(bis(4-methoxybenzyl)amino)-5-bromo-6-iodopyrrolo[2,1-f][1,2,4]triazin-7-yl)piperidin-4-yl)carbamate (83.81% yield) as a yellow oil. LCMS: m/z (M+H)⁺ = 779.0
**Step 3** : tert-butyl (1-(4-(bis(4-methoxybenzyl)amino)-5-bromo-6-iodopyrrolo[2,1-f][1,2,4]triazin-7-yl)piperidin-4-yl)carbamate (1eq) was dissolved in DMF, followed by addtion of methyl 2,2-difluoro-2-(fluorosulfonyl)acetate (4eq) and then CuI (1.1eq). The reaction solution was stirred at 80 °C for 32 hours. After completion of the reaction, water was added to the reaction solution and the mixture was extracted with EtOAc. The organic layer was evaporated under reduced pressure, and purified by column chromatography (Eluent of 45~70% Ethyl acetate/Petroleum ether) to obtain the target compound tert-butyl (1-(4-(bis(4-methoxybenzyl)amino)-5-bromo-6-(trifluoromethyl)pyrrolo[2,1-f][1,2,4]triazin-7-yl)piperidin-4-yl)carbamate (63.42% yield) as a yellow oil. LCMS: m/z (M+H)⁺ = 721.1
**Step 4** : tert-butyl (1-(4-(bis(4-methoxybenzyl)amino)-5-bromo-6-(trifluoromethyl)pyrrolo[2,1-f][1,2,4]triazin-7-yl)piperidin-4-yl)carbamate (1eq) was dissolved in TFA and heated at 100 °C for 2 hours. The reaction solution was evaporated under reduced pressure to obtain the target compound 7-(4-aminopiperidin-1-yl)-5-bromo-6-(trifluoromethyl)pyrrolo[2,1-f][1,2,4]triazin-4-amine as a yellow solid. LCMS: m/z (M+H)⁺ = 380.9
**Step 5** : 7-(4-aminopiperidin-1-yl)-5-bromo-6-(trifluoromethyl)pyrrolo[2,1-f][1,2,4]triazin-4-amine (1eq) and tert-butyl (2-methoxy-4-(4,4,5,5-tetramethyl-1,3,2-dioxaborolan-2-yl)phenyl)carbamate (1.2eq) were dissolved in dioxane and H₂O, follwed by addtion of K₃PO₄ (1.2eq) and Pd(dppf)Cl₂ (0.2eq). The reaction solution was stirred at 90 °C for 2 hours. After completion of the reaction, the mixture was evaporated under reduced pressure, and purified by column chromatography (Eluent of 80% MeOH/Ethyl acetate) to obtain the target compound tert-butyl (4-(4-amino-7-(4-aminopiperidin-1-yl)-6-(trifluoromethyl)pyrrolo[2,1-f][1,2,4]triazin-5-yl)-2-methoxyphenyl)carbamate (12.2% yield) as a white solid.

The compound of Example 126 according to the present invention was prepared using a method similar to that of Example 125.

The chemical structures, compound names, NMR, purity and yield results of the compounds of Examples 1 to 138, are as follows.

### Example 1:

### tert-butyl (4-(4-amino-7-(4-(dimethylamino)phenyl)pyrrolo[2,1-f][1,2,4]triazin-5-yl)-2-methoxyphenyl)carbamate

¹H NMR (400 MHz, DMSO-d₆) δ = 8.01 (s, 1H), 7.98 - 7.89 (m, 3H), 7.82 (d, J = 8.6 Hz, 1H), 7.13 (s, 1H), 7.07 - 7.01 (m, 1H), 6.98 (s, 1H), 6.81 (d, J = 8.9 Hz, 2H), 3.87 (s, 3H), 2.96 (s, 6H), 1.48 (s, 9H), LCMS: m/z (M+H)⁺= 475.2
Purity: 97.8 %, Yield: 28.3 %

### Example 2:

### tert-butyl (4-(4-amino-7-(4-(dimethylamino)-2-methylphenyl)pyrrolo[2,1-f][1,2,4]triazin-5-yl)-2-methoxyphenyl)carbamate

¹H NMR (400 MHz, DMSO-d₆) δ = 7.99 (s, 1H), 7.83 - 7.81 (m, 2H), 7.20 (d, J = 8.5 Hz, 1H), 7.13 (d, J = 1.8 Hz, 1H), 7.05 (dd, J = 1.9, 8.1 Hz, 1H), 6.69 (s, 1H), 6.67 (d, J = 2.4 Hz, 1H), 6.63 (dd, J = 2.9, 8.6 Hz, 1H), 3.87 (s, 3H), 2.94 (s, 6H), 2.15 (s, 3H), 1.48 (s, 9H), LCMS: m/z (M+H)⁺= 489.3.
Purity: 96.6 %, Yield: 4.42 %

### Example 3:

### tert-butyl (4-(4-amino-7-(4-(dimethylamino)-3-methylphenyl)pyrrolo[2,1-f][1,2,4]triazin-5-yl)-2-methoxyphenyl)carbamate

¹H NMR (400 MHz, DMSO-d₆) δ = 8.00 (s, 1H), 7.96 (s, 1H), 7.90 - 7.80 (m, 3H), 7.14 (d, J = 1.3 Hz, 1H), 7.10 (d, J = 8.4 Hz, 1H), 7.07 - 7.01 (m, 2H), 3.87 (s, 3H), 2.68 (s, 6H), 2.32 (s, 3H), 1.48 (s, 9H), LCMS: m/z (M+H)⁺=489.3.
Purity: 99.1 %, Yield: 10.5 %

### Example 4:

### tert-butyl (4-(4-amino-7-(4-(dimethylamino)-3-fluorophenyl)pyrrolo[2,1-f][1,2,4]triazin-5-yl)-2-methoxyphenyl)carbamate

¹H NMR (400 MHz, DMSO-d₆) δ = 8.04 - 7.94 (m, 3H), 7.84 (dd, J = 1.9, 8.4 Hz, 2H), 7.16 - 7.10 (m, 2H), 7.07 - 7.00 (m, 2H), 3.87 (s, 3H), 2.85 (s, 6H), 1.48 (s, 9H), LCMS: m/z (M+H)⁺=493.2.
Purity: 94.1 %, Yield: 9.67 %

### Example 5:

### tert-butyl (4-(4-amino-7-(4-(dimethylamino)-3-methoxyphenyl)pyrrolo[2,1-f][1,2,4]triazin-5-yl)-2-methoxyphenyl)carbamate

¹H NMR (400 MHz, DMSO-d₆, HCl salt) δ = 8.88 (s, 1H), 8.47 (br s, 1H), 8.23 - 8.11 (m, 1H), 8.06 (s, 1H), 7.94 - 7.82 (m, 1H), 7.42 - 7.33 (m, 1H), 7.22 (br s, 1H), 7.16 (s, 1H), 7.08 (d, J = 8.1 Hz, 1H), 3.87 (s, 3H), 3.24 (d, J = 5.3 Hz, 6H), 1.48 (s, 9H), LCMS: m/z (M+H)⁺=476.2.
Purity: 95.6 %, Yield: 18.6 %

### Example 6:

### tert-butyl (4-(4-amino-7-(6-(dimethylamino)pyridin-3-yl)pyrrolo[2,1-f][1,2,4]triazin-5-yl)-2-methoxyphenyl)carbamate

¹H NMR (400 MHz, DMSO-d₆, HCl salt) δ = 8.86 (br s, 1H), 8.23 (s, 1H), 8.07 (s, 1H), 7.91 (d, J = 8.1 Hz, 1H), 7.87 - 7.81 (m, 2H), 7.78 (br s, 1H), 7.42 (s, 1H), 7.19 (d, J = 1.6 Hz, 1H), 7.11 (dd, J = 1.6, 8.2 Hz, 1H), 4.02 (s, 3H), 3.88 (s, 3H), 3.12 (s, 6H), 1.48 (s, 9H), LCMS: m/z (M+H)⁺=505.3.
Purity: 96.7 %, Yield: 25.5 %

### Example 7:

### tert-butyl (4-(4-amino-7-(4-methyl-3,4-dihydro-2H-benzo[b][1,4]oxazin-7-yl)pyrrolo[2,1-f][1,2,4]triazin-5-yl)-2-methoxyphenyl)carbamate

¹H NMR (400 MHz, DMSO-d₆) δ = 8.01 (s, 1H), 7.94 (s, 1H), 7.82 (d, J = 8.7 Hz, 1H), 7.56 - 7.50 (m, 2H), 7.13 (d, J = 1.6 Hz, 1H), 7.03 (dd, J = 1.3, 8.3 Hz, 1H), 6.99 (s, 1H), 6.77 (d, J = 8.9 Hz, 1H), 4.30 - 4.22 (m, 2H), 3.87 (s, 3H), 3.31 - 3.27 (m, 2H), 2.88 (s, 3H), 1.48 (s, 9H), LCMS: m/z (M+H)⁺= 503.1.
Purity: 98.2 %, Yield: 8.33 %

### Example 8:

### tert-butyl (4-(4-amino-7-(4-methyl-3-oxo-3,4-dihydro-2H-benzo[b][1,4]oxazin-7-yl)pyrrolo[2,1-f][1,2,4]triazin-5-yl)-2-methoxyphenyl)carbamate

¹H NMR (400 MHz, DMSO-d6) δ = 8.02 - 8.00 (m, 2H), 7.90 - 7.80 (m, 3H), 7.26 (d, J = 8.4 Hz, 1H), 7.18 (s, 1H), 7.15 (d, J = 1.2 Hz, 1H), 7.09 - 7.02 (m, 1H), 4.71 (s, 2H), 3.87 (s, 3H), 3.27 (s, 3H), 1.48 (s, 9H), LCMS: m/z (M+H)+= 517.3.
Purity: 98.2 %, Yield: 39.9 %

### Example 9:

### tert-butyl (4-(4-amino-7-(3-methyl-2-oxo-2,3-dihydrobenzo[d]oxazol-6-yl)pyrrolo[2,1-f][1,2,4]triazin-5-yl)-2-methoxyphenyl)carbamate

¹H NMR (400 MHz, DMSO-d6) δ = 8.14 (d, J = 1.3 Hz, 1H), 8.07 - 7.96 (m, 3H), 7.84 (d, J = 8.1 Hz, 1H), 7.36 (d, J = 8.4 Hz, 1H), 7.19 (s, 1H), 7.15 (d, J = 1.6 Hz, 1H), 7.06 (dd, J = 1.7, 8.2 Hz, 1H), 3.87 (s, 3H), 3.39 (s, 3H), 1.48 (s, 9H), LCMS: m/z (M+H)+= 503.2.
Purity: 96.8 %, Yield: 41.3 %

### Example 10:

### tert-butyl (R)-(4-(4-amino-7-(3-(dimethylamino)pyrrolidin-1-yl)pyrrolo[2,1-f][1,2,4]triazin-5-yl)-2-methoxyphenyl)carbamate

1H NMR (400 MHz, DMSO-d6, FA salt) δ = 8.13 (s, 1H), 7.97 (s, 1H), 7.78 (d, J = 8.0 Hz, 1H), 7.75 (s, 1H), 7.04 (d, J = 1.8 Hz, 1H), 6.96 (dd, J = 1.8, 8.2 Hz, 1H), 6.13 (s, 1H), 3.84 (s, 3H), 3.55 - 3.38 (m, 5H), 2.38 (s, 6H), 2.19 - 2.11 (m, 1H), 1.96 - 1.83 (m, 1H), 1.46 (s, 9H), LCMS: m/z (M+H)+=468.3.
Purity: 95.5 %, Yield: 3.35 %

### Example 11:

### tert-butyl (S)-(4-(4-amino-7-(3-(dimethylamino)pyrrolidin-1-yl)pyrrolo[2,1-f][1,2,4]triazin-5-yl)-2-methoxyphenyl)carbamate

1H NMR (400 MHz, DMSO-d6, FA salt) δ = 8.13 (s, 1H), 7.99 (s, 1H), 7.80 (d, J = 8.3 Hz, 1H), 7.77 (s, 1H), 7.04 (d, J = 1.6 Hz, 1H), 6.97 (dd, J = 1.5, 8.3 Hz, 1H), 6.17 (s, 1H), 3.85 (s, 3H), 3.60 - 3.45 (m, 4H), 3.42 - 3.38 (m, 1H), 2.55 (s, 6H), 2.26 - 2.17 (m, 1H), 2.07 - 1.93 (m, 1H), 1.47 (s, 9H), LCMS: m/z (M+H)+=468.3.
Purity: 98.3 %, Yield: 4.45 %

### Example 12:

### tert-butyl (4-(4-amino-7-(4-aminopiperidin-1-yl)pyrrolo[2,1-f][1,2,4]triazin-5-yl)-2-methoxyphenyl)carbamate

1H NMR (400 MHz, DMSO-d6, FA salt) δ = 8.42 (s, 1H), 7.97 (s, 1H), 7.81 (s, 1H), 7.79 (d, J = 8.4 Hz, 1H), 7.05 (d, J = 1.7 Hz, 1H), 6.97 (dd, J = 1.6, 8.2 Hz, 1H), 6.24 (s, 1H), 3.85 (s, 3H), 3.69 - 3.65 (m, 2H), 2.97 - 2.90 (m, 1H), 2.74 (t, J = 10.8 Hz, 2H), 1.89 (dd, J = 1.5, 10.8 Hz, 2H), 1.65 - 1.51 (m, 2H), 1.47 (s, 9H), LCMS: m/z (M+H)+=454.3.
Purity: 99.9 %, Yield: 39.9 %

### Example 13:

### tert-butyl (4-(4-amino-7-(4-(dimethylamino)-4-methylpiperidin-1-yl)pyrrolo[2,1-f][1,2,4]triazin-5-yl)-2-methoxyphenyl)carbamate

1H NMR (400 MHz, DMSO-d6, FA salt) δ = 8.42 (s, 1H), 7.97 (s, 1H), 7.81 (s, 1H), 7.79 (d, J = 8.4 Hz, 1H), 7.05 (d, J = 1.7 Hz, 1H), 6.97 (dd, J = 1.6, 8.2 Hz, 1H), 6.24 (s, 1H), 3.85 (s, 3H), 3.69 - 3.65 (m, 2H), 2.97 - 2.90 (m, 1H), 2.74 (t, J = 10.8 Hz, 2H), 1.89 (dd, J = 1.5, 10.8 Hz, 2H), 1.65 - 1.51 (m, 2H), 1.47 (s, 9H), LCMS: m/z (M+H)+=454.3.
Purity: 90.0 %, Yield: 1.13 %

### Example 14:

### tert-butyl (4-(4-amino-7-(4-(diethylamino)piperidin-1-yl)pyrrolo[2,1-f][1,2,4]triazin-5-yl)-2-methoxyphenyl)carbamate

1H NMR (400 MHz, DMSO-d6, FA salt) δ = 7.99 (s, 1H), 7.90 - 7.73 (m, 2H), 7.05 (d, J = 1.5 Hz, 1H), 6.97 (dd, J = 1.4, 8.2 Hz, 1H), 6.23 (s, 1H), 3.85 (s, 3H), 3.77 (d, J = 12.4 Hz, 2H), 2.81 - 2.50 (m, 7H), 1.89 - 1.77 (m, 2H), 1.74 - 1.59 (m, 2H), 1.47 (s, 9H), 1.03 (t, J = 6.8 Hz, 6H), LCMS: m/z (M+H)+=510.3.
Purity: 92.8 %, Yield: 10.5 %

### Example 15:

### tert-butyl (4-(4-amino-7-(4-(dimethylamino)piperidin-1-yl)pyrrolo[2,1-f][1,2,4]triazin-5-yl)-2-methoxyphenyl)carbamate

1H NMR (400 MHz, DMSO-d6, FA salt) δ = 8.14 (s, 1H), 7.98 (s, 1H), 7.82 (s, 1H), 7.79 (d, J = 8.2 Hz, 1H), 7.05 (d, J = 1.5 Hz, 1H), 6.97 (dd, J = 1.5, 8.3 Hz, 1H), 6.26 (s, 1H), 3.85 (s, 3H), 3.80 (br d, J = 11.6 Hz, 2H), 2.96 - 2.85 (m, 1H), 2.70 (br t, J = 11.8 Hz, 2H), 2.56 (br s, 6H), 1.99 (br d, J = 10.3 Hz, 2H), 1.80 - 1.66 (m, 2H), 1.47 (s, 9H), LCMS: m/z (M+H)+=482.3.
Purity: 100 %, Yield: 27.8 %

### Example 16:

### tert-butyl (4-(4-amino-7-(4-(tert-butyl)piperazin-1-yl)pyrrolo[2,1-f][1,2,4]triazin-5-yl)-2-methoxyphenyl)carbamate

1H NMR (400 MHz, DMSO-d6) δ = 7.99 (s, 1H), 7.88 - 7.74 (m, 2H), 7.05 (s, 1H), 6.98 (dd, J = 1.3, 8.2 Hz, 1H), 6.28 (s, 1H), 3.85 (s, 3H), 3.20 - 2.81 (m, 8H), 1.47 (s, 9H), 1.20 (s, 9H), LCMS: m/z (M+H)+= 536.3.
Purity: 98.3 %, Yield: 0.42 %

### Example 17:

### tert-butyl (4-(4-amino-7-(4-cyclopropylpiperazin-1-yl)pyrrolo[2,1-f][1,2,4]triazin-5-yl)-2-methoxyphenyl)carbamate

1H NMR (40 MHz, DMSO-d6) δ = 7.98 (s, 1H), 7.81 (s, 1H), 7.79 (d, J = 8.1 Hz, 1H), 7.05 (s, 1H), 6.97 (d, J = 8.6 Hz, 1H), 6.21 (s, 1H), 3.85 (s, 3H), 3.20 - 3.12 (m, 4H), 2.76 - 2.68 (m, 4H), 1.70 (td, J = 3.3, 6.8 Hz, 1H), 1.47 (s, 9H), 0.47 - 0.43 (m, 2H), 0.36 - 0.32 (m, 2H), LCMS: m/z (M+H)+= 480.2.
Purity: 87.8 %, Yield: 0.68 %

### Example 18:

### tert-butyl (4-(4-amino-7-(6-(dimethylamino)-2-azaspiro[3.3]heptan-2-yl)pyrrolo[2,1-f][1,2,4]triazin-5-yl)-2-methoxyphenyl)carbamate

1H NMR (400 MHz, DMSO-d6, FA salt) δ = 8.14 (s, 1H), 7.98 (s, 1H), 7.78 (d, J = 8.2 Hz, 1H), 7.69 (s, 1H), 7.03 (d, J = 1.3 Hz, 1H), 6.96 (dd, J = 1.4, 8.3 Hz, 1H), 5.96 (s, 1H), 3.99 (s, 2H), 3.88 (s, 2H), 3.84 (s, 3H), 2.89 - 2.75 (m, 1H), 2.41 - 2.30 (m, 2H), 2.21 (s, 6H), 2.09 (t, J = 9.9 Hz, 2H), 1.47 (s, 9H), LCMS: m/z (M+H)+=494.2.
Purity: 98.1 %, Yield: 22.3 %

### Example 19:

### tert-butyl (4-(4-amino-7-(4-((dimethylamino)methyl)piperidin-1-yl)pyrrolo[2,1-f][1,2,4]triazin-5-yl)-2-methoxyphenyl)carbamate

1H NMR (400 MHz, DMSO-d6, FA salt) δ = 8.00 (s, 1H), 7.90 - 7.80 (m, 1H), 7.79 (d, J = 7.9 Hz, 1H), 7.05 (d, J = 1.6 Hz, 1H), 6.97 (dd, J = 1.7, 8.2 Hz, 1H), 6.23 (s, 1H), 3.85 (s, 3H), 3.69 (d, J = 11.7 Hz, 2H), 2.67 (t, J = 11.2 Hz, 2H), 2.57 - 2.52 (m, 2H), 2.40 (s, 6H), 1.82 - 1.68 (m, 2H), 1.47 (s, 9H), 1.41 - 1.27 (m, 2H), LCMS: m/z (M+H)+=496.3.
Purity: 93.0 %, Yield: 4.11 %

### Example 20:

### tert-butyl (4-(4-amino-7-(3-((dimethylamino)methyl)pyrrolidin-1-yl)pyrrolo[2,1-f][1,2,4]triazin-5-yl)-2-methoxyphenyl)carbamate

1H NMR (400 MHz, DMSO-d6) δ = 7.97 (s, 1H), 7.78 (d, J = 7.1 Hz, 1H), 7.73 (br s, 1H), 7.05 (s, 1H), 6.97 (d, J = 7.3 Hz, 1H), 6.06 (s, 1H), 3.85 (s, 3H), 3.52 (t, J = 8.1 Hz, 1H), 3.42 - 3.34 (m, 2H), 3.17 - 3.11 (m, 1H), 2.40 - 2.19 (m, 3H), 2.15 (s, 6H), 2.08 - 1.99 (m, 1H), 1.65 - 1.54 (m, 1H), 1.47 (s, 9H), LCMS: m/z (M+H)+=482.2.
Purity: 95.6 %, Yield: 3.73 %

### Example 21:

### tert-butyl (4-(4-amino-7-(2-methyl-2,7-diazaspiro[3.5]nonan-7-yl)pyrrolo[2,1-f][1,2,4]triazin-5-yl)-2-methoxyphenyl)carbamate

1H NMR (400 MHz, Methanol-d4) δ = 7.94 (d, J = 8.3 Hz, 1H), 7.81 (br s, 1H), 7.10 - 7.06 (m, 1H), 7.05 - 7.01 (m, 1H), 6.29 (s, 1H), 3.93 (s, 3H), 3.48 - 3.36 (m, 4H), 3.22 - 3.14 (m, 4H), 2.55 (s, 3H), 2.04 - 1.98 (m, 4H), 1.56 (s, 9H), LCMS: m/z (M+H)+=494.2.
Purity: 90.3 %, Yield: 0.45 %

### Example 22:

### tert-butyl (4-(4-amino-7-(2-methyl-2,8-diazaspiro[4.5]decan-8-yl)pyrrolo[2,1-f][1,2,4]triazin-5-yl)-2-methoxyphenyl)carbamate

1H NMR (400 MHz, Methanol-d4) δ = 7.93 (d, J = 8.0 Hz, 1H), 7.79 (br s, 1H), 7.06 (s, 1H), 7.02 (d, J = 8.6 Hz, 1H), 6.29 (s, 1H), 3.92 (s, 3H), 3.26 - 3.16 (m, 4H), 2.74 - 2.65 (m, 2H), 2.59 (s, 2H), 2.41 (s, 3H), 1.92 - 1.75 (m, 6H), 1.54 (s, 9H), LCMS: m/z (M+H)+=508.2.
Purity: 97.5 %, Yield: 1.14 %

### Example 23:

### tert-butyl (4-(4-amino-7-(1,8-diazaspiro[4.5]decan-8-yl)pyrrolo[2,1-f][1,2,4]triazin-5-yl)-2-methoxyphenyl)carbamate

1H NMR (400 MHz, DMSO-d6, FA salt) δ = 8.85 - 8.68 (m, 2H), 8.01 (s, 1H), 7.85 (s, 1H), 7.81 (d, J = 8.1 Hz, 1H), 7.05 (d, J = 1.8 Hz, 1H), 6.98 (dd, J = 1.7, 8.2 Hz, 1H), 6.29 (s, 1H), 3.85 (s, 3H), 3.57 - 3.50 (m, 2H), 3.29 - 3.23 (m, 2H), 3.03 (t, J = 9.6 Hz, 2H), 2.06 - 1.87 (m, 8H), 1.47 (s, 9H), LCMS: m/z (M+H)+=494.2.
Purity: 99.0 %, Yield: 24.5 %

### Example 24:

### tert-butyl (4-(4-amino-7-(1-methyl-1,8-diazaspiro[4.5]decan-8-yl)pyrrolo[2,1-f][1,2,4]triazin-5-yl)-2-methoxyphenyl)carbamate

1H NMR (400 MHz, DMSO-d6, FA salt) δ = 8.21 (s, 1H), 7.98 (s, 1H), 7.85 - 7.75 (m, 2H), 7.05 (d, J = 1.8 Hz, 1H), 6.97 (dd, J = 1.7, 8.2 Hz, 1H), 6.25 (s, 1H), 3.85 (s, 3H), 3.70 (d, J = 11.6 Hz, 2H), 2.79 - 2.66 (m, 4H), 2.29 (s, 3H), 1.84 (dt, J = 3.8, 12.5 Hz, 2H), 1.80 - 1.68 (m, 4H), 1.47 (s, 9H), 1.33 (d, J = 12.3 Hz, 2H), LCMS: m/z (M+H)+=508.3.
Purity: 97.3 %, Yield: 8.59 %

### Example 25:

### tert-butyl (4-(4-amino-7-(octahydro-5H-pyrrolo[3,2-c]pyridin-5-yl)pyrrolo[2,1-f][1,2,4]triazin-5-yl)-2-methoxyphenyl)carbamate

1H NMR (400 MHz, DMSO-d6, FA salt) δ = 9.04 (br s, 1H), 8.69 (br s, 1H), 8.01 (s, 1H), 7.86 (s, 1H), 7.81 (d, J = 8.1 Hz, 1H), 7.05 (d, J = 1.6 Hz, 1H), 6.98 (dd, J = 1.8, 8.1 Hz, 1H), 6.30 (s, 1H), 3.85 (s, 3H), 3.79 - 3.72 (m, 1H), 3.34 - 3.20 (m, 5H), 3.06 - 2.96 (m, 1H), 2.56 - 2.53 (m, 1H), 2.17 - 1.97 (m, 3H), 1.95 - 1.83 (m, 1H), 1.47 (s, 9H), LCMS: m/z (M+H)+=480.3.
Purity: 99.0 %, Yield: 22.6 %

### Example 26:

### tert-butyl (4-(4-amino-7-(1-methyloctahydro-5H-pyrrolo[3,2-c]pyridin-5-yl)pyrrolo[2,1-f][1,2,4]triazin-5-yl)-2-methoxyphenyl)carbamate

1H NMR (400 MHz, Methanol-d4) δ = 7.96 (d, J = 8.2 Hz, 1H), 7.84 (s, 1H), 7.08 (s, 1H), 7.04 (dd, J = 1.5, 8.1 Hz, 1H), 6.39 (s, 1H), 3.93 (s, 3H), 3.90 - 3.80 (m, 1H), 3.77 - 3.66 (m, 1H), 3.52 - 3.36 (m, 2H), 3.29 - 3.09 (m, 2H), 3.04 - 2.87 (m, 4H), 2.78 - 2.59 (m, 1H), 2.43 - 1.99 (m, 4H), 1.54 (s, 9H), LCMS: m/z (M+H)+=494.2.
Purity: 97.2 %, Yield: 2.34 %

### Example 27:

### tert-butyl (4-(4-amino-7-(2-methyl-3-oxo-2,8-diazaspiro[4.5]decan-8-yl)pyrrolo[2,1-f][1,2,4]triazin-5-yl)-2-methoxyphenyl)carbamate

1H NMR (400 MHz, Methanol-d4, HCl salt) δ = 7.98 (d, J = 8.1 Hz, 1H), 7.86 (s, 1H), 7.10 (s, 1H), 7.06 (d, J = 8.4 Hz, 1H), 6.43 (s, 1H), 3.93 (s, 3H), 3.46 - 3.39 (m, 2H), 3.37 (s, 2H), 3.36 - 3.32 (m, 2H), 2.86 (s, 3H), 2.39 (s, 2H), 1.92 - 1.78 (m, 4H), 1.54 (s, 9H), LCMS: m/z (M+H)+=522.3.
Purity: 99.5 %, Yield: 6.72 %

### Example 28:

### tert-butyl (4-(4-amino-7-(4-(2-(dimethylamino)ethyl)piperazin-1-yl)pyrrolo[2,1-f][1,2,4]triazin-5-yl)-2-methoxyphenyl)carbamate

1H NMR (400 MHz, Methanol-d4) δ = 8.01 - 7.92 (m, 1H), 7.84 (d, J = 2.2 Hz, 1H), 7.12 - 7.00 (m, 2H), 6.37 (d, J = 2.4 Hz, 1H), 3.93 (d, J = 2.3 Hz, 3H), 3.45 - 3.39 (m, 4H), 3.36 - 3.33 (m, 2H), 2.95 (s, 3H), 2.94 (s, 3H), 2.84 - 2.74 (m, 6H), 1.54 (d, J = 2.3 Hz, 9H), LCMS: m/z (M+H)+=511.3.
Purity: 94.5 %, Yield: 2.72 %

### Example 29:

### tert-butyl (4-(4-amino-7-(4-(2-methoxyethyl)piperazin-1-yl)pyrrolo[2,1-f][1,2,4]triazin-5-yl)-2-methoxyphenyl)carbamate

1H NMR (400 MHz, DMSO-d6) δ = 7.98 (s, 1H), 7.80 (s, 1H), 7.78 (s, 1H), 7.05 (d, J = 1.5 Hz, 1H), 6.97 (d, J = 8.2 Hz, 1H), 6.23 (s, 1H), 3.85 (s, 3H), 3.47 (t, J = 5.7 Hz, 2H), 3.25 (s, 3H), 3.23 - 3.17 (m, 4H), 2.65 - 2.57 (m, 4H), 2.56 - 2.50 (m, 2H), 1.47 (s, 9H), LCMS: m/z (M+H)+=498.2.
Purity: 100 %, Yield: 9.38 %

### Example 30:

### tert-butyl (4-(4-amino-7-((3S,4R)-4-(dimethylamino)-3-fluoropiperidin-1-yl)pyrrolo[2,1-f][1,2,4]triazin-5-yl)-2-methoxyphenyl)carbamate

1H NMR (400 MHz, DMSO-d6) δ = 7.98 (s, 1H), 7.82 (s, 1H), 7.79 (d, J = 8.3 Hz, 1H), 7.05 (d, J = 1.8 Hz, 1H), 6.97 (dd, J = 1.7, 8.2 Hz, 1H), 6.23 (s, 1H), 5.16 - 4.95 (m, 1H), 4.03 - 3.92 (m, 1H), 3.88 - 3.86 (m, 1H), 3.85 (s, 3H), 2.92 (dd, J = 13.2, 38.0 Hz, 1H), 2.72 (t, J = 11.6 Hz, 1H), 2.44 - 2.35 (m, 1H), 2.29 (s, 6H), 2.05 - 1.88 (m, 1H), 1.81 - 1.69 (m, 1H), 1.47 (s, 9H), LCMS: m/z (M+H)+=500.3.
Purity: 99.2 %, Yield: 10.4 %

### Example 31:

### tert-butyl (4-(4-amino-7-((3S,4S)-4-(dimethylamino)-3-fluoropiperidin-1-yl)pyrrolo[2,1-f][1,2,4]triazin-5-yl)-2-methoxyphenyl)carbamate

1H NMR (400 MHz, DMSO-d6) δ = 7.98 (s, 1H), 7.84 (s, 1H), 7.79 (d, J = 8.1 Hz, 1H), 7.06 (d, J = 1.8 Hz, 1H), 6.98 (dd, J = 1.5, 8.3 Hz, 1H), 6.31 (s, 1H), 4.95 - 4.73 (m, 1H), 4.04 - 3.93 (m, 1H), 3.85 (s, 3H), 3.71 - 3.62 (m, 1H), 2.82 - 2.77 (m, 1H), 2.77 - 2.68 (m, 2H), 2.37 (br s, 6H), 1.94 - 1.82 (m, 1H), 1.74 - 1.62 (m, 1H), 1.47 (s, 9H), LCMS: m/z (M+H)+=500.3.
Purity: 99.6%, Yield: 2.24%

### Example 32:

### tert-butyl (4-(4-amino-7-((3R,4R)-4-(dimethylamino)-3-fluoropiperidin-1-yl)pyrrolo[2,1-f][1,2,4]triazin-5-yl)-2-methoxyphenyl)carbamate

1H NMR (400 MHz, Methanol-d4) δ = 8.52 (br s, 1H), 7.94 (br d, J = 8.1 Hz, 1H), 7.79 (s, 1H), 7.06 (d, J = 1.5 Hz, 1H), 7.02 (dd, J = 1.8, 8.3 Hz, 1H), 6.33 (s, 1H), 5.07 - 4.94 (m, 1H), 4.11 - 4.00 (m, 1H), 3.92 (s, 3H), 3.76 - 3.67 (m, 1H), 2.97 - 2.87 (m, 1H), 2.86 - 2.76 (m, 2H), 2.53 (s, 6H), 2.07 - 1.97 (m, 1H), 1.95 - 1.81 (m, 1H), 1.54 (s, 9H), LCMS: m/z (M+H)+=500.3.
Purity: 94.6 %, Yield: 18.6 %

### Example 33:

### tert-butyl (4-(4-amino-7-((3R,4S)-4-(dimethylamino)-3-fluoropiperidin-1-yl)pyrrolo[2,1-f][1,2,4]triazin-5-yl)-2-methoxyphenyl)carbamate

1H NMR (400 MHz, DMSO-d6) δ = 9.94 - 9.58 (m, 1H), 7.99 (s, 1H), 7.84 (s, 1H), 7.80 (br d, J = 8.3 Hz, 1H), 7.05 (d, J = 1.6 Hz, 1H), 6.97 (dd, J = 1.8, 8.3 Hz, 1H), 6.28 (s, 1H), 5.48 - 5.10 (m, 1H), 4.21 - 4.02 (m, 1H), 3.97 - 3.88 (m, 1H), 3.85 (s, 3H), 3.12 - 2.91 (m, 2H), 2.90 - 2.69 (m, 6H), 2.17 - 1.94 (m, 3H), 1.47 (s, 11H), LCMS: m/z (M+H)+=500.3.
Purity: 96.7%, Yield: 12.9%

### Example 34:

### tert-butyl (4-(4-amino-7-((3aR,6aS)-5-methylhexahydropyrrolo[3,4-c]pyrrol-2(1H)-yl)pyrrolo[2,1-f][1,2,4]triazin-5-yl)-2-methoxyphenyl)carbamate

1H NMR (400 MHz, Methanol-d4) δ = 7.93 (br d, J = 8.1 Hz, 1H), 7.73 (s, 1H), 7.06 (s, 1H), 7.04 - 6.98 (m, 1H), 6.18 (s, 1H), 3.92 (s, 3H), 3.44 (br d, J = 9.2 Hz, 2H), 3.26 (br d, J = 5.5 Hz, 2H), 2.97 (br d, J = 4.3 Hz, 4H), 2.53 - 2.43 (m, 2H), 2.39 (s, 3H), 1.54 (s, 9H), LCMS: m/z (M+H)+=480.3.
Purity: 99.5 %, Yield: 4.03 %

### Example 35A:

### tert-butyl (4-(4-amino-7-((3aR,7aR)-5-methyloctahydro-2H-pyrrolo[3,4-c]pyridin-2-yl)pyrrolo[2,1-f][1,2,4]triazin-5-yl)-2-methoxyphenyl)carbamate

1H NMR (400 MHz, DMSO-d6) δ = 7.97 (s, 1H), 7.78 (d, J = 7.6 Hz, 1H), 7.66 (s, 1H), 7.05 (d, J = 1.2 Hz, 1H), 6.97 (d, J = 8.0 Hz, 1H), 5.99 (s, 1H), 3.85 (s, 3H), 3.58 - 3.51 (m, 2H), 3.51 - 3.43 (m, 1H), 3.39 - 3.35 (m, 1H), 2.38 (br d, J = 8.3 Hz, 3H), 2.34 - 2.28 (m, 1H), 2.24 - 2.17 (m, 1H), 2.15 (s, 3H), 2.13 - 2.07 (m, 1H), 1.75 - 1.65 (m, 1H), 1.62 - 1.52 (m 1H), 1.47 (s, 9H), LCMS: m/z (M+H)+=494.2.
Purity: 98.6 %, Yield: 1.94 %

### Example 35B:

### tert-butyl (4-(4-amino-7-((3aS,7aS)-5-methyloctahydro-2H-pyrrolo[3,4-c]pyridin-2-yl)pyrrolo[2,1-f][1,2,4]triazin-5-yl)-2-methoxyphenyl)carbamate

1H NMR (400 MHz, DMSO-d6) δ = 7.98 (s, 1H), 7.77 (br d, J = 8.3 Hz, 1H), 7.66 (s, 1H), 7.05 (d, J = 1.8 Hz, 1H), 6.97 (dd, J = 1.7, 8.2 Hz, 1H), 5.99 (s, 1H), 3.85 (s, 3H), 3.54 (dt, J = 3.4, 6.4 Hz, 2H), 3.50 - 3.44 (m, 1H), 3.39 - 3.34 (m, 1H), 2.43 - 2.34 (m, 3H), 2.33 - 2.27 (m, 1H), 2.24 - 2.17 (m, 1H), 2.15 (s, 3H), 2.12 - 2.06 (m, 1H), 1.77 - 1.64 (m, 1H), 1.63 - 1.53 (m, 1H), 1.47 (s, 9H), LCMS: m/z (M+H)+=494.2.
Purity: 98.2 %, Yield: 1.61 %

### Example 36:

### tert-butyl (4-(4-amino-7-(8-methyl-2,8-diazaspiro[4.5]decan-2-yl)pyrrolo[2,1-f][1,2,4]triazin-5-yl)-2-methoxyphenyl)carbamate

1H NMR (400 MHz, Methanol-d4) δ = 7.92 (d, J = 8.0 Hz, 1H), 7.67 (s, 1H), 7.06 (d, J = 1.6 Hz, 1H), 7.02 (dd, J = 1.8, 8.2 Hz, 1H), 6.11 (s, 1H), 3.92 (s, 3H), 3.55 (t, J = 7.0 Hz, 2H), 3.38 (s, 2H), 2.89 - 2.61 (m, 4H), 2.49 (s, 3H), 1.90 (t, J = 7.0 Hz, 2H), 1.86 - 1.75 (m, 4H), 1.54 (s, 9H), LCMS: m/z (M+H)+=508.3.
Purity: 95.8 %, Yield: 6.83 %

### Example 37:

### tert-butyl (4-(4-amino-7-(2-methyl-2,6-diazaspiro[3.4]octan-6-yl)pyrrolo[2,1-f][1,2,4]triazin-5-yl)-2-methoxyphenyl)carbamate

1H NMR (400 MHz, Methanol-d4) δ = 7.92 (d, J = 8.1 Hz, 1H), 7.67 (s, 1H), 7.05 (s, 1H), 7.02 (dd, J = 1.7, 8.2 Hz, 1H), 6.11 (s, 1H), 3.92 (s, 3H), 3.62 (s, 2H), 3.46 (t, J = 7.0 Hz, 2H), 3.42 - 3.34 (m, 4H), 2.40 (s, 3H), 2.20 (t, J = 7.0 Hz, 2H), 1.54 (s, 9H), LCMS: m/z (M+H)+=480.2.
Purity: 95.6 %, Yield: 4.89 %

### Example 38:

### tert-butyl (4-(4-amino-7-(7-methyl-2,7-diazaspiro[3.5]nonan-2-yl)pyrrolo[2,1-f][1,2,4]triazin-5-yl)-2-methoxyphenyl)carbamate

1H NMR (400 MHz, DMSO-d6, FA salt) δ = 8.01 (s, 1H), 7.79 (d, J = 8.3 Hz, 1H), 7.70 (s, 1H), 7.03 (d, J = 1.8 Hz, 1H), 6.96 (dd, J = 1.7, 8.2 Hz, 1H), 5.97 (s, 1H), 3.85 (s, 3H), 3.80 (s, 4H), 3.18 - 2.86 (m, 4H), 2.67 (s, 3H), 2.05 - 1.87 (m, 4H), 1.47 (s, 9H), LCMS: m/z (M+H)+=494.3.
Purity: 99.0 %, Yield: 7.72 %

### Example 39:

### tert-butyl (4-(4-amino-7-(6-methyl-2,6-diazaspiro[3.4]octan-2-yl)pyrrolo[2,1-f][1,2,4]triazin-5-yl)-2-methoxyphenyl)carbamate

1H NMR (400 MHz, Methanol-d4) δ = 7.92 (d, J = 8.1 Hz, 1H), 7.65 (s, 1H), 7.05 (d, J = 1.6 Hz, 1H), 7.01 (dd, J = 1.7, 8.1 Hz, 1H), 6.03 (s, 1H), 4.04 (d, J = 7.2 Hz, 2H), 3.97 (d, J = 7.2 Hz, 2H), 3.92 (s, 3H), 2.89 (s, 2H), 2.64 (t, J = 7.0 Hz, 2H), 2.40 (s, 3H), 2.20 (t, J = 7.1 Hz, 2H), 1.54 (s, 9H), LCMS: m/z (M+H)+=480.2.
Purity: 99.6 %, Yield: 4.87 %

### Example 40:

### tert-butyl (S)-(4-(4-amino-7-(3-((dimethylamino)methyl)piperidin-1-yl)pyrrolo[2,1-f][1,2,4]triazin-5-yl)-2-methoxyphenyl)carbamate

1H NMR (400 MHz, Methanol-d4) δ = 7.94 (d, J = 8.4 Hz, 1H), 7.87 (s, 1H), 7.05 (d, J = 1.6 Hz, 1H), 7.02 (dd, J = 1.8, 8.2 Hz, 1H), 6.31 (s, 1H), 3.92 (s, 3H), 3.43 - 3.33 (m, 2H), 3.16 - 2.88 (m, 4H), 2.83 (s, 6H), 2.37 - 2.27 (m, 1H), 1.98 - 1.88 (m, 2H), 1.86 - 1.78 (m, 1H), 1.54 (s, 9H), 1.50 - 1.41 (m, 1H), LCMS: m/z (M+H)+=496.3.
Purity: 96.91 %, Yield: 5.72 %

### Example 41:

### tert-butyl (R)-(4-(4-amino-7-(3-((dimethylamino)methyl)piperidin-1-yl)pyrrolo[2,1-f][1,2,4]triazin-5-yl)-2-methoxyphenyl)carbamate

1H NMR (400 MHz, Methanol-d4) δ = 7.93 (br d, J = 7.8 Hz, 1H), 7.77 (s, 1H), 7.06 (s, 1H), 7.02 (dd, J = 1.3, 8.4 Hz, 1H), 6.29 (s, 1H), 3.92 (s, 3H), 3.70 - 3.58 (m, 2H), 3.42 - 3.36 (m, 2H), 2.83 - 2.70 (m, 1H), 2.56 - 2.38 (m, 2H), 2.31 (br s, 6H), 2.17 - 2.05 (m, 1H), 1.96 - 1.77 (m, 3H), 1.54 (s, 9H), LCMS: m/z (M+H)+=496.3.
Purity: 93.8 %, Yield: 10.8 %

### Example 42:

### tert-butyl (4-(4-amino-7-((1R,5S,6s)-6-(dimethylamino)-3-azabicyclo[3.1.0]hexan-3-yl)pyrrolo[2,1-f][1,2,4]triazin-5-yl)-2-methoxyphenyl)carbamate

1H NMR (400 MHz, Methanol-d4) δ = 7.92 (d, J = 8.2 Hz, 1H), 7.67 (s, 1H), 7.04 (d, J = 1.6 Hz, 1H), 7.00 (dd, J = 1.7, 8.3 Hz, 1H), 6.07 (s, 1H), 3.95 (d, J = 9.2 Hz, 2H), 3.91 (s, 3H), 3.24 (d, J = 9.2 Hz, 2H), 2.45 (s, 6H), 2.10 (s, 1H), 1.77 (s, 2H), 1.54 (s, 9H), LCMS: m/z (M+H)+=480.2.
Purity: 93.8 %, Yield: 13.4 %

### Example 44:

### tert-butyl (4-(4-amino-7-(4-(methylamino)piperidin-1-yl)pyrrolo[2,1-f][1,2,4]triazin-5-yl)-2-methoxyphenyl)carbamate

1H NMR (400 MHz, DMSO-d6) δ = 8.30 (s, 1H), 7.98 (s, 1H), 7.82 (s, 1H), 7.80 (d, J = 8.2 Hz, 1H), 7.05 (d, J = 1.5 Hz, 1H), 6.97 (dd, J = 1.5, 8.2 Hz, 1H), 6.26 (s, 1H), 3.85 (s, 3H), 3.73 (d, J = 11.2 Hz, 2H), 2.95 - 2.85 (m, 1H), 2.73 (t, J = 11.4 Hz, 2H), 2.47 (s, 3H), 2.07 - 1.99 (m, 2H), 1.69 - 1.56 (m, 2H), 1.47 (s, 9H). LCMS: m/z (M+H)+ = 468.2.
Purity : 99% Yield 31%

### Example 45:

### tert-butyl (4-(4-amino-7-(4-amino-4-methylpiperidin-1-yl)pyrrolo[2,1-f][1,2,4]triazin-5-yl)-2-methoxyphenyl)carbamate

1H NMR (400 MHz, DMSO-d6) δ = 8.00 (br s, 3H), 7.84 (s, 1H), 7.81 (d, J = 8.0 Hz, 1H), 7.04 (d, J = 2.0 Hz, 1H), 6.97 (dd, J = 1.6, 8.0 Hz, 1H), 6.30 (s, 1H), 3.85 (s, 3H), 3.56 - 3.48 (m, 2H), 3.05 (t, J = 10.0 Hz, 2H), 2.00 - 1.87 (m, 2H), 1.84 - 1.75 (m, 2H), 1.47 (s, 9H), 1.36 (s, 3H). LCMS: m/z (M+H)+ = 468.3.
Purity : 99% Yield 17%

### Example 46:

### tert-butyl (4-(4-amino-7-((3S,4R)-4-amino-3-fluoropiperidin-1-yl)pyrrolo[2,1-f][1,2,4]triazin-5-yl)-2-methoxyphenyl)carbamate

LCMS: m/z (M+H)+=472.2. 1H NMR (400 MHz, METHANOL-d4) δ = 7.94 (d, J = 8.1 Hz, 1H), 7.78 (s, 1H), 7.06 (d, J = 1.4 Hz, 1H), 7.02 (dd, J = 1.6, 8.3 Hz, 1H), 6.31 (s, 1H), 4.99 - 4.91 (m, 1H), 4.14 - 4.02 (m, 1H), 3.92 (s, 3H), 3.85 (d, J = 12.6 Hz, 1H), 3.42 - 3.36 (m, 1H), 3.23 - 3.11 (m, 1H), 2.93 (t, J = 12.0 Hz, 1H), 2.25 - 2.12 (m, 1H), 2.00 - 1.88 (m, 1H), 1.54 (s, 9H).
Purity : 98% Yield : 14%

### Example 48:

### tert-butyl (4-(4-amino-7-(4-amino-3-methylpiperidin-1-yl)pyrrolo[2,1-f][1,2,4]triazin-5-yl)-2-methoxyphenyl)carbamate

LCMS: m/z (M+H)+=468.2. 1H NMR (400 MHz, DMSO-d6) δ = 7.99 (s, 1H), 7.94 (br s, 3H), 7.83 (s, 1H), 7.80 (d, J = 8.3 Hz, 1H), 7.04 (d, J = 1.8 Hz, 1H), 6.97 (dd, J = 1.7, 8.2 Hz, 1H), 6.27 (s, 1H), 3.85 (s, 3H), 3.78 (d, J = 11.9 Hz, 1H), 3.67 (dd, J = 2.7, 11.8 Hz, 1H), 2.99 - 2.86 (m, 1H), 2.82 - 2.71 (m, 1H), 2.59 - 2.56 (m, 1H), 2.07 - 1.98 (m, 1H), 1.96 - 1.86 (m, 1H), 1.72 (dq, J = 3.4, 12.3 Hz, 1H), 1.47 (s, 9H), 1.00 (d, J = 6.5 Hz, 3H).
Purity : 99% Yield 33%

### Example 49:

### tert-butyl (4-(4-amino-7-(4-amino-3,5-dimethylpiperidin-1-yl)pyrrolo[2,1-f][1,2,4]triazin-5-yl)-2-methoxyphenyl)carbamate

1H NMR (400 MHz, METHANOL-d4) δ = 7.93 (d, J = 8.3 Hz, 1H), 7.80 - 7.75 (m, 1H), 7.09 - 7.00 (m, 2H), 6.32 - 6.22 (m, 1H), 3.92 (s, 3H), 3.73 - 3.51 (m, 1H), 3.39 - 3.34 (m, 1H), 3.05 - 2.99 (m, 1H), 2.72 (t, J = 12.0 Hz, 1H), 2.64 - 2.45 (m, 1H), 2.29 - 2.17 (m, 2H), 1.98 - 1.79 (m, 1H), 1.54 (s, 9H), 1.11 - 0.97 (m, 6H).
Purity : 93% Yield 18%

### Example 51:

### tert-butyl (4-(4-amino-7-(5-amino-2-azabicyclo[2.2.1]hepten-2-yl)pyrrolo[2,1-f][1,2,4]triazin-5-yl)-2-methoxyphenyl)carbamate

LCMS: m/z (M+H)+=466.2. 1H NMR (400 MHz, DMSO-d6) δ = 8.30 (br s, 1H), 7.98 (s, 1H), 7.79 (d, J = 7.9 Hz, 1H), 7.69 (s, 1H), 7.04 (d, J = 1.5 Hz, 1H), 6.97 (dd, J = 1.5, 8.3 Hz, 1H), 6.08 (s, 1H), 4.40 (s, 1H), 3.85 (s, 3H), 3.52 (dd, J = 3.7, 9.5 Hz, 1H), 3.17 - 3.13 (m, 1H), 2.98 (d, J = 9.2 Hz, 1H), 2.46 - 2.44 (m, 1H), 2.13 - 2.02 (m, 1H), 1.90 (d, J = 10.5 Hz, 1H), 1.68 - 1.61 (m, 1H), 1.47 (s, 9H), 1.37 - 1.28 (m, 1H).
Purity : 99% Yield 5%

### Example 52:

### tert-butyl (4-(4-amino-7-(3-oxo-2,8-diazaspiro[4.5]decan-8-yl)pyrrolo[2,1-f][1,2,4]triazin-5-yl)-2-methoxyphenyl)carbamate

1H NMR (400 MHz, DMSO-d6) δ = 7.97 (s, 1H), 7.84 - 7.74 (m, 2H), 7.55 (s, 1H), 7.06 (d, J = 1.6 Hz, 1H), 6.97 (dd, J = 1.6, 8.4 Hz, 1H), 6.24 (s, 1H), 3.85 (s, 3H), 3.27 - 3.17 (m, 2H), 3.15 - 3.04 (m, 4H), 2.12 (s, 2H), 1.80- 1.65 (m, 4H), 1.47 (s, 9H). LCMS: m/z (M+H)+ =508.3.
Purity : 99% Yield 16%

### Example 53:

### tert-butyl (4-(4-amino-7-(1-oxo-2,8-diazaspiro[4.5]decan-8-yl)pyrrolo[2,1-f][1,2,4]triazin-5-yl)-2-methoxyphenyl)carbamate

1H NMR (400 MHz, DMSO-d6) δ = 7.97 (s, 1H), 7.82 (s, 1H), 7.79 (d, J = 8.4 Hz, 1H), 7.59 (s, 1H), 7.06 (d, J = 1.2 Hz, 1H), 6.98 (d, J = 8.0 Hz, 1H), 6.25 (s, 1H), 3.85 (s, 3H), 3.71 - 3.60 (m, 2H), 3.20 (t, J = 6.8 Hz, 2H), 2.82 (t, J = 10.6 Hz, 2H), 2.01 (t, J = 6.8 Hz, 2H), 1.93 - 1.84 (m, 2H), 1.52 - 1.35 (m, 11H). LCMS: m/z (M+H)+ =508.2.
Purity : 96% Yield 6%

### Example 54:

### tert-butyl (4-(4-amino-7-(2-oxo-1,8-diazaspiro[4.5]decan-8-yl)pyrrolo[2,1-f][1,2,4]triazin-5-yl)-2-methoxyphenyl)carbamate

LCMS: m/z (M+H)+=508.3. 1H NMR (400 MHz, DMSO-d6) δ = 8.00 (s, 1H), 7.97 (s, 1H), 7.84 - 7.77 (m, 2H), 7.05 (d, J = 1.6 Hz, 1H), 6.97 (dd, J = 1.7, 8.2 Hz, 1H), 6.23 (s, 1H), 3.85 (s, 3H), 3.44 - 3.36 (m, 2H), 3.12 - 3.04 (m, 2H), 2.22 (t, J = 7.9 Hz, 2H), 1.90 (t, J = 7.9 Hz, 2H), 1.83 - 1.66 (m, 4H), 1.47 (s, 9H).
Purity : 99% Yield 5%

### Example 55:

### tert-butyl (4-(4-amino-7-(1-oxo-2,7-diazaspiro[3.5]nonan-7-yl)pyrrolo[2,1-f][1,2,4]triazin-5-yl)-2-methoxyphenyl)carbamate

1H NMR (400 MHz, DMSO-d6) δ = 7.98 (s, 1H), 7.82 (s, 1H), 7.81 - 7.77 (m, 2H), 7.06 (d, J = 1.6 Hz, 1H), 6.97 (dd, J = 1.6, 8.8 Hz, 1H), 6.25 (s, 1H), 3.85 (s, 3H), 3.57 - 3.45 (m, 2H), 3.10 (s, 2H), 3.00 (t, J = 8.8 Hz, 2H), 2.00 - 1.91 (m, 2H), 1.90 - 1.83 (m, 2H), 1.47 (s, 9H). LCMS: m/z (M+H)+ =494.3.
Purity : 98% Yield 8%

### Example 56:

### tert-butyl (4-(4-amino-7-(2-oxo-1,7-diazaspiro[3.5]nonan-7-yl)pyrrolo[2,1-f][1,2,4]triazin-5-yl)-2-methoxyphenyl)carbamate

LCMS: m/z (M+H)+=494.2. 1H NMR (400 MHz, DMSO-d6) δ = 8.37 (s, 1H), 8.12 - 7.93 (m, 1H), 7.86 (d, J = 8.1 Hz, 1H), 7.08 (s, 1H), 7.02 (dd, J = 1.8, 8.2 Hz, 1H), 6.48 (s, 1H), 3.83 (s, 3H), 3.33 - 3.18 (m, 4H), 2.67 (s, 2H), 1.92 - 1.76 (m, 4H), 1.45 (s, 9H).
Purity : 97% Yield 12%

### Example 58:

### tert-butyl (4-(4-amino-7-(4-(methylamino)piperidin-1-yl)pyrrolo[2,1-f][1,2,4]triazin-5-yl)-2-methoxyphenyl)carbamate

1H NMR (400 MHz, DMSO-d6) δ = 8.37 (br s, 2H), 7.97 (s, 1H), 7.85 (s, 1H), 7.81 (d, J = 8.3 Hz, 1H), 7.05 (d, J = 1.8 Hz, 1H), 6.98 (dd, J = 1.8, 8.2 Hz, 1H), 6.28 (s, 1H), 3.85 (s, 3H), 3.73 (d, J = 11.5 Hz, 2H), 2.94 - 2.86 (m, J = 5.4 Hz, 2H), 2.75 - 2.65 (m, 2H), 2.60 (t, J = 5.1 Hz, 3H), 1.87 - 1.77 (m, 3H), 1.47 - 1.39 (m, 2H). LCMS: m/z (M+H)+ = 482.3.
Purity 99.2%, Yield : 18%

### Example 59:

### tert-butyl (4-(4-amino-7-(4-(aminomethyl)piperidin-1-yl)pyrrolo[2,1-f][1,2,4]triazin-5-yl)-2-methoxyphenyl)carbamate

1H NMR (400 MHz, DMSO-d6) δ = 8.25 (br s, 1H), 7.99 (s, 1H), 7.83 - 7.77 (m, 2H), 7.05 (d, J = 1.8 Hz, 1H), 6.97 (dd, J = 1.7, 8.2 Hz, 1H), 6.25 (s, 1H), 3.85 (s, 3H), 3.72 (d, J = 11.8 Hz, 2H), 2.82 - 2.74 (m, 2H), 2.70 - 2.60 (m, 2H), 1.81 (d, J = 12.0 Hz, 2H), 1.76 - 1.66 (m, 1H), 1.47 (s, 9H), 1.45 - 1.36 (m, 2H). LCMS: m/z (M+H)+ = 468.1
Purity 99.48%, Yield : 14%

### Example 60:

### 1-fluoro-2-methylpropan-2-yl (4-(4-amino-7-(4-aminopiperidin-1-yl-6-methylpyrrolo[2,1-f][1,2,4]triazin-5-yl)-2-methoxyphenyl)carbamate

1H NMR (400 MHz, DMSO-d6) δ = 8.43 (s, 1H), 8.28 (s, 1H), 7.79 (s, 1H), 7.75 (d, J = 8.0 Hz, 1H), 6.98 (d, J = 1.6 Hz, 1H), 6.89 (dd, J = 1.6, 8.0 Hz, 1H), 4.56 (d, J = 47.6 Hz, 2H), 3.82 (s, 3H), 3.41 (t, J = 11.0 Hz, 2H), 3.11 (d, J = 11.6 Hz, 2H), 3.06 - 2.98 (m, 1H), 2.04 (s, 3H), 1.95 - 1.89 (m, 2H), 1.66 - 1.54 (m, 2H), 1.47 (d, J = 2.0 Hz, 6H). LCMS: m/z (M+H)+ = 486.1.
Purity : 98%, Yield : 26%

### Example 61:

### 1-fluoro-2-methylpropan-2-yl (4-(4-amino-6-methyl-7-(4-(methylamino)piperidin-1-yl)pyrrolo[2,1-f][1,2,4]triazin-5-yl)-2-methoxyphenyl)carbamate

1H NMR (400 MHz, DMSO-d6) δ = 8.34 (s, 1H), 8.28 (s, 1H), 7.79 (s, 1H), 7.75 (d, J = 8.3 Hz, 1H), 6.98 (d, J = 1.8 Hz, 1H), 6.89 (dd, J = 1.6, 8.1 Hz, 1H), 4.56 (d, J = 47.6 Hz, 2H), 3.82 (s, 3H), 3.40 (t, J = 10.9 Hz, 2H), 3.14 (d, J = 11.9 Hz, 2H), 2.91 - 2.77 (m, 1H), 2.47 (s, 3H), 2.04 (s, 3H), 2.03 - 1.97 (m, 2H), 1.62 - 1.51 (m, 2H), 1.47 (d, J = 2.1 Hz, 6H).
Purity : 98.87%, Yield : 14.83%

### Example 62:

### 1-fluoro-2-methylpropan-2-yl (4-(4-amino-7-(4-(dimethylamino)piperidin-1-yl)-6-methylpyrrolo[2,1-f][1,2,4]triazin-5-yl)-2-methylmethoxy)carbamate

1H NMR (400 MHz, DMSO-d6) δ = 8.27 - 8.25 (m, 2H), 7.79 (s, 1H), 7.75 (d, J = 8.0 Hz, 1H), 6.98 (d, J = 1.6 Hz, 1H), 6.89 (dd, J = 1.6, 8.0 Hz, 1H), 4.55 (d, J = 47.6 Hz, 2H), 3.82 (s, 3H), 3.46 - 3.32 (m, 2H), 3.14 (d, J = 11.6 Hz, 2H), 2.39 - 2.32 (m, 1H), 2.27 (s, 6H), 2.04 (s, 3H), 1.83 (d, J = 10.8 Hz, 2H), 1.61 - 1.49 (m, 2H), 1.47 (d, J = 2.0 Hz, 6H). LCMS: m/z (M+H)+ = 514.3.
Purity : 100%, Yield : 59%

### Example 63:

### 1-fluoro-2-methylpropan-2-yl (4-(4-amino-6-methyl-7-(4-(2-(methylamino)ethyl)piperidin-1-yl)pyrrolo[2,1-f][1,2,4]triazin-5-yl)-2-methoxyphenyl)carbamate

1H NMR (400 MHz, DMSO-d6) δ = 8.39 (s, 1H), 8.26 (s, 1H), 7.78 (s, 1H), 7.75 (d, J = 8.4 Hz, 1H), 6.98 (d, J = 1.6 Hz, 1H), 6.89 (dd, J = 1.6, 8.0 Hz, 1H), 4.55 (d, J = 47.6 Hz, 2H), 3.82 (s, 3H), 3.43 - 3.35 (m, 2H), 3.07 (d, J = 11.6 Hz, 2H), 2.81 (t, J = 7.2 Hz, 2H), 2.46 (s, 3H), 2.04 (s, 3H), 1.73 (d, J = 11.2 Hz, 2H), 1.61 - 1.50 (m, 3H), 1.47 (d, J = 2.0 Hz, 6H), 1.36 - 1.24 (m, 2H). LCMS: m/z (M+H)+ = 528.2.
Purity : 98.62%, Yields : 48%

### Example 64:

### 1-fluoro-2-methylpropan-2-yl (4-(4-amino-6-methyl-7-(2-(4-(methylamino)piperidin-1-yl)ethyl)pyrrolo[2,1-f][1,2,4]triazin-5-yl)-2-methoxyphenyl)carbamate

1H NMR (400 MHz, DMSO-d6) δ = 8.30 (s, 1H), 8.28 (s, 1H), 7.84 (s, 1H), 7.76 (d, J = 8.0 Hz, 1H), 6.97 (d, J = 1.6 Hz, 1H), 6.89 (dd, J = 1.8, 8.0 Hz, 1H), 4.56 (d, J = 47.6 Hz, 2H), 3.13 - 2.98 (m, 4H), 2.81 - 2.72 (m, 1H), 2.56 - 2.52 (m, 2H), 2.46 (s, 3H), 2.08 (s, 3H), 2.00 (t, J = 11.0 Hz, 2H), 1.94 - 1.90 (m, 2H), 1.47 (d, J = 2.0 Hz, 6H), 1.46 - 1.36 (m, 2H). LCMS: m/z (M+H)+ = 528.2.
Purity : 99%, Yield : 34%

### Example 65:

### 1-fluoro-2-methylpropan-2-yl (4-(4-amino-7-(4-aminopiperidin-1-yl)pyrrolo[2,1-f][1,2,4]triazin-5-yl)-2-methoxyphenyl)carbamate

1H NMR (400 MHz, DMSO-d6) δ = 8.41 (s, 1H), 8.25 (s, 1H), 7.82 (s, 1H), 7.74 (d, J = 8.4 Hz, 1H), 7.06 (d, J = 1.6 Hz, 1H), 6.97 (dd, J = 1.6, 8.4 Hz, 1H), 6.25 (s, 1H), 4.55 (d, J = 47.6 Hz, 2H), 3.85 (s, 3H), 3.68 (d, J = 12.0 Hz, 2H), 3.01 - 2.92 (m, 1H), 2.74 (t, J = 10.4 Hz, 2H), 1.98 - 1.85 (m, 2H), 1.65 - 1.54 (m, 2H), 1.47 (d, J = 2.0 Hz, 6H). LCMS: m/z (M+H)+ = 472.1.
Purity : 98%, Yield: 11%.

### Example 66:

### 1-fluoro-2-methylpropan-2-yl (4-(4-amino-7-(4-(2-(methylamino)ethyl)piperidin-1-yl)pyrrolo[2,1-f][1,2,4]triazin-5-yl)-2-methoxyphenyl)carbamate

1H NMR (400 MHz, DMSO-d6) δ = 8.24 (s, 1H), 7.80 (s, 1H), 7.73 (d, J = 8.0 Hz, 1H), 7.06 (d, J = 1.2 Hz, 1H), 6.98 (dd, J = 1.6, 8 Hz, 1H), 6.22 (s, 1H), 4.55 (d, J = 47.6 Hz, 2H), 3.85 (s, 3H), 3.67 (d, J = 11.2 Hz, 2H), 2.68 - 2.59 (m, 2H), 2.56 - 2.53 (m, 2H), 2.29 (s, 3H), 1.74 (d, J = 10.4 Hz, 2H), 1.55 - 1.28 (m, 11H). LCMS: m/z (M+H)+ = 514.3.
Purity : 96%, Yield : 19%

### Example 67:

### 1-fluoro-2-methylpropan-2-yl (4-(4-amino-7-(2-(4-(methylamino)piperidin-1-yl)ethyl)pyrrolo[2,1-f][1,2,4]triazin-5-yl)-2-methoxyphenyl)carbamate

1H NMR (400 MHz, DMSO-d6) δ = 8.33 (s, 1H), 8.26 (s, 1H), 7.89 (s, 1H), 7.73 (d, J = 7.6 Hz, 1H), 7.05 (d, J = 1.6 Hz, 1H), 6.97 (dd, J = 1.6, 8.0 Hz, 1H), 6.60 (s, 1H), 4.55 (d, J = 47.6 Hz, 2H), 3.85 (s, 3H), 3.07 - 3.02 (m, 2H), 2.99 - 2.91 (m, 2H), 2.66 (d, J = 8.0 Hz, 2H), 2.49 - 2.43 (m, 1H), 2.39 (s, 3H), 2.05 - 1.94 (m, 2H), 1.90 - 1.79 (m, 2H), 1.47 (d, J = 2.0 Hz, 6H), 1.42 - 1.29 (m, 2H). LCMS: m/z (M+H)+ = 514.2.
Purity : 95% Yield 6%.

### Example 68:

### 1-fluoro-2-methylpropan-2-yl (4-(4-amino-7-(2-(4-aminopiperidin-1-yl)ethyl)pyrrolo[2,1-f][1,2,4]triazin-5-yl)-2-methoxyphenyl)carbamate

1H NMR (400 MHz, DMSO-d6) δ = 8.35 (s, 2H), 8.26 (s, 1H), 7.89 (s, 1H), 7.74 (d, J = 7.6 Hz, 1H), 7.05 (d, J = 1.2 Hz, 1H), 7.00 - 6.94 (m, 1H), 6.61 (s, 1H), 4.55 (d, J = 47.6 Hz, 2H), 3.85 (s, 3H), 3.04 (t, J = 7.6 Hz, 2H), 2.95 (d, J = 11.2 Hz, 2H), 2.90 - 2.84 (m, 1H), 2.70 - 2.62 (m, 2H), 2.02 (t, J = 11.4 Hz, 2H), 1.87 - 1.79 (m, 2H), 1.52 - 1.40 (m, 8H). LCMS: m/z (M+H)+ = 500.2.
Purity : 98%. Yield : 7%

### Example 69:

### 1-fluoro-2-methylpropan-2-yl (4-(4-amino-7-(2-(4-(dimethylamino)piperidin-1-yl)ethyl)pyrrolo[2,1-f][1,2,4]triazin-5-yl)-2-methoxyphenyl)carbamate

1H NMR (400 MHz, DMSO-d6) δ = 8.25 (s, 2H), 7.89 (s, 1H), 7.74 (d, J = 7.6 Hz, 1H), 7.05 (d, J = 1.6 Hz, 1H), 6.97 (dd, J = 1.6, 8.4 Hz, 1H), 6.60 (s, 1H), 4.55 (d, J = 47.6 Hz, 2H), 3.85 (s, 3H), 3.08 - 2.97 (m, 4H), 2.64 (t, J = 7.8 Hz, 2H), 2.21 (s, 6H), 2.17 - 2.09 (m, 1H), 1.97 (t, J = 11.0 Hz, 2H), 1.78 - 1.70 (m, 2H), 1.47 (d, J = 2.0 Hz, 6H), 1.44 - 1.31 (m, 2H). LCMS: m/z (M+H)+ = 528.2.
Purity : 96% Yield : 7%

### Example 70:

### 1-methylcyclopropyl (7-(4-amino-7-(4-aminopiperidin-1-yl)pyrrolo[2,1-f][1,2,4]triazin-5-yl)benzo[d][1,3]dioxo-4-yl)carbamate

1H NMR (400 MHz, DMSO-d6) δ = 9.20 (br s, 1H), 8.36 (s, 1H), 7.81 (s, 1H), 7.09 (br d, J = 8.6 Hz, 1H), 6.78 (d, J = 8.6 Hz, 1H), 6.19 (s, 1H), 6.02 (s, 2H), 3.68 - 3.66 (m, 2H), 3.13 - 3.07 (m, 1H), 2.73 (t, J = 11.1 Hz, 2H), 1.96 (d, J = 10.1 Hz, 2H), 1.75 - 1.61 (m, 2H), 1.52 (s, 3H), 0.89 - 0.81 (m, 2H), 0.70 - 0.63 (m, 2H).
Purity : 99% Yield : 26%

### Example 71:

### 1-methylcyclopropyl (4-(4-amino-7-(4-aminopiperidin-1-yl)pyrrolo[2,1-f][1,2,4]triazin-5-yl)-2-methoxyphenyl)carbamate

1H NMR (400 MHz, DMSO-d6) δ = 8.42 (s, 1H), 8.34 (s, 1H), 7.82 (s, 1H), 7.77 (d, J = 7.2 Hz, 1H), 7.05 (d, J = 1.6 Hz, 1H), 6.98 (dd, J = 1.6, 8.4 Hz, 1H), 6.25 (s, 1H), 3.83 (s, 3H), 3.67 (d, J = 12.0 Hz, 2H), 3.00 - 2.93 (m, 1H), 2.74 (t, J = 11.2 Hz, 2H), 1.94 - 1.86 (m, 2H), 1.64 - 1.54 (m, 2H), 1.53 (s, 3H), 0.89 - 0.81 (m, 2H), 0.71 - 0.61 (m, 2H). LCMS: m/z (M+H)+ = 452.2.
Purity 99% Yield : 7%

### Example 72:

### 1,1,1-trifluoro-2-methylpropan-2-yl (4-(4-amino-7-(4-aminopiperidin-1-yl)pyrrolo[2,1-f][1,2,4]triazin-5-yl)-2-methoxyphenyl)carbamate

1H NMR (400 MHz, DMSO-d6) δ = 8.73 (br s, 1H), 8.44 (br s, 1H), 7.82 (s, 1H), 7.63 (br s, 1H), 7.08 (d, J = 1.6 Hz, 1H), 6.99 (dd, J = 1.6, 8.2 Hz, 1H), 6.26 (s, 1H), 3.85 (s, 3H), 3.69 (d, J = 11.6 Hz, 2H), 3.06 - 2.96 (m, 1H), 2.74 (t, J = 11.1 Hz, 2H), 1.97 - 1.89 (m, 2H), 1.79 - 1.56 (m, 8H). LCMS: m/z (M+H)+ = 508.2.
Purity : 99% Yield : 20%

### Example 73:

### tert-butyl (7-(4-amino-7-(4-aminopiperidin-1-yl)pyrrolo[2,1-f][1,2,4]triazin-5-yl)benzo[d][1,3]dioxo-4-yl)carbamate

1H NMR (400 MHz, DMSO-d6) δ = 8.94 (s, 1H), 8.41 (s, 1H), 7.81 (s, 1H), 7.06 (d, J = 8.4 Hz, 1H), 6.77 (d, J = 8.4 Hz, 1H), 6.18 (s, 1H), 6.02 (s, 2H), 3.65 (d, J = 12.0 Hz, 2H), 3.08 - 2.98 (m, 1H), 2.72 (t, J = 10.8 Hz, 2H), 1.97 - 1.89 (m, 2H), 1.68 - 1.58 (m, 2H), 1.46 (s, 9H) LCMS: m/z (M+H)+ =468.1.
Purity : 99% Yield : 34%

### Example 74:

### tert-butyl (4-(4-amino-7-(4-aminopiperidin-1-yl)-6-methylpyrrolo[2,1-f][1,2,4]triazin-5-yl)-2-methoxyphenyl)carbamate

1H NMR (400 MHz, DMSO-d6) δ = 8.01 (s, 1H), 7.84 - 7.77 (m, 2H), 6.96 (d, J = 1.6 Hz, 1H), 6.88 (dd, J = 1.6, 8.2 Hz, 1H), 3.82 (s, 3H), 3.45 - 3.39 (m, 2H), 3.18 - 3.09 (m, 3H), 2.04 (s, 3H), 1.98 - 1.90 (m, 2H), 1.67 - 1.57 (m, 2H), 1.47 (s, 9H). LCMS: m/z (M+H)+ = 468.3.
Purity : 97% Yield : 7%

### Example 75:

### tert-butyl (4-(4-amino-6-methyl-7-(4-(methylamino)piperidin-1-yl)pyrrolo[2,1-f][1,2,4]triazin-5-yl)-2-methoxyphenyl)carbamate

1H NMR (400 MHz, DMSO-d6) δ = 8.38 (s, 1H), 8.01 (s, 1H), 7.85 - 7.76 (m, 2H), 6.96 (d, J = 1.6 Hz, 1H), 6.88 (dd, J = 1.5, 8.1 Hz, 1H), 3.82 (s, 3H), 3.40 (t, J = 11.1 Hz, 2H), 3.14 (d, J = 11.9 Hz, 2H), 2.90 - 2.80 (m, 1H), 2.47 (s, 3H), 2.04 (s, 3H), 2.03 - 1.99 (m, 2H), 1.64 - 1.50 (m, 2H), 1.47 (s, 9H). LCMS: m/z (M+H)+=482.3.
Purity : 98% Yield : 33%

### Example 76:

### tert-butyl (4-(4-amino-7-(4-(dimethylamino)piperidin-1-yl)-6-methylpyrrolo[2,1-f][1,2,4]triazin-5-yl)-2-methoxyphenyl)carbamate

LCMS: m/z (M+H)+=496.3. 1H NMR (400 MHz, DMSO-d6) δ = 8.21 (s, 1H), 8.00 (s, 1H), 7.83 - 7.77 (m, 2H), 6.97 (d, J = 2 Hz, 1H), 6.88 (dd, J = 1.6, 8 Hz, 1H), 3.82 (s, 3H), 3.40 (t, J = 10.8 Hz, 2H), 3.14 (d, J = 12.0 Hz, 2H), 2.42 - 2.37 (m, 1H), 2.30 (s, 6H), 2.04 (s, 3H), 1.84 (d, J = 10.4 Hz, 2H), 1.62 - 1.51 (m, 2H), 1.47 (s, 9H).
Purity : 99% Yield : 57%

### Example 77:

### tert-butyl (4-(4-amino-6-methyl-7-(4-(2-(methylamino)ethyl)piperidin-1-yl)pyrrolo[2,1-f][1,2,4]triazin-5-yl)-2-methoxyphenyl)carbamate

1H NMR (400 MHz, DMSO-d6) δ = 8.38 (s, 1H), 8.01 (s, 1H), 7.83 - 7.76 (m, 2H), 6.96 (d, J = 1.6 Hz, 1H), 6.88 (dd, J = 1.8, 8 Hz, 1H), 3.82 (s, 3H), 3.43 - 3.33 (m, 2H), 3.11 - 3.03 (m, 2H), 2.83 - 2.77 (m, 2H), 2.46 (s, 3H), 2.04 (s, 3H), 1.76 - 1.68 (m, 2H), 1.55 - 1.47 (m, 12H), 1.38 - 1.19 (m, 2H). LCMS: m/z (M+H)+ = 510.4.
Purity 99% Yield 22%

### Example 78:

### tert-butyl (4-(4-amino-7-(4-aminopiperidin-1-yl)-6-methylpyrrolo[2,1-f][1,2,4]triazin-5-yl)-2-methoxyphenyl)carbamate

1H NMR (400 MHz, DMSO-d6) δ = 8.29 (s, 2H), 8.01 (s, 1H), 7.84 (s, 1H), 7.82 (d, J = 8.4 Hz, 1H), 6.96 (d, J = 1.6 Hz, 1H), 6.88 (dd, J = 1.6, 8.0 Hz, 1H), 3.82 (s, 3H), 3.10 - 2.99 (m, 4H), 2.90 - 2.80 (m, 1H), 2.56 - 2.52 (m, 2H), 2.50 (br s, 3H), 2.08 (s, 3H), 2.04 - 1.91 (m, 4H), 1.52 - 1.42 (m, 11H). LCMS: m/z (M+H)+ = 510.2.
Purity : 99% Yield 45%

### Example 79:

### tert-butyl (4-(4-amino-7-(4-(aminomethyl)-4-methylpiperidin-1-yl)pyrrolo[2,1-f][1,2,4]triazin-5-yl)-2-methoxyphenyl)carbamate

1H NMR (400 MHz, DMSO-d6) δ = 8.17 (s, 1H), 7.99 (s, 1H), 7.82 - 7.78 (m, 2H), 7.05 (d, J = 1.6 Hz, 1H), 6.97 (dd, J = 1.6, 8.4 Hz, 1H), 6.26 (s, 1H), 3.85 (s, 3H), 3.07 - 2.97 (m, 2H), 2.80 (s, 2H), 1.74 - 1.63 (m, 2H), 1.56 - 1.49 (m, 2H), 1.47 (s, 9H), 1.06 (s, 3H). LCMS: m/z (M+H)+ = 482.2.
Purity : 99% Yield : 17%

### Example 80:

### tert-butyl (4-(4-amino-7-(8-amino-5-azaspiro[2.5]octen-5-yl)pyrrolo[2,1-f][1,2,4]triazin-5-yl)-2-methoxyphenyl)carbamate

1H NMR (400 MHz, DMSO-d6) δ = 8.33 (s, 1H), 7.98 (s, 1H), 7.86 - 7.73 (m, 2H), 7.04 (d, J = 1.6 Hz, 1H), 6.96 (d, J = 8.0 Hz, 1H), 6.20 (s, 1H), 3.85 (s, 3H), 3.22 - 3.18 (m, 3H), 2.85 (d, J = 11.6 Hz, 1H), 2.75 - 2.73 (m, 1H), 2.08 - 1.89 (m, 1H), 1.76 - 1.61 (m, 1H), 1.47 (s, 9H), 0.70 - 0.56 (m, 2H), 0.44 - 0.29 (m, 2H). LCMS: m/z (M+H)+ = 480.2.
Purity : 99% Yield : 14%

### Example 81:

### tert-butyl (4-(4-amino-7-(4-(1-aminoethyl)piperidin-1-yl)pyrrolo[2,1-f][1,2,4]triazin-5-yl)-2-methoxyphenyl)carbamate

1H NMR (400 MHz, DMSO-d6) δ = 8.43 (s, 1H), 7.98 (s, 1H), 7.87 - 7.73 (m, 2H), 7.05 (d, J = 1.2 Hz, 1H), 6.97 (dd, J = 1.2, 8.0 Hz, 1H), 6.24 (s, 1H), 3.85 (s, 3H), 3.75 (d, J = 11.2 Hz, 2H), 2.95 - 2.90 (m, 1H), 2.60 (t, J = 10.0 Hz, 2H), 1.84 - 1.77 (m, 1H), 1.76 - 1.69 (m, 1H), 1.47 (s, 12H), 1.11 (d, J = 6.4 Hz, 3H). LCMS: m/z (M+H)+ = 482.2.
Purity 99% Yield 7%

### Example 82:

### tert-butyl (4-(4-amino-7-(4-amino-4-ethylpiperidin-1-yl)pyrrolo[2,1-f][1,2,4]triazin-5-yl)-2-methoxyphenyl)carbamate

1H NMR (400 MHz, DMSO-d6) δ = 8.37 (br s, 1H), 7.98 (s, 1H), 7.84 - 7.75 (m, 2H), 7.05 (s, 1H), 7.01 - 6.93 (m, 1H), 6.26 (s, 1H), 3.85 (s, 3H), 3.32 - 3.17 (m, 4H), 1.80 - 1.65 (m, 4H), 1.59 (q, J = 7.6 Hz, 2H), 1.47 (s, 9H), 0.90 (t, J = 7.2 Hz, 3H). LCMS: m/z (M+H)+ = 482.2.
Purity : 99% Yield 18%

### Example 83:

### tert-butyl (4-(4-amino-7-(4-amino-4-(trifluoromethyl)piperidin-1-yl)pyrrolo[2,1-f][1,2,4]triazin-5-yl)-2-methoxyphenyl)carbamate

1H NMR (400 MHz, DMSO-d6) δ = 8.40 (s, 1H), 7.99 (s, 1H), 7.85 - 7.75 (m, 2H), 7.06 (d, J = 1.5 Hz, 1H), 6.98 (dd, J = 1.6, 8.3 Hz, 1H), 6.28 (s, 1H), 3.85 (s, 3H), 3.59 -3.51 (m, 2H), 3.07 (t, J = 10.7 Hz, 2H), 1.93 - 1.82 (m, 2H), 1.59 (d, J = 12.3 Hz, 2H), 1.47 (s, 9H) LCMS: m/z (M+H)+ = 522.2
Purity : 95% Yield 10%

### Example 84:

### tert-butyl (4-(4-amino-7-(4-(methylamino)-4-(trifluoromethyl)piperidin-1-yl)pyrrolo[2,1-f][1,2,4]triazin-5-yl)-2-methoxyphenyl)carbamate

1H NMR (400 MHz, DMSO-d6) δ = 7.97 (s, 1H), 7.82 (s, 1H), 7.79 (d, J = 8.1 Hz, 1H), 7.07 (d, J = 1.8 Hz, 1H), 6.98 (dd, J = 1.7, 8.2 Hz, 1H), 6.28 (s, 1H), 3.85 (s, 3H), 3.53 (d, J = 11.0 Hz, 2H), 3.05 - 2.90 (m, 2H), 2.34 (s, 3H), 1.91 - 1.74 (m, 4H), 1.47 (s, 9H). LCMS: m/z (M+H)+ = 536.1.
Purity : 95% Yield 26%

### Example 85:

### tert-butyl (4-(4-amino-7-((3S,4S)-4-amino-3-(trifluoromethyl)piperidin-1-yl)pyrrolo[2,1-f][1,2,4]triazin-5-yl)-2-methoxyphenyl)carbamate

1H NMR (400 MHz, METHANOL-d4) δ = 7.95 (d, J = 8.0 Hz, 1H), 7.81 (s, 1H), 7.09 (s, 1H), 7.05 (d, J = 9.6 Hz, 1H), 6.38 (s, 1H), 3.94 (s, 3H), 3.70 - 3.60 (m, 3H), 3.40 - 3.38 (m, 1H), 3.23 - 3.21 (m, 1H), 2.92 - 2.86 (m, 1H), 2.18 - 2.11 (m, 1H), 1.90 - 1.84 (m, 1H), 1.56 (s, 9H)).
Purity 94.58% Yield 5%

### Example 86:

### tert-butyl (4-(4-amino-7-((3S,4R)-4-amino-3-(trifluoromethyl)piperidin-1-yl)pyrrolo[2,1-f][1,2,4]triazin-5-yl)-2-methoxyphenyl)carbamate

1H NMR (400 MHz, METHANOL-d4) δ = 7.93 (d, J = 8.0 Hz, 1H), 7.80 (s, 1H), 7.06 (s, 1H), 7.05 - 6.99 (m, 1H), 6.32 (s, 1H), 3.98 (d, J = 10.3 Hz, 1H), 3.92 (s, 3H), 3.70 - 3.65 (m, 1H), 3.13 - 3.08 (m, 1H), 2.90 (dt, J = 2.3, 12.3 Hz, 1H), 2.78 - 2.71 (m, 1H), 2.66 - 2.59 (m, 1H), 2.09 - 2.02 (m, 1H), 1.86 - 1.75 (m, 1H), 1.54 (s, 9H)).
Purity 99.15% Yield 6%

### Example 87:

### tert-butyl (4-(4-amino-7-((3S,4R)-3-fluoro-4-(methylamino)piperidin-1-yl)pyrrolo[2,1-f][1,2,4]triazin-5-yl)-2-methoxyphenyl)carbamate

1H NMR (400 MHz, METHANOL-d4) δ = 7.94 (d, J = 8.0 Hz, 1H), 7.78 (s, 1H), 7.05 (s, 1H), 7.02 (d, J = 8.9 Hz, 1H), 6.31 (s, 1H), 5.26 - 5.06 (m, 1H), 4.26 - 4.14 (m, 1H), 4.01 - 3.88 (m, 4H), 3.56 - 3.39 (m, 1H), 3.25 - 3.06 (m, 1H), 2.92 (t, J = 11.6 Hz, 1H), 2.81 (s, 3H), 2.31 - 2.18 (m, 1H), 2.16 - 2.07 (m, 1H), 1.54 (s, 9H).
Purity : 98% Yield 40%

### Example 89:

### 1-(4-amino-5-(4-((tert-butoxycarbonyl)amino)-3-methoxyphenyl)pyrrolo[2,1-f][1,2,4]triazin-7-yl)piperidine-4-carboxylic acid

1H NMR (400 MHz, DMSO-d6) δ = 12.15 (br s, 1H), 7.98 (s, 1H), 7.82 (s, 1H), 7.79 (d, J = 8.0 Hz, 1H), 7.06 (d, J = 2.0 Hz, 1H), 6.98 (dd, J = 2.0, 8.0 Hz, 1H), 6.24 (s, 1H), 3.85 (s, 3H), 3.68 - 3.55 (m, 2H), 2.85 - 2.72 (m, 2H), 2.45 - 2.39 (m, 1H), 1.98 - 1.90 (m, 2H), 1.83 - 1.69 (m, 2H), 1.47 (s, 9H). LCMS: m/z (M+H)+ = 483.2.
Purity 99% Yield 26%

### Example 90:

### tert-butyl (4-(4-amino-7-(4-hydroxypiperidin-1-yl)pyrrolo[2,1-f][1,2,4]triazin-5-yl)-2-methoxyphenyl)carbamate

1H NMR (400 MHz, DMSO-d6) δ = 7.97 (s, 1H), 7.81 (s, 1H), 7.79 (d, J = 8.3 Hz, 1H), 7.05 (d, J = 1.6 Hz, 1H), 6.97 (dd, J = 1.6, 8.1 Hz, 1H), 6.22 (s, 1H), 4.71 (br s, 1H), 3.85 (s, 3H), 3.70 - 3.60 (m, 1H), 3.57 - 3.46 (m, 2H), 2.84 (t, J = 9.5 Hz, 2H), 1.91 - 1.81 (m, 2H), 1.63 - 1.52 (m, 2H), 1.47 (s, 9H) LCMS: m/z (M+H)+ = 455.2.
Purity 99% Yield 29%

### Example 91:

### tert-butyl (4-(4-amino-7-(4-amino-3,5-dimethylpiperidin-1-yl)pyrrolo[2,1-f][1,2,4]triazin-5-yl)-2-methoxyphenyl)carbamate

LCMS: m/z (M+H)+=482.2. 1H NMR (400 MHz, METHANOL-d4) δ = 7.93 (d, J = 8.3 Hz, 1H), 7.80 - 7.75 (m, 1H), 7.09 - 7.00 (m, 2H), 6.32 - 6.22 (m, 1H), 3.92 (s, 3H), 3.73 - 3.51 (m, 1H), 3.39 - 3.34 (m, 1H), 3.05 - 2.99 (m, 1H), 2.72 (t, J = 12.0 Hz, 1H), 2.64 - 2.45 (m, 1H), 2.29 - 2.17 (m, 2H), 1.98 - 1.79 (m, 1H), 1.54 (s, 9H), 1.11 - 0.97 (m, 6H).
Purity : 93% Yield 18%

### Example 92:

### tert-butyl (4-(4-amino-7-(4-(ethylamino)piperidin-1-yl)pyrrolo[2,1-f][1,2,4]triazin-5-yl)-2-methoxyphenyl)carbamate

1H NMR (400 MHz, DMSO-d6) δ = 8.34 (br s, 1H), 7.98 (s, 1H), 7.85 - 7.76 (m, 2H), 7.05 (d, J = 1.6 Hz, 1H), 6.97 (dd, J = 1.7, 8.2 Hz, 1H), 6.25 (s, 1H), 3.85 (s, 3H), 3.73 (d, J = 11.9 Hz, 2H), 3.00 - 2.92 (m, 1H), 2.89 - 2.81 (m, 2H), 2.73 (t, J = 11.3 Hz, 2H), 2.06 - 1.98 (m, 2H), 1.70 - 1.56 (m, 2H), 1.47 (s, 9H), 1.15 (t, J = 6.5 Hz, 3H) LCMS: m/z (M+H)+ = 482.2
Purity : 99% Yield 38%

### Example 93:

### tert-butyl (4-(4-amino-7-(6-amino-3-azabicyclo[3.1.1]hepten-3-yl)pyrrolo[2,1-f][1,2,4]triazin-5-yl)-2-methoxyphenyl)carbamate

1H NMR (400 MHz, DMSO-d6) δ = 8.16 (s, 1H), 8.01 (s, 1H), 7.79 (d, J = 8.4 Hz, 1H), 7.78 (s, 1H), 7.05 (d, J = 1.6 Hz, 1H), 6.98 (dd, J = 1.8, 8.1 Hz, 1H), 6.29 (s, 1H), 3.85 (s, 3H), 3.80 - 3.69 (m, 4H), 3.45 (t, J = 5.6 Hz, 1H), 2.57 - 2.52 (m, 2H), 1.83 (td, J = 5.9, 9.4 Hz, 1H), 1.53 (d, J = 9.3 Hz, 1H), 1.47 (s, 9H) LCMS: m/z (M+H)+ = 466.2
Purity 98% Yield 18%

### Example 94:

### tert-butyl (4-(4-amino-7-(4-amino-3,3-dimethylpiperidin-1-yl)pyrrolo[2,1-f][1,2,4]triazin-5-yl)-2-methoxyphenyl)carbamate

1H NMR (400 MHz, DMSO-d6) δ = 8.01 (s, 1H), 7.88 - 7.82 (m, 4H), 7.79 (d, J = 8.1 Hz, 1H), 7.03 (d, J = 1.8 Hz, 1H), 6.96 (dd, J = 1.7, 8.2 Hz, 1H), 6.24 (s, 1H), 3.85 (s, 3H), 3.75 - 3.67 (m, 1H), 3.12 - 2.99 (m, 1H), 2.80 - 2.69 (m, 1H), 2.64 - 2.53 (m, 2H), 1.93 - 1.83 (m, 2H), 1.47 (s, 9H), 1.10 (s, 3H), 1.06 (s, 3H). LCMS: m/z (M+H)+ = 482.3
Purity 97% Yield 23%

### Example 95:

### tert-butyl (4-(4-amino-7-(4-(methylcarbamoyl)piperidin-1-yl)pyrrolo[2,1-f][1,2,4]triazin-5-yl)-2-methoxyphenyl)

1H NMR (400 MHz, DMSO-d6) δ = 7.97 (s, 1H), 7.84 - 7.71 (m, 3H), 7.05 (d, J = 1.6 Hz, 1H), 6.97 (dd, J = 1.7, 8.2 Hz, 1H), 6.24 (s, 1H), 3.85 (s, 3H), 3.71 (d, J = 11.4 Hz, 2H), 2.72 - 2.63 (m, 2H), 2.58 (d, J = 4.5 Hz, 3H), 2.31 - 2.21 (m, 1H), 1.82 - 1.72 (m, 4H), 1.47 (s, 9H). LCMS: m/z (M+H)+ = 496.3.
Purity 99% Yield 6.8%

### Example 96:

### tert-butyl (4-(7-(4-acetamido-4-methylpiperidin-yl)-4-aminopyrrolo[2,1-f][1,2,4]triazin-5-yl)-2-methoxyphenyl)carbamate

1H NMR (400 MHz, DMSO-d6) δ = 7.98 (s, 1H), 7.81 (s, 1H), 7.79 (d, J = 8.0 Hz, 1H), 7.39 (s, 1H), 7.06 (d, J = 2.0 Hz, 1H), 6.97 (dd, J = 1.6, 8.0 Hz, 1H), 6.23 (s, 1H), 3.85 (s, 3H), 3.39 - 3.34 (m, 2H), 2.97 (t, J = 10.0 Hz, 2H), 2.21 (d, J = 12.8 Hz, 2H), 1.82 (s, 3H), 1.70 - 1.58 (m, 2H), 1.50 (s, 9H), 1.33 (s, 3H). LCMS: m/z (M+H)+ = 510.2.
Purity 99% Yield 18%

### Example 97:

### tert-butyl (4-(4-amino-7-(4-carbamoylpiperidin-1-yl)pyrrolo[2,1-f][1,2,4]triazin-5-yl)-2-methoxyphenyl)carbamate

1H NMR (400 MHz, DMSO-d6) δ = 7.97 (s, 1H), 7.85 - 7.75 (m, 2H), 7.29 (s, 1H), 7.06 (s, 1H), 6.97 (d, J = 8.3 Hz, 1H), 6.79 (s, 1H), 6.24 (s, 1H), 3.85 (s, 3H), 3.71 (d, J = 11.3 Hz, 2H), 2.73 - 2.62 (m, 2H), 2.30 - 2.21 (m, 1H), 1.84 - 1.70 (m, 4H), 1.47 (s, 9H). LCMS: m/z (M+H)+ = 482.2.
Purity 97% Yield 8%

### Example 98:

### tert-butyl (4-(4-amino-7-(2-(4-(dimethylamino)piperidin-1-yl)ethyl)pyrrolo[2,1-f][1,2,4]triazin-5-yl)-2-methoxyphenyl)carbamate

1H NMR (400 MHz, DMSO-d6) δ = 8.25 (s, 1H), 7.98 (s, 1H), 7.89 (s, 1H), 7.79 (d, J = 8.4 Hz, 1H), 7.04 (d, J = 1.6 Hz, 1H), 7.00 - 6.93 (m, 1H), 6.60 (s, 1H), 3.85 (s, 3H), 3.04 (t, J = 7.2 Hz, 2H), 2.98 (d, J = 11.6 Hz, 2H), 2.66 - 2.59 (m, 2H), 2.19 (s, 6H), 2.14 - 2.04 (m, 1H), 1.96 (t, J = 10.4 Hz, 2H), 1.74 (d, J = 11.6 Hz, 2H), 1.47 (s, 9H), 1.42 - 1.31 (m, 2H). LCMS: m/z (M+H)+ = 510.3.
Purity 96% Yield 22%

### Example 99:

### tert-butyl (4-(4-amino-7-(2-(4-(methylamino)piperidin-1-yl)ethyl)pyrrolo[2,1-f][1,2,4]triazin-5-yl)-2-methoxyphenyl)carbamate

1H NMR (400 MHz, DMSO-d6) δ = 8.30 (s, 1H), 8.00 (s, 1H), 7.89 (s, 1H), 7.79 (d, J = 8.4 Hz, 1H), 7.04 (d, J = 1.6 Hz, 1H), 6.96 (dd, J = 1.6, 8.0 Hz, 1H), 6.60 (s, 1H), 3.85 (s, 3H), 3.06 - 2.97 (m, 4H), 2.83 - 2.72 (m, 1H), 2.66 (t, J = 7.6 Hz, 2H), 2.47 (s, 3H), 2.00 (t, J = 11.6 Hz, 2H), 1.91 (d, J = 10.4 Hz, 2H), 1.47 (s, 9H), 1.46 - 1.38 (m, 2H). LCMS: m/z (M+H)+ = 496.3.
Purity 97% Yield 18%

### Example 100:

### tert-butyl (4-(4-amino-7-(2-(4-aminopiperidin-1-yl)ethyl)pyrrolo[2,1-f][1,2,4]triazin-5-yl)-2-methoxyphenyl)carbamate

1H NMR (400 MHz, DMSO-d6) δ = 8.26 (s, 1H), 8.00 (s, 1H), 7.89 (s, 1H), 7.79 (d, J = 8.4 Hz, 1H), 7.04 (d, J = 1.6 Hz, 1H), 6.96 (dd, J = 1.6, 8.0 Hz, 1H), 6.60 (s, 1H), 3.85 (s, 3H), 3.04 (t, J = 7.6 Hz, 2H), 2.96 (d, J = 11.6 Hz, 2H), 2.70 - 2.62 (m, 2H), 2.55 - 2.51 (m, 1H), 2.03 (t, J = 11.6 Hz, 2H), 1.90 - 1.77 (m, 2H), 1.53 - 1.41 (m, 11H). LCMS: m/z (M+H)+ = 482.2.
Purity 98% Yield 5%

### Example 101:

### 2-(1-(4-amino-5-(4-((tert-butoxycarbonyl)amino)-3-methoxyphenyl)pyrrolo[2,1-f][1,2,4]triazin-7-yl)piperidin-4-yl)acetic acid

1H NMR (400 MHz, DMSO-d6) δ = 12.05 (br s, 1H), 7.97 (s, 1H), 7.84 - 7.75 (m, 2H), 7.05 (d, J = 1.6 Hz,1H), 6.97 (dd, J = 1.6, 8.0 Hz, 1H), 6.23 (s, 1H), 3.85 (s, 3H), 3.67 (d, J = 10.8 Hz, 2H), 2.66 (t, J = 11.2 Hz, 2H), 2.23 (d, J = 6.6 Hz, 2H), 1.87 - 1.75 (m, 3H), 1.47 (s, 9H), 1.43 - 1.33 (m, 2H). LCMS: m/z (M+H)+ = 497.2.
Purity 99% Yield 9%

### Example 102:

### tert-butyl (4-(4-amino-7-(4-(hydroxymethyl)piperidin-1-yl)pyrrolo[2,1-f][1,2,4]triazin-5-yl)-2-methoxyphenyl)carbamate

LCMS: m/z (M+H)+=469.1. 1H NMR (400 MHz, DMSO-d6) δ = 7.97 (s, 1H), 7.86 - 7.72 (m, 2H), 7.05 (d, J = 1.6 Hz, 1H), 6.97 (dd, J = 1.7, 8.2 Hz, 1H), 6.22 (s, 1H), 4.49 (br s, 1H), 3.85 (s, 3H), 3.70 (d, J = 11.3 Hz, 2H), 3.43 - 3.37 (m, 2H), 2.64 (t, J = 10.9 Hz, 2H), 1.76 (d, J = 11.1 Hz, 2H), 1.58 - 1.50 (m, 1H), 1.47 (s, 9H), 1.41 - 1.28 (m, 2H).
Purity : 99% Yield 11%

### Example 103:

### tert-butyl (4-(7-(4-acetamidopiperidin-1-yl)-4-aminopyrrolo[2,1-f][1,2,4]triazin-5-yl)-2-methoxyphenyl)carbamate

1H NMR (400 MHz, CDCL3) δ = 8.18 (m, 1H), 7.88 (s, 1H), 7.12 (s, 1H), 7.04 - 7.02 (d, 1H), 6.94 (s,1H), 6.15 (s, 1H), 5.40 (d, 1H), 5.53 (s, 2H), 3.96 (m, 1H), 3.91 (s, 3H), 3.74 (d, 2H), 2.90 (m, 2H), 2.13 (m, 3H), 2.01 (s, 3H) , 1.74 (m, 3H), 1.54 (s, 9H). LCMS: m/z (M+H)+=496.3.
Purity : 98% Yield : 33%

### Example 104:

### tert-butyl (4-(4-amino-7-(4-(aminomethyl)-3,3-dimethylpiperidin-1-yl)pyrrolo[2,1-f][1,2,4]triazin-5-yl)-2-methoxyphenyl)carbamate

LCMS: m/z (M+H)+=496.3.

1H NMR (400 MHz, DMSO-d6) δ = 8.35 (s, 1H), 7.99 (s, 1H), 7.84 - 7.76 (m, 2H), 7.04 (d, J = 1.6 Hz, 1H), 6.96 (dd, J = 1.7, 8.1 Hz, 1H), 6.22 (s, 1H), 3.85 (s, 3H), 3.76 - 3.73 (m, 2H), 3.00 (d, J = 11.7 Hz, 2H), 2.48 - 2.40 (m, 2H), 1.95 - 1.84 (m, 1H), 1.60 - 1.50 (m, 1H), 1.47 (s, 9H), 1.44 - 1.36 (m, 1H), 0.99 (s, 3H), 0.97 (s, 3H).
Purity 99% Yield 30%

### Example 105:

### tert-butyl (4-(4-amino-7-(4-methyl-4-(methylamino)piperidin-1-yl)pyrrolo[2,1-f][1,2,4]triazin-5-yl)-2-methoxyphenyl)carbamate

LCMS: m/z (M+H)+=482.2.

1H NMR (400 MHz, DMSO-d6) δ = 8.38 (s, 1H), 7.97 (s, 1H), 7.80 (s, 1H), 7.79 (d, J = 8.3 Hz, 1H), 7.05 (d, J = 1.5 Hz, 1H), 6.97 (dd, J = 1.5, 8.3 Hz, 1H), 6.25 (s, 1H), 3.85 (s, 3H), 3.38 - 3.30 (m, 2H), 3.13 - 3.05 (m, 2H), 2.30 (s, 3H), 1.79 - 1.71 (m, 2H), 1.70 - 1.62 (m, 2H), 1.47 (s, 9H), 1.16 (s, 3H).
Purity 99% Yield 6%

### Example 106:

### tert-butyl (4-(4-amino-7-(4-(2-aminopropan-2-yl)piperidin-1-yl)pyrrolo[2,1-f][1,2,4]triazin-5-yl)-2-methoxyphenyl)carbamate

LCMS: m/z (M+H)+=496.4.

1H NMR (400 MHz, METHANOL-d4) δ = 8.54 (br s, 1H), 7.93 (d, J = 8.3 Hz, 1H), 7.77 (s, 1H), 7.05 (d, J = 1.5 Hz, 1H), 7.02 (dd, J = 1.7, 8.2 Hz, 1H), 6.29 (s, 1H), 3.92 (s, 3H), 3.83 (d, J = 10.9 Hz, 2H), 2.78 - 2.68 (m, 2H), 1.86 - 1.80 (m, 2H), 1.76 - 1.67 (m, 3H), 1.54 (s, 9H), 1.37 (s, 6H).
Purity : 98% Yield 3%

### Example 107:

### tert-butyl (4-(4-amino-7-(4-(2-aminoethyl)piperidin-1-yl)pyrrolo[2,1-f][1,2,4]triazin-5-yl)-2-methoxyphenyl)carbamate

LCMS: m/z (M+H)+=482.3.

1H NMR (400 MHz, DMSO-d6) δ = 8.46 - 8.38 (m, 1H), 7.99 (s, 1H), 7.83 - 7.77 (m, 2H), 7.06 (d, J = 1.8 Hz, 1H), 6.98 (dd, J = 1.8, 8.3 Hz, 1H), 6.23 (s, 1H), 3.86 (s, 3H), 3.69 (d, J = 11.4 Hz, 2H), 2.84 - 2.77 (m, 2H), 2.68 - 2.61 (m, 2H), 1.76 (d, J = 11.1 Hz, 2H), 1.53 - 1.50 (m, 2H), 1.48 (s, 9H), 1.40 - 1.34 (m, 3H).
Purity : 100% Yield 18%

### Example 108:

### tert-butyl (4-(4-amino-7-(4-(2-(methylamino)ethyl)piperidin-1-yl)pyrrolo[2,1-f][1,2,4]triazin-5-yl)-2-methoxyphenyl)carbamate

LCMS: m/z (M+H)+=496.3.

1H NMR (400 MHz, DMSO-d6) δ = 8.35 (s, 1H), 7.98 (s, 1H), 7.82 - 7.76 (m, 2H), 7.05 (d, J = 1.8 Hz, 1H), 6.97 (dd, J = 1.6, 8.1 Hz, 1H), 6.22 (s, 1H), 3.85 (s, 3H), 3.68 (d, J = 11.6 Hz, 2H), 2.80 - 2.74 (m, 2H), 2.68 - 2.63 (m, 2H), 2.44 (s, 3H), 1.75 (d, J = 11.8 Hz, 2H), 1.56 - 1.47 (m, 11H), 1.41 - 1.32 (m, 3H).
Purity : 96% Yield 14%

### Example 109:

### tert-butyl (4-(4-amino-7-(4-(2-(dimethylamino)ethyl)piperidin-1-yl)pyrrolo[2,1-f][1,2,4]triazin-5-yl)-2-methoxyphenyl)carbamate

1H NMR (400 MHz, DMSO-d6) δ = 7.99 (s, 1H), 7.85 - 7.74 (m, 2H), 7.05 (d, J = 1.8 Hz, 1H), 6.97 (dd, J = 1.8, 8.1 Hz, 1H), 6.23 (s, 1H), 3.85 (s, 3H), 3.69 (d, J = 11.8 Hz, 2H), 3.12 - 2.99 (m, 2H), 2.73 (s, 6H), 2.69 - 2.61 (m, 2H), 1.77 (d, J = 10.9 Hz, 2H), 1.65 - 1.56 (m, 2H), 1.47 (s, 9H), 1.45 - 1.34 (m, 3H). LCMS: m/z (M+H)+=510.3.
Purity : 98% Yield : 18%

### Example 110:

### tert-butyl (4-(4-amino-7-((((1S,3R)-3-aminocyclopentyl)methyl)amino)pyrrolo[2,1-f][1,2,4]triazin-5-yl)-2-methoxyphenyl)carbamate

1H NMR (400 MHz, DMSO-d6) δ = 8.40 (br s, 1H), 7.97 (s, 1H), 7.78 (d, J = 8.0 Hz, 1H), 7.72 (s, 1H), 7.04 (d, J = 1.6 Hz, 1H), 6.97 (dd, J = 1.6, 8.0 Hz, 1H), 5.97 (s, 1H), 5.61 (br s, 1H), 3.85 (s, 3H), 3.21 - 3.15 (m, 2H), 2.33 - 2.22 (m, 1H), 2.21 - 2.05 (m, 1H), 1.91 - 1.83 (m, 1H), 1.76 - 1.67 (m, 1H), 1.66 - 1.48 (m, 3H), 1.47 (s, 9H), 1.27 - 1.16 (m, 1H). LCMS: m/z (M+H)+ =468.2.
Purity 98% Yield 18%

### Example 111:

### tert-butyl (4-(4-amino-7-(((1R,3S)-3-(aminomethyl)cyclopentyl)amino)pyrrolo[2,1-f][1,2,4]triazin-5-yl)-2-methoxyphenyl)carbamate

1H NMR (400 MHz, DMSO-d6) δ = 8.41 (br s, 1H), 7.98 (s, 1H), 7.78 (d, J = 8.0 Hz, 1H), 7.73 (s, 1H), 7.04 (d, J = 1.6 Hz, 1H), 6.98 (dd, J = 1.6, 8.0 Hz, 1H), 5.98 (s, 1H), 5.43 (br s, 1H), 3.85 (s, 3H), 3.21 - 3.15 (m, 1H), 2.77 - 2.75 (m, 2H), 2.31 - 2.21 (m, 1H), 2.13 - 2.07 (m, 1H), 2.05 - 1.90 (m, 2H), 1.79 - 1.63 (m, 2H), 1.47 (s, 9H), 1.33 - 1.27 (m, 1H). LCMS: m/z (M+H)+ = 468.2.
Purity 98% Yield 3%

### Example 112:

### tert-butyl (4-(4-amino-7-(((1S,3S)-3-(dimethylamino)cyclopentyl)amino)pyrrolo[2,1-f][1,2,4]triazin-5-yl)-2-methoxyphenyl)carbamate

LCMS: m/z (M+H)+=482.3.

1H NMR (400 MHz, DMSO-d6) δ = 7.98 (s, 1H), 7.78 (d, J = 8.1 Hz, 1H), 7.72 (s, 1H), 7.05 (d, J = 1.5 Hz, 1H), 6.98 (dd, J = 1.6, 8.2 Hz, 1H), 5.96 (s, 1H), 5.32 (d, J = 7.1 Hz, 1H), 3.92 - 3.86 (m, 1H), 3.85 (s, 3H), 2.69 - 2.61 (m, 1H), 2.18 - 2.08 (m, 7H), 1.93 - 1.84 (m, 1H), 1.83 - 1.77 (m, 2H), 1.65 - 1.53 (m, 1H), 1.47 (s, 9H), 1.43 - 1.34 (m, 1H).
Purity 99% Yield 20%

### Example 113:

### tert-butyl (4-(4-amino-7-(((1S,3R)-3-aminocyclopentyl)amino)pyrrolo[2,1-f][1,2,4]triazin-5-yl)-2-methoxyphenyl)carbamate

1H NMR (400 MHz, DMSO-d6) δ = 8.45 (br s, 1H), 7.97 (s, 1H), 7.78 (d, J = 8.0 Hz, 1H), 7.73 (s, 1H), 7.04 (d, J = 2.0 Hz, 1H), 6.98 (dd, J = 1.6, 8.0 Hz, 1H), 5.98 (s, 1H), 5.68 (br s, 1H), 3.93 - 3.85 (m, 4H), 3.48 - 3.44 (m, 1H), 2.01 - 1.92 (m, 1H), 1.89 - 1.74 (m, 2H), 1.67 - 1.58 (m, 1H), 1.54 - 1.50 (m, 1H), 1.47 (s, 9H), 1.46 - 1.41 (m, 1H). LCMS: m/z (M+H)+ =454.2.
Purity 90% Yield 0.39%

### Example 114:

### tert-butyl (4-(4-amino-7-(((1R,3S)-3-aminocyclopentyl)amino)pyrrolo[2,1-f][1,2,4]triazin-5-yl)-2-methoxyphenyl)carbamate

1H NMR (400 MHz, DMSO-d6) δ = 8.05 (s, 1H), 8.00 (s, 1H), 7.97 - 7.84 (m, 4H), 7.64 (br s, 1H), 7.10 (d, J = 1.6 Hz, 1H), 7.04 (dd, J = 1.7, 8.2 Hz, 1H), 6.61 (br s, 1H), 6.29 (s, 1H), 4.03 - 3.95 (m, 1H), 3.86 (s, 3H), 3.57 - 3.48 (m, 1H), 2.08 - 1.90 (m, 2H), 1.85 - 1.69 (m, 2H), 1.63 (td, J = 7.0, 13.7 Hz, 1H), 1.51 - 1.46 (m, 10H) LCMS: m/z (M+H)+ = 454.2
Purity : 99% Yield 60%.

### Example 115:

### tert-butyl (4-(4-amino-7-(((1R,3R)-3-aminocyclopentyl)amino)pyrrolo[2,1-f][1,2,4]triazin-5-yl)-2-methoxyphenyl)carbamate

LCMS: m/z (M+H)+=454.2. 1H NMR (400 MHz, DMSO-d6) δ = 8.14 (s, 1H), 8.01 (s, 1H), 7.97 - 7.72 (m, 5H), 7.06 (d, J = 1.8 Hz, 1H), 7.00 (dd, J = 1.8, 8.3 Hz, 1H), 6.07 (s, 1H), 5.99 (br s, 1H), 4.11 - 4.01 (m, 1H), 3.86 (s, 3H), 3.74 - 3.64 (m, 1H), 2.23 - 2.09 (m, 2H), 2.07 - 1.92 (m, 2H), 1.73 - 1.63 (m, 1H), 1.62 - 1.54 (m, 1H), 1.47 (s, 9H).Purity : 99% Yield : 6%

### Example 116:

### tert-butyl (4-(4-amino-7-(((1S,3S)-3-aminocyclopentyl)amino)pyrrolo[2,1-f][1,2,4]triazin-5-yl)-2-methoxyphenyl)carbamate

LCMS: m/z (M+H)+=454.2. 1H NMR (400 MHz, DMSO-d6) δ = 8.05 (s, 1H), 7.95 (s, 1H), 7.88 (d, J = 8.4 Hz, 1H), 7.82 (br s, 3H), 7.48 (br s, 1H), 7.09 (d, J = 1.8 Hz, 1H), 7.03 (dd, J = 1.8, 8.1 Hz, 1H), 6.54 (br s, 1H), 6.21 (s, 1H), 4.14 - 4.04 (m, 1H), 3.86 (s, 3H), 3.74 - 3.64 (m, 1H), 2.21 - 2.09 (m, 2H), 2.05 - 1.93 (m, 2H), 1.73 - 1.63 (m, 1H), 1.61 - 1.52 (m, 1H), 1.48 (s, 9H).
Purity 96% Yield 10%

### Example 117:

### tert-butyl (4-(4-amino-7-((piperidin-4-ylmethyl)amino)pyrrolo[2,1-f][1,2,4]triazin-5-yl)-2-methoxyphenyl)carbamate

1H NMR (400 MHz, DMSO-d6) δ = 8.44 (s, 1H), 7.96 (s, 1H), 7.78 (d, J = 8.5 Hz, 1H), 7.72 (s, 1H), 7.04 (s, 1H), 6.98 (dd, J = 1.5, 8.1 Hz, 1H), 5.96 (s, 1H), 5.58 (t, J = 6.4 Hz, 1H), 3.85 (s, 3H), 3.12 - 3.01 (m, 4H), 2.61 - 2.53 (m, 2H), 1.86 - 1.67 (m, 3H), 1.47 (s, 9H), 1.26 - 1.09 (m, 2H) LCMS: m/z (M+H)+ = 468.2
Purity 99% Yield 11%

### Example 118:

### tert-butyl(4-(4-amino-7-(((1s,3s)-3-aminocyclobutyl)amino)pyrrolo[2,1-f][1,2,4]triazin-5-yl)-2-methoxyphenyl)carbamate

LCMS: m/z (M+H)+=440.2. 1H NMR (400 MHz, DMSO-d6) δ = 8.12 - 7.95 (m, 5H), 7.87 (d, J = 8.2 Hz, 1H), 7.51 (br s, 1H), 7.08 (s, 1H), 7.03 (d, J = 8.3 Hz, 1H), 6.74 (br s, 1H), 6.18 (s, 1H), 3.89 - 3.80 (m, 4H), 3.45 - 3.41 (m, 1H), 2.80 - 2.70 (m, 2H), 2.17 (q, J = 8.9 Hz, 2H), 1.48 (s, 9H).
Purity : 99% Yield 97%

### Example 119:

### tert-butyl (4-(4-amino-7-(((1r,3r)-3-aminocyclobutyl)amino)pyrrolo[2,1-f][1,2,4]triazin-5-yl)-2-methoxyphenyl)carbamate

1H NMR (400 MHz, DMSO-d6) δ = 8.14 (s, 1H), 7.98 (s, 1H), 7.79 (d, J = 8.3 Hz, 1H), 7.76 (s, 1H), 7.03 (s, 1H), 6.97 (d, J = 8.4 Hz, 1H), 6.07 (d, J = 7.1 Hz, 1H), 5.82 (s, 1H), 4.22 - 4.13 (m, 1H), 3.87 - 3.79 (m, 4H), 2.46 - 2.38 (m, 4H), 1.47 (s, 9H) LCMS: m/z (M+H)+ = 440.2
Purity 94% Yield 5%

### Example 120:

### tert-butyl (4-(4-amino-7-(piperidin-4-ylamino)pyrrolo[2,1-f][1,2,4]triazin-5-yl)-2-methoxyphenyl)carbamate

1H NMR (400 MHz, DMSO-d6) δ = 8.65 - 8.55 (m, 1H), 8.42 - 8.29 (m, 1H), 8.04 (s, 1H), 7.90 (s, 1H), 7.85 (d, J = 8.4 Hz, 1H), 7.07 (d, J = 1.2 Hz, 1H), 7.02 (dd, J = 1.6, 8.4 Hz, 1H), 6.34 (br s, 1H), 6.24 (s, 1H), 3.85 (s, 3H), 3.70 - 3.60 (m, 1H), 3.40 - 3.30 (m, 2H), 3.06 - 2.90 (m, 2H), 2.11 - 2.03 (m, 2H), 1.83 - 1.68 (m, 2H), 1.47 (s, 9H). LCMS: m/z (M+H)+ = 454.2.
Purity 100% Yield 21%

### Example 121:

### tert-butyl (4-(4-amino-7-(((1s,4s)-4-aminocyclohexyl)amino)pyrrolo[2,1-f][1,2,4]triazin-5-yl)-2-methoxyphenyl)carbamate

1H NMR (400 MHz, DMSO-d6) δ = 8.14 (s, 1H), 8.01 (s, 1H), 7.88 - 7.80 (m, 5H), 7.07 (d, J = 1.6 Hz, 1H), 7.02 (dd, J = 1.6, 8.0 Hz, 1H), 6.21 (s, 1H), 5.80 (br s, 1H), 3.86 (s, 3H), 3.49 - 3.41 (m, 1H), 3.02 - 2.94 (m, 1H), 2.16 - 1.84 (m, 4H), 1.47 (s, 9H), 1.46 - 1.33 (m, 4H). LCMS: m/z (M+H)+ = 468.3.
Purity 98% Yield 26%

### Example 122:

### tert-butyl (4-(4-amino-7-(((1s,4s)-4-aminocyclohexyl)amino)pyrrolo[2,1-f][1,2,4]triazin-5-yl)-2-methoxyphenyl)carbamate

1H NMR (400 MHz, DMSO-d6) δ = 8.13 (s, 1H), 8.01 (s, 1H), 7.88 (s, 1H), 7.83 (d, J = 8.0 Hz, 1H), 7.77 (br s, 3H), 7.08 (d, J = 1.6 Hz, 1H), 7.01 (dd, J = 1.6, 8.4 Hz, 1H), 6.18 (s, 1H), 5.45 (br s, 1H), 3.86 (s, 3H), 3.68 - 3.62 (m, 1H), 3.20 - 3.14 (m, 1H), 1.95 - 1.85 (m, 2H), 1.78 - 1.66 (m, 6H), 1.47 (s, 9H). LCMS: m/z (M+H)+ = 468.2.
Purity : 97% Yield 31%

### Example 123:

### tert-butyl (4-(4-amino-7-(4-(aminomethyl)-4-methylpiperidin-1-yl)pyrrolo[2,1-f][1,2,4]triazin-5-yl)-2-methoxyphenyl)carbamate

1H NMR (400 MHz, DMSO-d6) δ = 8.42 (s, 1H), 8.04 (s, 1H), 7.89 (s, 1H), 7.84 (d, J = 8.0 Hz, 1H), 7.03 (d, J = 1.6 Hz, 1H), 6.94 (dd, J = 1.6, 8.4 Hz, 1H), 3.82 (s, 3H), 3.30 - 3.18 (m, 4H), 3.00 - 2.94 (m, 1H), 1.89 (d, J = 9.2 Hz, 2H), 1.63 - 1.52 (m, 2H), 1.48 (s, 9H) LCMS: m/z (M+H)+ = 488.1.
Purity 99% Yield 4%

### Example 124:

### tert-butyl (4-(4-amino-6-chloro-7-(4-(dimethylamino)piperidin-1-yl)pyrrolo[2,1-f][1,2,4]triazin-5-yl)-2-methoxyphenyl)carbamate

1H NMR (400 MHz, DMSO-d6) 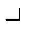 = 8.04 (s, 1H), 7.89 (s, 1H), 7.84 (d, J = 8.0 Hz, 1H), 7.03 (d, J = 1.6 Hz, 1H), 6.94 (dd, J = 2.0, 8.0 Hz, 1H), 3.82 (s, 3H), 3.36 - 3.24 (m, 4H), 2.34 - 2.24 (m, 1H), 2.22 (s, 6H), 1.81 (d, J = 11.2 Hz, 2H), 1.62 - 1.52 (m, 2H), 1.48 (s, 9H). LCMS: m/z (M+H)+ = 516.3.
Purity : 99% Yield : 21%

### Example 125:

### tert-butyl (4-(4-amino-7-(4-aminopiperidin-1-yl)-6-(trifluoromethyl)pyrrolo[2,1-f][1,2,4]triazin-5-yl)-2-methoxyphenyl)carbamate

1H NMR (400 MHz, DMSO-d6) δ = 8.38 (s, 1H), 8.07 (s, 1H), 7.95 (s, 1H), 7.82 (d, J = 8.0 Hz, 1H), 7.02 (d, J = 1.6 Hz, 1H), 6.93 (dd, J = 1.4, 8.2 Hz, 1H), 3.79 (s, 3H), 3.44 - 3.38 (m, 2H), 3.16 - 3.12 (m, 2H), 3.07 - 3.01 (m, 1H), 1.94 - 1.88 (m, 2H), 1.64 - 1.52 (m, 2H), 1.47 (s, 9H). LCMS: m/z (M+H)+ = 522.3
Purity 98% Yield 12%

### Example 126:

### tert-butyl (4-(4-amino-7-(4-aminopiperidin-1-yl)-6-iodopyrrolo[2,1-f][1,2,4]triazin-5-yl)-2-methoxyphenyl)carbamate

1H NMR (400 MHz, DMSO-d6) δ = 8.43 (s, 1H), 8.03 (s, 1H), 7.86 - 7.80 (m, 2H), 6.99 (d, J = 1.6 Hz, 1H), 6.90 (dd, J = 1.6, 8.4 Hz, 1H), 3.83 (s, 3H), 3.46 (t, J = 11.2 Hz, 2H), 3.11 (d, J = 11.6 Hz, 2H), 3.06 - 2.95 (m, 1H), 1.93 (d, J = 11.2 Hz, 2H), 1.68 - 1.53 (m, 2H), 1.48 (s, 9H). LCMS: m/z (M+H)+ = 580.1
Purity 99% Yield 11%

### Example 127:

### tert-butyl (4-(4-amino-7-(4-aminopiperidin-1-yl)-6-fluoropyrrolo[2,1-f][1,2,4]triazin-5-yl)-2-methoxyphenyl)carbamate

1H NMR (400 MHz, DMSO-d6) δ = 8.42 (s, 1H), 8.04 (s, 1H), 7.94 (s, 1H), 7.84 (d, J = 7.9 Hz, 1H), 7.05 (s, 1H), 6.97 (d, J = 7.9 Hz, 1H), 3.83 (s, 3H), 3.44 - 3.38 (m, 2H), 3.12 (t, J = 11.4 Hz, 2H), 3.00 - 2.94 (m, 1H), 1.95 - 1.83 (m, 2H), 1.62 - 1.49 (m, 2H), 1.47 (s, 9H). LCMS: m/z (M+H)+=472.1.
Purity 98% Yield 20%

### Example 128:

### tert-butyl (4-(4-amino-7-(4-(dimethylamino)piperidin-1-yl)-6-fluoropyrrolo[2,1-f][1,2,4]triazin-5-yl)-2-methoxyphenyl)carbamate

1H NMR (400 MHz, DMSO-d6) δ = 8.19 (s, 1H), 8.03 (s, 1H), 7.93 (s, 1H), 7.84 (d, J = 8.4 Hz, 1H), 7.05 (d, J = 1.2 Hz, 1H), 6.97 (dd, J = 1.6, 8.4 Hz, 1H), 3.83 (s, 3H), 3.46 (d, J = 11.6 Hz, 2H), 3.09 (t, J = 11.6 Hz, 2H), 2.41 - 2.38 (m, 1H), 2.28 (s, 6H), 1.85 (d, J = 11.2 Hz, 2H), 1.63 - 1.53 (m, 2H), 1.47 (s, 9H). LCMS: m/z (M+H)+ = 500.2.
Yield 53% Purity : 98%

### Example 129:

### tert-butyl (4-(4-amino-6-fluoro-7-(4-(methylamino)piperidin-1-yl)pyrrolo[2,1-f][1,2,4]triazin-5-yl)-2-methoxyphenyl)carbamate

1H NMR (400 MHz, DMSO-d6) δ = 8.32 (s, 1H), 8.04 (s, 1H), 7.94 (s, 1H), 7.84 (d, J = 8.4 Hz, 1H), 7.05 (d, J = 1.6 Hz, 1H), 6.97 (dd, J = 1.2, 8.0 Hz, 1H), 3.83 (s, 3H), 3.45 (d, J = 11.6 Hz, 2H), 3.12 (t, J = 11.2 Hz, 2H), 2.95 - 2.85 (m, 1H), 2.48 (s, 3H), 2.05 - 1.99 (m, 2H), 1.67 - 1.53 (m, 2H), 1.47 (s, 9H). LCMS: m/z (M+H)+ = 486.2
Purity 99% Yield 61%

### Example 130:

### tert-butyl (4-(4-amino-6-fluoro-7-(2-(4-(methylamino)piperidin-1-yl)ethyl)pyrrolo[2,1-f][1,2,4]triazin-5-yl)-2-methoxyphenyl)carbamate

1H NMR (400 MHz, DMSO-d6) δ = 8.32 (s, 1H), 8.04 (s, 1H), 7.99 (s, 1H), 7.85 (d, J = 8.0 Hz, 1H), 7.05 (d, J = 1.6 Hz, 1H), 7.01 - 6.95 (m, 1H), 3.83 (s, 3H), 3.06 (t, J = 7.4 Hz, 2H), 2.94 (d, J = 12.0 Hz, 2H), 2.62 (t, J = 7.6 Hz, 2H), 2.53 - 2.51 (m, 1H), 2.40 (s, 3H), 1.99 (t, J = 10.6 Hz, 2H), 1.92 - 1.80 (m, 2H), 1.48 (s, 9H), 1.40 - 1.28 (m, 2H). LCMS: m/z (M+H)+ = 514.4
Purity : 98% Yield 15%

### Example 131:

### tert-butyl (4-(4-amino-6-fluoro-7-(4-(2-(methylamino)ethyl)piperidin-1-yl)pyrrolo[2,1-f][1,2,4]triazin-5-yl)-2-methoxyphenyl)carbamate

1H NMR (400 MHz, DMSO-d6) δ = 8.38 (s, 1H), 8.03 (s, 1H), 7.93 (s, 1H), 7.84 (d, J = 8.2 Hz, 1H), 7.05 (d, J = 1.3 Hz, 1H), 6.97 (d, J = 7.9 Hz, 1H), 3.83 (s, 3H), 3.39 (d, J = 12.0 Hz, 2H), 3.07 (t, J = 11.3 Hz, 2H), 2.74 (t, J = 7.1 Hz, 2H), 2.42 (s, 3H), 1.73 (d, J = 12.0 Hz, 2H), 1.54 - 1.47 (m, 12H), 1.36 - 1.28 (m, 2H).
Purity : 99% Yield : 19%

### Example 132:

### tert-butyl (4-(1-amino-6-(4-aminopiperidin-1-yl)pyrrolo[1,2-a]pyrazin-8-yl)-2-methoxyphenyl)carbamate

LCMS: m/z (M+H)+=453.3. 1H NMR (400 MHz, DMSO-d6) δ = 8.33 (br s, 1H), 7.97 (s, 1H), 7.76 (d, J = 8.1 Hz, 1H), 7.26 (d, J = 4.6 Hz, 1H), 7.02 (s, 1H), 7.00 (d, J = 4.8 Hz, 1H), 6.94 (d, J = 8.1 Hz, 1H), 6.37 (s, 1H), 5.61 (br s, 2H), 3.84 (s, 3H), 3.22 - 3.18 (m, 2H), 3.11 - 3.08 (m, 1H), 2.77 (t, J = 11.3 Hz, 3H), 2.02 - 1.91 (m, 2H), 1.78 - 1.64 (m, 2H), 1.47 (s, 9H).
Purity : 99% Yield 8%

### Example 133:

### tert-butyl (4-(1-amino-6-(4-(methylamino)piperidin-1-yl)pyrrolo[1,2-a]pyrazin-8-yl)-2-methoxyphenyl)carbamate

LCMS: m/z (M+H)+=467.4. 1H NMR (400 MHz, DMSO-d6) δ = 8.32 (br s, 1H), 7.97 (s, 1H), 7.76 (d, J = 7.8 Hz, 1H), 7.26 (d, J = 4.9 Hz, 1H), 7.03 (d, J = 1.6 Hz, 1H), 6.99 (d, J = 4.9 Hz, 1H), 6.95 (dd, J = 1.5, 8.3 Hz, 1H), 6.37 (s, 1H), 5.60 (br s, 2H), 3.84 (s, 3H), 3.25 - 3.15 (m, 3H), 2.79 - 2.70 (m, 2H), 2.43 (s, 3H), 2.06 - 1.97 (m, 2H), 1.67 - 1.55 (m, 2H), 1.47 (s, 9H).
Purity : 99% Yield 14%

### Example 134:

### tert-butyl (4-(1-amino-6-(4-(dimethylamino)piperidin-1-yl)pyrrolo[1,2-a]pyrazin-8-yl)-2-methoxyphenyl)carbamate

LCMS: m/z (M+H)+=481.3. 1H NMR (400 MHz, DMSO-d6) δ = 7.97 (s, 1H), 7.75 (d, J = 8.1 Hz, 1H), 7.27 (d, J = 4.9 Hz, 1H), 7.03 (d, J = 1.5 Hz, 1H), 6.98 (d, J = 4.9 Hz, 1H), 6.95 (dd, J = 1.5, 8.1 Hz, 1H), 6.35 (s, 1H), 5.60 (br s, 2H), 3.84 (s, 3H), 3.22 (d, J = 11.6 Hz, 2H), 2.70 (t, J = 11.0 Hz, 2H), 2.37 - 2.29 (m, 1H), 2.25 (s, 6H), 1.91 - 1.83 (m, 2H), 1.65 (dq, J = 3.4, 11.7 Hz, 2H), 1.47 (s, 9H).
Purity : 99% Yield : 12%

### Example 135:

### 7-(4-aminopiperidin-1-yl)-5-(4-phenoxyphenyl)pyrrolo[2,1-f][1,2,4]triazin-4-amine

1H NMR (400 MHz, DMSO-d6) δ = 8.41 (s, 1H), 7.82 (s, 1H), 7.48 - 7.38 (m, 4H), 7.20 - 7.14 (m, 1H), 7.13 - 7.06 (m, 4H), 6.22 (s, 1H), 3.69 (br d, J = 11.6 Hz, 2H), 3.07 - 2.98 (m, 1H), 2.81 - 2.68 (m, 2H), 1.93 (d, J = 10.4 Hz, 2H), 1.70 - 1.55 (m, 2H). LCMS: m/z (M+H)+ = 401.1
Purity : 99% Yield 33%

### Example 136:

### 7-(4-(dimethylamino)piperidin-1-yl)-5-(4-phenoxyphenyl)pyrrolo[2,1-f][1,2,4]triazin-4-amine

1H NMR (400 MHz, DMSO-d6) δ = 7.82 (s, 1H), 7.49 - 7.39 (m, 4H), 7.20 - 7.15 (m, 1H), 7.12 - 7.06 (m, 4H), 6.20 (s, 1H), 3.73 (d, J = 11.8 Hz, 2H), 2.71 - 2.64 (m, 2H), 2.29 - 2.24 (m, 1H), 2.22 (s, 6H), 1.91 - 1.78 (m, 2H), 1.66 - 1.50 (m, 2H). LCMS: m/z (M+H)+ = 429.2.
Purity 99% Yield 3%

### Example 137:

### N-(4-(4-amino-7-(4-aminopiperidin-1-yl)pyrrolo[2,1-f][1,2,4]triazin-5-yl)-2-methoxyphenyl)furan-2-carboxamide

1H NMR (400 MHz, DMSO-d6) δ = 9.14 (s, 1H), 8.38 (s, 1H), 8.08 (d, J = 8.0 Hz, 1H), 7.93 (s, 1H), 7.83 (s, 1H), 7.33 (d, J = 3.6 Hz, 1H), 7.15 (d, J = 1.6 Hz, 1H), 7.05 (dd, J = 1.6, 8.4 Hz, 1H), 6.73 (dd, J = 1.6, 3.2 Hz, 1H), 6.30 (s, 1H), 3.93 (s, 3H), 3.71 (d, J = 11.6 Hz, 2H), 3.15 - 3.05 (m, 1H), 2.76 (t, J = 11.4 Hz, 2H), 2.01 - 1.91 (m, 2H), 1.75 - 1.60 (m, 2H). LCMS: m/z (M+H)+ = 448.1.
Purity 99% Yield 7%

### Example 138:

### N-(4-(4-amino-7-(4-aminopiperidin-1-yl)pyrrolo[2,1-f][1,2,4]triazin-5-yl)-2-methoxyphenyl)furan-3-carboxamide

LCMS: m/z (M+H)+=448.2. 1H NMR (400 MHz, DMSO-d6) δ = 9.23 (s, 1H), 8.43 (s, 1H), 8.38 - 8.34 (m, 1H), 7.86 - 7.79 (m, 3H), 7.13 (d, J = 1.7 Hz, 1H), 7.04 (dd, J = 1.7, 8.1 Hz, 1H), 6.99 (d, J = 1.2 Hz, 1H), 6.31 (s, 1H), 3.89 (s, 3H), 3.71 (d, J = 11.7 Hz, 2H), 3.13 - 3.05 (m, 1H), 2.76 (t, J = 10.9 Hz, 2H), 1.98 - 1.92 (m, 2H), 1.72 - 1.62 (m, 2H).
Purity : 98% Yield 19%

### Experimental Example 1. Evaluation of inhibitory activity on YES1 Kinase of the compound according to the present invention

To evaluate the inhibitory activity against YES1 kinase of the compound according to the present invention, ADP Glo assay (Promega) was used, and the following method was performed with reference to the Promega kinase enzyme system protocol. To proceed with the experiment, buffers A-F and compound dilution plates were required.

To prepare A.4X reaction buffer, 800µl of 5X reaction buffer and 200µl of distilled water were used. To prepare B.4X kinase buffer, 996.6µl of A.4X reaction buffer, 2µl of 0.1M DTT, and 3.2µl of 2.5M MnCl₂ were added and mixed. To prepare C.1X kinase buffer (containing 5% DMSO) used for compound dilution, 250µl of B.4X kinase buffer, 700µl of distilled water, and 50µl of DMSO were added, so that the final DMSO concentration became 5%. Then, 10 mM compounds were diluted 100-fold to prepare 100µM solutions, and then D.5X inhibitor solution was prepared so that the compound concentration became 5uM. Using a 96-well plate, 5µM compounds were 3-fold diluted using C.1X kinase buffer (containing 5% DMSO) to prepare compound plates with concentrations ranging from 0.25µM to 5µM. The resulting plates were stored at room temperature. To prepare E. 2.5X ATP/substrate mixture, 50µl of B.4X kinase buffer, 50µl of 10µM ATP, and 100µl of substrate were mixed. To prepare F. YES1 kinase enzyme solution, 35µl of B.4X kinase buffer, 0.4µl of YES1 enzyme, and 104.6µl of distilled water were used. Once the diluted compound plates, ATP/substrate mixture, and YES1 kinase solution were all prepared, 3 µL each was transferred from the diluted compound plate to a luciferase 96-well white plate (Since the total volume of the ATP/substrate and YES1 kinase solution that reacts with the compound is 15 µL, the compounds were diluted 5-fold, making the final compound concentration 0.05 µM-1 µM.). Then, 6 µL of F. YES1 Kinase Enzyme Solution was transferred to each well, pipetted 10 times to mix, and incubated at room temperature in a shaker for 10 minutes. Next, 6 µL of E. 2.5X ATP/Substrate Mixture was transferred to each well, pipetted 10 times to mix, and centrifuged at 12,000 rpm for 5 minutes. The plate was then incubated in a shaker at room temperature for 40 minutes. After that, 15 µL of ADP-Glo Reagent, dissolved at room temperature, was added to each well, pipetted 10 times, and incubated in a shaker at room temperature for 40 minutes. Finally, 30 µL of Kinase Detection Reagent, dissolved at room temperature, was added, pipetted 10 times, incubated in a shaker at room temperature for 30 minutes. Luminescence was measured using a luminescence plate reader. Then, the background value was subtracted from all measured values, and the YES1 kinase activity at compound concentrations of 1 nM, 10 nM, and 100 nM was calculated. The measured values were calculated as percentages using the average value of the negative control. Graphs were drawn using GraphPad Prism, and the enzyme activity was calculated as a percentage of YES1 kinase inhibition at compound concentrations of 1 nM, 10 nM, and 100 nM. IC₅₀ values were obtained using Microsoft Excel.

The results of the calculated YES1 enzyme inhibition rate are shown in Table 1 below.

**[Table 1]**

| example | YES1 enzyme, % inhibition | | | YES1 enzyme, IC₅₀ |
|---|---|---|---|---|
| | 1 nM | 10 nM | 100 nM | |
| 1 | | 17 | 31 | |
| 2 | | 21 | 28 | |
| 3 | | 44 | 49 | |
| 4 | | 31 | 29 | |
| 5 | | 38 | 68 | |
| 6 | | 41 | 46 | |
| 7 | | 28 | 49 | |
| 8 | | 33 | 64 | |
| 9 | | 39 | 57 | |
| 10 | | 8 | 38 | |
| 11 | | 21 | 44 | |
| 12 | | 65 | 86 | |
| 13 | | 77 | 93 | |
| 14 | | 19 | 77 | |
| 15 | | 67 | 88 | |
| 16 | | 30 | 36 | |
| 17 | | 53 | 59 | |
| 18 | | 39 | 81 | |
| 19 | | 69 | 86 | |
| 20 | | 36 | 70 | |
| 21 | | 67 | 92 | |
| 22 | | 60 | 87 | |
| 23 | | 58 | 87 | |
| 24 | | 50 | 82 | |
| 25 | | 28 | 76 | |
| 26 | | 30 | 69 | |
| 27 | | 59 | 74 | |
| 28 | | 41 | 85 | |
| 29 | | 28 | 47 | |
| 30 | | 73 | 94 | |
| 32 | | 51 | 82 | |
| 34 | | 37 | 74 | |
| 36 | | 36 | 78 | |
| 37 | | 24 | 69 | |
| 38 | | 44 | 80 | |
| 39 | | 57 | 89 | |
| 40 | | 5 | 43 | |
| 42 | | 63 | 90 | |
| 44 | | 80 | 96 | A |
| 45 | | 80 | 92 | A |
| 46 | | 52 | 77 | |
| 48 | | 70 | 96 | |
| 51 | | 70 | 90 | |
| 52 | | 60 | 90 | A |
| 53 | | 29 | 60 | |
| 54 | | 20 | 60 | A |
| 55 | | 40 | 76 | |
| 56 | | 23 | 68 | A |
| 58 | | | | A |
| 59 | | 56 | 80 | A |
| 65 | | | | A |
| 74 | 43 | 66 | | A |
| 83 | 14 | 24 | | |
| 85 | | 59 | 78 | A |
| 87 | | 61 | 80 | A |
| 89 | 19 | 34 | | |
| 90 | 12 | 47 | | |
| 91 | | 21 | 59 | |
| 92 | | 66 | 81 | A |
| 93 | | 14 | 48 | |
| 94 | | 41 | 78 | A |
| 98 | | 65 | 90 | |
| 99 | | 71 | 90 | |
| 100 | | 70 | 88 | |
| 101 | 16 | 40 | | |
| 102 | 11 | 39 | | |
| 103 | | 27 | 66 | |
| 104 | 33 | 45 | | |
| 105 | | | | A |
| 106 | 37 | 43 | | |
| 107 | 38 | 52 | | A |
| 108 | 38 | 52 | | A |
| 109 | 37 | 51 | | A |
| 112 | 29 | 60 | | |
| 116 | 30 | 63 | | |
| 117 | | 39 | 64 | |
| 119 | 24 | 55 | | |
| 120 | | 40 | 78 | |
| 121 | | 50 | 77 | |
| 123 | | | | A |
| 127 | | 67 | 88 | A |
| 128 | | 72 | 92 | A |
| 129 | | 70 | 92 | A |
| 135 | | 62 | 87 | |
| 136 | | 64 | 90 | |
| 137 | | | | A |
| 138 | | | | A |

| | | | | |
|---|---|---|---|---|
| (A: IC₅₀ ≤ 100nM; B: 100nM < IC₅₀ ≤ 1 µM; C: 1µM < IC₅₀ ≤ 10 µM; D: 10 µM < IC₅₀) | | | | |

As confirmed from Table 1, the compounds of the present invention exhibited excellent inhibitory activity against YES1 kinase. In particular, it was confirmed that YES1 kinase could be inhibited generally with a considerably high inhibition rate, even when the compounds of the present invention were treated at a low concentration of 10 nM. In the case of compounds 74, 83, 89, 90, 101, 102, 104, 106 to 109, 112, 116 and 119 of the present invention, it was confirmed that YES 1 kinase could be inhibited even at a low concentration of 1 nM.

### Experimental Example 2. Evaluation of cell growth inhibition of the compound according to the present invention

The cell line according to the present invention was selected as KYSE-70, an esophageal cancer cell line in which YES1 is amplified. The evaluation of cell growth inhibition was performed as follows.

KYSE-70 was cultured in RPMI-1640 (Capricorn) supplemented with 10% FBS (Cellgro). The cells were seeded 24 hours prior to compound treatment, into three 96-well F-bottom clear plates (Greiner), with 7,500 cells per well in 180 µL of medium. The 10 mM compound was diluted 100-fold in medium to prepare a 100 µM compound, which was then placed into a 96-well plate and further 3-fold diluted in medium to prepare a compound plate with concentrations ranging from 0.046 µM to 100 µM. 20 µL of each compound from the drug plate was added to the cell-seeded plates to achieve final concentrations of 0.0046 µM to 10 µM. At the same time, 50 µL of 33% trichloroacetic acid was injected into one plate and the plate was fixed by incubating at 4 °C. This was referred to as the D₀ plate. Viable cells were measured 72 hours after compound treatment by injecting 50 µL of 33% trichloroacetic acid and incubating at 4 °C for more than 1 hour to fix the cells. This was referred to as the D₃ plate. After the D₀ plate and D₃ plates were washed 5 times with sterile water, 100 µL of 0.04% Sulforhodamine B solution in 1% acetic acid was added and reacted for 1 hour to stain the cells. After staining, the plates were washed 4 times with 1% acetic acid solution and completely dried. Then, the Sulforhodamine B bound to cellular proteins was completely dissolved with 10 mM Tris base solution (pH 10.5). The absorbance was measured at a wavelength of 510 nm using a microplate reader (TECAN, sunrise microplate reader). The analysis was performed using GraphPad Prism. The average absorbance of the D₀ plate was set to 0%, and the average absorbance of the negative control of the D₃ plate was set to 100%, from which a graph was drawn and the GI₅₀ values were calculated. Each test was performed twice.

### Experimental Example 3. Evaluation of TEAD inhibition of the compound according to the present invention

To evaluate the inhibitory activity on TEAD activity in KYSE-70 cells in which YES1 is overexpressed, the following experiment was performed.

TEAD luciferase reporter lentivirus (BPS Bioscience; TEAD luciferase reporter lentivirus) was co-cultured with KYSE-70 cell line for 48 hours, and then 1 ug/ml puromycin (Invitrogen) was added to the culture to induce cell death only in cells that were not infected with the virus, thereby generating a KYSE-70 cell line into which the TEAD luciferase reporter was introduced. The KYSE-70 TEAD luciferase reporter cell line was cultured in RPMI-1640 (Capricorn) supplemented with 10% FBS (Cellgro). The cells were seeded 24 hours prior to compound treatment into two 96-well F-bottom clear plates (Greiner) and two white clear bottom 96-well plates (Greiner), at 20,000 cells in 180 µL medium per well.

The 10 mM compound was diluted 100-fold in medium to prepare a 100 µM compound, then placed in a 96-well plate and further 3-fold diluted in medium to prepare a compound plate with concentrations ranging from 0.046 µM to 100 µM. 20 µL of each compound from the drug plate was injected into the cell-seeded plates to prepare the final concentrations 0.0046 µM to 10 µM. For TEAD activity measurement, 24 hours after compound treatment on the white clear bottom 96-well plate, 100 µL of medium was removed from each well, and 100 µL of One-Step^{™} Luciferase Assay System solution was added. After reacting at room temperature for 15 minutes, luminescence intensity was measured using a microplate reader (VICTOR X3, PerkinElmer). Since TEAD activity values needed to be normalized by cell viability, a cell viability assay was also performed. For measuring cell viability, 24 hours after compound treatment on the 96-well F-bottom clear plate, 50 µL of 33% trichloroacetic acid was added to each well and was fixed by incubating at 4 °C for 1 hour. After that, the plate was washed 5 times with sterile water, and 100 µL of 0.04% Sulforhodamine B solution in 1% acetic acid was added to react for 1 hour to stain the cells. After staining, the plates were washed 4 times with 1% acetic acid solution and completely dried. Then the Sulforhodamine B bound to cellular proteins was completely dissolved with 10mM Tris base solution (pH 10.5). The absorbance was measured at a wavelength of 510 nm using a microplate reader (TECAN, sunrise microplate reader). The TEAD activity value and cell viability value were normalized, and a graph was drawn using the GraphPad Prism version 5.0 program, and the IC₅₀ value was calculated.

The results for Experimental Examples 2 and 3 are shown in Table 2 below.

**[Table 2]**

| example | KYSE70 | |
|---|---|---|
| | GI₅₀ | TEAD IC₅₀ |
| 1 | C | D |
| 2 | D | C |
| 3 | D | A |
| 4 | C | D |
| 5 | B | B |
| 6 | C | A |
| 7 | C | B |
| 8 | D | B |
| 9 | C | A |
| 10 | C | B |
| 11 | B | B |
| 12 | B | A |
| 13 | A | A |
| 14 | B | A |
| 15 | A | A |
| 16 | C | C |
| 17 | C | A |
| 18 | B | A |
| 19 | A | A |
| 20 | B | A |
| 21 | B | A |
| 22 | B | A |
| 23 | A | A |
| 24 | B | A |
| 25 | B | A |
| 26 | C | A |
| 27 | C | A |
| 28 | C | A |
| 29 | D | B |
| 30 | A | A |
| 32 | N.T. | N.T. |
| 34 | N.T. | N.T. |
| 36 | B | B |
| 37 | B | C |
| 38 | A | A |
| 39 | A | B |
| 40 | C | C |
| 42 | A | A |
| 44 | A | A |
| 45 | A | A |
| 46 | B | A |
| 48 | A | A |
| 51 | B | A |
| 52 | B | A |
| 53 | A | A |
| 54 | A | B |
| 55 | A | B |
| 56 | A | A |
| 58 | B | A |
| 59 | B | A |
| 60 | A | A |
| 61 | A | A |
| 62 | A | A |
| 63 | A | A |
| 64 | A | A |
| 65 | B | A |
| 66 | A | A |
| 67 | B | A |
| 68 | A | A |
| 69 | A | A |
| 70 | B | A |
| 71 | A | A |
| 72 | B | A |
| 73 | A | A |
| 74 | A | A |
| 75 | A | A |
| 76 | A | A |
| 77 | B | A |
| 78 | A | A |
| 79 | A | A |
| 80 | B | A |
| 81 | B | A |
| 82 | A | A |
| 83 | B | B |
| 85 | A | A |
| 87 | A | A |
| 89 | D | D |
| 90 | B | A |
| 91 | B | A |
| 92 | B | A |
| 93 | B | A |
| 94 | A | A |
| 95 | B | A |
| 96 | D | B |
| 97 | D | A |
| 98 | A | A |
| 99 | A | A |
| 100 | A | A |
| 101 | D | D |
| 102 | B | A |
| 103 | B | A |
| 104 | B | B |
| 105 | A | A |
| 106 | A | A |
| 107 | B | A |
| 108 | A | A |
| 109 | A | A |
| 110 | B | B |
| 111 | D | B |
| 112 | B | A |
| 116 | B | A |
| 117 | B | B |
| 119 | B | A |
| 120 | B | A |
| 121 | B | A |
| 123 | A | A |
| 124 | A | A |
| 125 | B | B |
| 126 | B | A |
| 127 | A | A |
| 128 | A | A |
| 129 | A | A |
| 130 | A | A |
| 131 | A | A |
| 132 | B | A |
| 133 | B | B |
| 134 | A | A |
| 135 | A | A |
| 136 | A | A |
| 137 | B | D |
| 138 | D | D |

| | | |
|---|---|---|
| (A: GI₅₀ ≤ 100nM; B: 100nM < GI₅₀ ≤ 1 µM; C: 1µM < GI₅₀ ≤ 10 µM; D: 10 µM < GI₅₀) (A: IC₅₀ ≤ 100nM; B: 100nM < IC₅₀ ≤ 1 µM; C: 1µM < IC₅₀ ≤ 10 µM; D: 10 µM < IC₅₀) N.T.: not tested | | |

As confirmed from Table 2, the compound of the present invention was confirmed to exhibit strong anti-proliferative activity in esophageal cancer cell lines, which are a type of solid cancer. In addition, it was confirmed to exhibit excellent inhibitory activity against transcriptional enhanced associated domain (TEAD) in esophageal cancer cell lines.

From this, it was confirmed that the compound of the present invention has excellent inhibitory activity against YES1 kinase and transcriptional enhanced associated domain (TEAD), and excellent effects in the prevention or treatment of solid cancer associated thereto.

## Claims

1. A compound represented by the following Formula 1 or a pharmaceutically acceptable salt thereof : 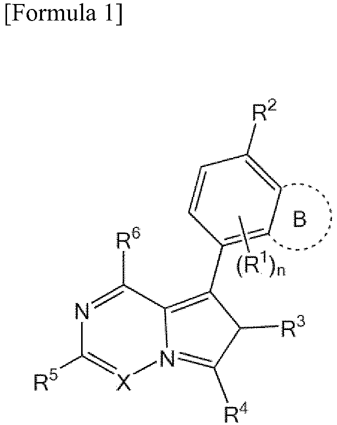
wherein in Formula 1,
X is C or N,
Ring B is absent or is (3-10 membered)cycloalkyl or (3-10 membered)heterocyclyl,
R¹ is each independently selected from the group consisting of hydrogen, halogen, C₁₋₁₀ alkyl, C₁₋₁₀ haloalkyl, C₁₋₁₀ alkoxy, C₁₋₆ haloalkoxy, (3-10 membered)cycloalkyl, C₁₋₁₀ alkylene-(3-10 membered)cycloalkyl, (3-10 membered)heterocyclyl, C₁₋₁₀ alkylene-(3-10 membered)heterocyclyl, (6-14 membered)aryl, C₁₋₁₀ alkylene-(6-14 membered)aryl, (5-12 membered)heteroaryl, C₁₋₁₀ alkylene-(5-12 membered)heteroaryl, NRₐR_{b}, O-C₁₋₁₀ alkylene-NRₐR_{b}, C₁₋₁₀ alkylene-O-NRₐR_{b}, C(O)-NRₐR_{b}, C₁₋₁₀ alkylene-C(O)-NRₐR_{b} and C(O)O-NRₐR_{b},
wherein (3-10 membered)cycloalkyl, (3-10 membered)heterocyclyl, (6-14 membered)aryl and (5-12 membered)heteroaryl are each independently unsubstituted or optionally substituted with a substituent selected from the group consisting of C₁₋₆ alkyl, C₁₋₆ alkoxy, halogen, C₁₋₆ haloalkyl, NH₂, NO₂, OH and CN,
n is an integer from 1 to 4,
R² is selected from the group consisting of hydrogen, halogen, C₁₋₅ alkyl, C₁₋₅ haloalkyl, C₁₋₅ alkoxy, C₁₋₅ haloalkoxy, C(O)-C₁₋₅ alkyl, C(O)-C₁₋₅ haloalkyl, C(O)O-C₁₋₅ alkyl, C(O)O-C₁₋₅ haloalkyl, CC(O)OC₁₋₅ alkyl, CC(O)O-C₁₋₅ haloalkyl, NR_{c}R_{d}, O-(6-14 membered) aryl, and O-(5-12 membered) heteroaryl,
wherein R_{c} and R_{d} are each independently optionally substituted with at least one substituent selected from the group consisting of hydrogen, C₁₋₅ alkyl, C₁₋₅ haloalkyl, C₁₋₅ alkoxy, C₁₋₅ haloalkoxy, C₁₋₃ aryl, C₁₋₃ heteroaryl, C(O)O-C₁₋₅ alkyl, C(O)O-C₁₋₅ haloalkyl, C(O)O-(3-10 membered) cycloalkyl, C(O)C₁₋₃ aryl, C(O)-(5-12 membered) heteroaryl, and C(O)NHC₁₋₅alkyl,
wherein (3-10 membered)cycloalkyl and (5-12 membered) heteroaryl are each independently unsubstituted or optionally substituted with a substituent selected from the group consisting of C₁₋₆alkyl, C₁₋₆alkoxy, halogen and C₁₋6haloalkyl,
R³ is selected from the group consisting of hydrogen, halogen, C₁₋₆alkyl, C₁₋₆haloalkyl, C₁₋₆alkoxy, NH₂, OH, CN, COOH, NO₂, C(O)C₁₋₆alkyl, C(O)OC₁₋₆alkyl and C₁₋₆alkylamino,
R⁴ is selected from the group consisting of (3-10 membered) cycloalkyl, (3-14 membered) heterocyclyl, (3-14 membered) N-heterocyclyl, (6-14 membered) aryl, (5-12 membered) heteroaryl, C₁₋₆ alkylene-(3-10 membered)cycloalkyl, C₁₋₆ alkylene-(3-14 membered) heterocyclyl, C₁₋₆ alkylene-(3-10 membered) N-heterocyclyl, C₁₋₆ alkylene-(6-14
membered) aryl, C₁₋₆ alkylene-(5-12 membered) heteroaryl, NRₒRₚ and 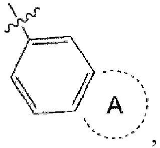
wherein ring A is (3-10 membered) cycloalkyl or (3-14 membered) heterocyclyl, wherein R⁴ is optionally substituted with one or more R⁷,
wherein R⁷ is each independently selected from the group consisting of halogen, C₁₋₆ alkyl, C₁₋₆ haloalkyl, C₁₋₆ alkoxy, C₁₋₆ haloalkoxy, C₁₋₁₀ alkylene-C₁₋₆ alkoxy, C₁₋₁₀ alkylene-C₁₋₆ haloalkoxy, (3-10 membered) cycloalkyl, C₁₋₁₀ alkylene-(3-10 membered) cycloalkyl, (6-14 membered) aryl, C₁₋₁₀ alkylene-(6-14 membered) aryl, (5-12 membered) heteroaryl, C₁₋₁₀ alkylene-(5-12 membered) heteroaryl, NRₑR_{f}, C₁₋₁₀ alkyl-NRₑR_{f}, C₁₋₁₀ alkylene-NRₑR_{f}, oxo, C(O)-NRₑR_{f}, C₁₋₁₀ alkylene-C(O)-NRₑR_{f}, C(O)O-NRₑR_{f}, CN, OH, NO₂, C(O)Rₑ, C(O)ORₑ, C₁₋₆ alkylene-C(O)ORₑ and C₁₋₆ alkylene-OH,
R⁵ is selected from the group consisting of hydrogen, halogen, C₁₋₆ alkyl, C₁₋₆ haloalkyl, C₁₋₆ alkoxy, NH₂, OH, CN, COOH, NO₂, C(O)C₁₋₆ alkyl, C(O)OC₁₋₆ alkyl and C₁₋₆ alkylamino,
R⁶ is NR_{g}Rₕ,
Rₐ, R_{b}, Rₑ, R_{f}, R_{g} and Rₕ are each independently selected from the group consisting of hydrogen, halogen, C₁₋₆ alkyl, C₁₋₆ haloalkyl, C(O)C₁₋₆ alkyl, C(O)C₁₋₆ haloalkyl, C(O)OC₁₋₆ alkyl and C(O)OC₁₋₆ haloalkyl,
Rₒ and Rₚ are each independently selected from the group consisting of hydrogen, halogen, C₁₋₆ alkyl, C₁₋₆ haloalkyl, (3-10 membered)cycloalkyl, C₁₋₁₀ alkylene-(3-10 membered)cycloalkyl, (3-10 membered) heterocyclyl and C₁₋₁₀ alkylene-(3-10 membered) heterocyclyl,
wherein (3-10 membered)cycloalkyl, C₁₋₁₀ alkylene-(3-10 membered)cycloalkyl, (3-10 membered)heterocyclyl or C₁₋₁₀ alkylene-(3-10 membered) heterocyclyl are optionally substituted with one or more R⁸, wherein R⁸ is each independently selected from the group consisting halogen, C₁₋₆ alkyl, C₁₋₆ haloalkyl, C₁₋₆ alkoxy, NR_{q}Rᵣ, C₁₋₁₀ alkylene-NR_{q}Rᵣ, oxo, C(O)-NR_{q}Rᵣ, C₁₋₁₀ alkylene-C(O)-NR_{q}Rᵣ, C(O)O-NR_{q}Rᵣ, CN, OH, NO₂, C(O)R_{q}, C(O)OR_{q}, C₁₋₆ alkylene-C(O)OR_{q} and C₁₋₆ alkylene-OH,
R_{q} and Rᵣ are each independently selected from the group consisting of hydrogen, halogen, C₁₋₆ alkyl and C₁₋₆ haloalkyl.

2. The compound of claim 1, which is represented by Formula 1, or a pharmaceutically acceptable salt thereof :
R¹ is selected from the group consisting of hydrogen, halogen, C₁₋₁₀ alkyl, C₁₋₁₀ haloalkyl, C₁₋₁₀ alkoxy, C₁₋₆ haloalkoxy, NRₐR_{b}, O-C₁₋₁₀ alkylene-NRₐR_{b}, C₁₋₁₀ alkylene-O-NRₐR_{b}, C(O)-NRₐR_{b}, C₁₋₁₀ alkylene-C(O)-NRₐR_{b} and C(O)O-NRₐR_{b},
n is an integer from 1 to 4,
R² is selected from the group consisting of hydrogen, halogen, C₁₋₅ alkyl, C₁₋₅ haloalkyl, C₁₋₅ alkoxy, C₁₋₅ haloalkoxy, NR_{c}R_{d}, O-(6-14 membered) aryl and O-(5-12 membered) heteroaryl,
wherein R_{c} and R_{d} are each independently optionally substituted with at least one substituent selected from the group consisting of hydrogen, C₁₋₅ alkyl, C₁₋₅ haloalkyl, C₁₋₅ alkoxy, C₁₋₅ haloalkoxy, C(O)O-C₁₋₅ alkyl, C(O)O-C₁₋₅ haloalkyl, C(O)O-(3-10 membered)cycloalkyl and C(O)-(5-12 membered)heteroaryl,
wherein (3-10 membered)cycloalkyl and (5-12 membered) heteroaryl are each independently unsubstituted or optionally substituted with C₁₋₆ alkyl or C₁₋₆ haloalkyl,
R³ is selected from the group consisting of hydrogen, halogen, C₁₋₆ alkyl and C₁₋₆ haloalkyl.

3. The compound of claim 1 or 2, which is represented by Formula 1, or a pharmaceutically acceptable salt thereof :
R⁴ is selected from the group consisting of (3-10 membered) cycloalkyl, (3-14 membered) heterocyclyl, (3-14 membered) N-heterocyclyl, (6-14 membered) aryl, (5-12 membered) heteroaryl, C₁₋₆ alkylene-(3-10 membered) cycloalkyl, C₁₋₆ alkylene-(3-14 membered) heterocyclyl, C₁₋₆ alkylene-(3-10 membered) N-heterocyclyl, C₁₋₆ alkylene-(6-14 membered) aryl, C₁₋₆ alkylene-(5-12 membered) heteroaryl, NRₒRₚ and 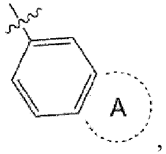
wherein ring A is (3-10 membered) cycloalkyl or (3-14 membered) heterocyclyl,
wherein R⁴ is optionally substituted with one or more R⁷, wherein R⁷ is selected from the group consisting of halogen, C₁₋₆ alkyl, C₁₋₆ haloalkyl, C₁₋₆ alkoxy, C₁₋₆ haloalkoxy, C₁₋₁₀ alkylene-C₁₋₆ alkoxy, C₁₋₁₀ alkylene-C₁₋₆ haloalkoxy, (3-10 membered) cycloalkyl, (6-14 membered) aryl, (5-12 membered) heteroaryl, NRₑR_{f}, C₁₋₁₀ alkylene-NRₑR_{f}, C₁₋₁₀ alkylene-NRₑR_{f}, oxo, C(O)-NRₑR_{f}, C₁₋₁₀ alkylene-C(O)-NRₑR_{f}, C(O)O-NRₑR_{f}, CN, OH, NO₂, C(O)Rₑ, C(O)ORₑ, C(O)ORₑ, C₁₋₆ alkylene-C(O)ORₑ, and C₁₋₆ alkylene-OH,
Rₒ and Rₚ are each independently selected from the group consisting of hydrogen, halogen, C₁₋₆ alkyl, C₁₋₆ haloalkyl, (3-10 membered) cycloalkyl, C₁₋₁₀ alkylene-(3-10 membered) cycloalkyl, (3-10 membered) heterocyclyl and C₁₋₁₀ alkylene-(3-10 membered) heterocyclyl,
wherein (3-10 membered) cycloalkyl, C₁₋₁₀ alkylene-(3-10 membered) cycloalkyl, (3-10 membered) heterocyclyl or C₁₋₁₀ alkylene-(3-10 membered) heterocyclyl are optionally substituted with one or more R⁸, wherein R⁸ is halogen, C₁₋₆ alkyl, C₁₋₆ haloalkyl, C₁₋₆ alkoxy, NR_{q}Rᵣ, C₁₋₁₀ alkylene-NR_{q}Rᵣ, oxo, C(O)-NR_{q}Rᵣ, C₁₋₁₀ alkylene-C(O)-NR_{q}Rᵣ, C(O)O-NR_{q}Rᵣ, CN, OH, NO₂, C(O)R_{q}, C(O)OR_{q}, C₁₋₆ alkylene-C(O)OR_{q} and C₁₋₆ alkylene-OH,
R_{q} and Rᵣ are each independently selected from the group consisting of hydrogen, halogen, C₁₋₆ alkyl and C₁₋₆ haloalkyl.

4. The compound of any one of claims 1 to 3, which is represented by Formula 1, or a pharmaceutically acceptable salt thereof :
R⁵ is selected from the group consisting of hydrogen, halogen, C₁₋₆ alkyl, C₁₋₆ haloalkyl, C₁₋₆ alkoxy, NH₂, OH, CN, COOH, NO₂, C(O)C₁₋₆ alkyl, C(O)OC₁₋₆ alkyl, and C₁₋₆ alkylamino,
R⁶ is NR_{g}Rₕ,
R_{g} and Rₕ are each independently selected from the group consisting of hydrogen, C₁₋₆ alkyl, C₁₋₆ haloalkyl, and C(O)C₁₋₆ alkyl.

5. The compound of any one of claims 1 to 4, which is represented by Formula 1, or a pharmaceutically acceptable salt thereof :
R¹ is selected from the group consisting of hydrogen, halogen, C₁₋₁₀ alkyl, C₁₋₁₀ haloalkyl, C₁₋₁₀ alkoxy, and C₁₋₆ haloalkoxy,
R² is NR_{c}R_{d}, O-(6-14 membered) aryl or O-(5-12 membered) heteroaryl,
wherein R_{c} and R_{d} are each independently hydrogen, C(O)O-C₁₋₅ alkyl, C(O)O-C₁₋₅ haloalkyl, C(O)O-(3-10 membered) cycloalkyl and C(O)-(5-12 membered) heteroaryl,
wherein (3-10 membered) cycloalkyl and (5-12 membered) heteroaryl are each independently unsubstituted or optionally substituted with a substituent selected from the group consisting of C₁₋₆ alkyl, C₁₋₆ alkoxy, halogen, and C₁₋₆ haloalkyl,
R³ is selected from the group consisting of hydrogen, halogen, C₁₋₆ alkyl and C₁₋₆ haloalkyl,
R⁴ is selected from the group consisting of monocyclic (3-7 membered) cycloalkyl, monocyclic (3-6 membered) heterocyclyl, spirocyclic (7-14 membered) heterocyclyl, fused cyclic (7-14 membered) heterocyclyl, (6-10 membered) aryl, (5-10 membered) heteroaryl, C₁₋₆ alkylene-(3-10 membered) N-heterocyclyl, NRₒRₚ and 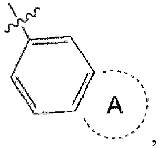
wherein ring A is (3-10 membered) cycloalkyl or (3-14 membered) heterocyclyl,
wherein R⁴ is optionally substituted with one or more R⁷, wherein R⁷ is selected from the group consisting of halogen, C₁₋₆ alkyl, C₁₋₆ haloalkyl, C₁₋₆ alkoxy, C₁₋₆ haloalkoxy, C₁₋₁₀ alkylene-C₁₋₆ alkoxy, (3-10 membered) cycloalkyl, (6-14 membered) aryl, (5-12 membered) heteroaryl, NRₑR_{f}, C₁₋₁₀ alkylene-NRₑR_{f}, oxo, CN, OH, NO₂, C(O)ORₑ, C₁₋₆ alkylene-C(O)ORₑ and C₁₋₆ alkylene-OH,
R⁵ is hydrogen or halogen,
R⁶ is NR_{g}Rₕ,
Rₑ, R_{f}, R_{g} and Rₕ are each independently selected from the group consisting of hydrogen, C₁₋₆ alkyl, C₁₋₆ haloalkyl and C(O)C₁₋₆ alkyl,
Rₒ and Rₚ are each independently selected from the group consisting of hydrogen, halogen, C₁₋₆ alkyl, C₁₋₆ haloalkyl, (3-10 membered) cycloalkyl, C₁₋₁₀ alkylene-(3-10 membered) cycloalkyl, (3-10 membered) heterocyclyl and C₁₋₁₀ alkylene-(3-10 membered) heterocyclyl,
wherein (3-10 membered) cycloalkyl, C₁₋₁₀ alkylene-(3-10 membered) cycloalkyl, (3-10 membered) heterocyclyl or C₁₋₁₀ alkylene-(3-10 membered) heterocyclyl is optionally substituted with one or more R⁸, wherein R⁸ is selected from the group consisting of halogen, C₁₋₆ alkyl, C₁₋₆ haloalkyl, C₁₋₆ alkoxy, NR_{q}Rᵣ, C₁₋₁₀ alkylene-NR_{q}Rᵣ, oxo, C(O)-NR_{q}Rᵣ, C₁₋₁₀ alkylene-C(O)-NR_{q}Rᵣ, C(O)O-NR_{q}Rᵣ, CN, OH, NO₂, C(O)R_{q}, C(O)OR_{q}, C₁₋₆ alkylene-C(O)OR_{q} and C₁₋₆ alkylene-OH,
R_{q} and Rᵣ are each independently selected from the group consisting of hydrogen, halogen, C₁₋₆ alkyl and C₁₋₆ haloalkyl.

6. The compound of any one of claims 1 to 5, which is represented by Formula 1, or a pharmaceutically acceptable salt thereof :
wherein ring B is absent or is (3-10 membered) heterocyclyl,
wherein R¹ is hydrogen or methoxy,
wherein R² is selected from the group consisting of 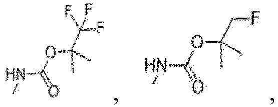 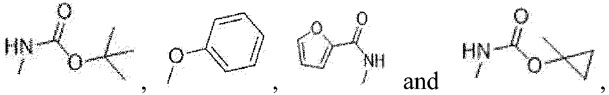
wherein R³ is selected from the group consisting of hydrogen, halogen, C₁₋₃ alkyl and C₁₋₃ haloalkyl.

7. The compound of any one of claims 1 to 6, which is represented by Formula 1, or a pharmaceutically acceptable salt thereof :
wherein R⁴ is **characterized by** being one selected from the group consisting of

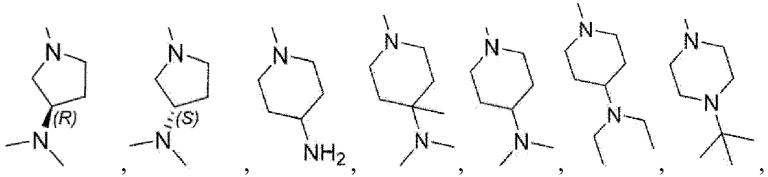

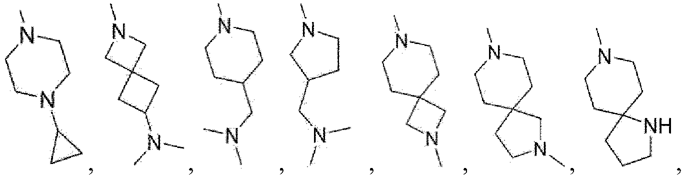

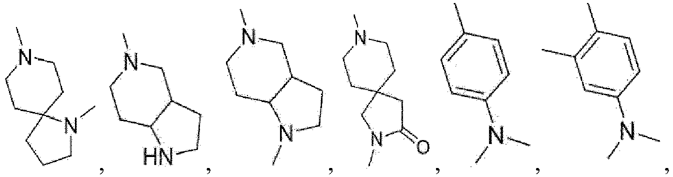

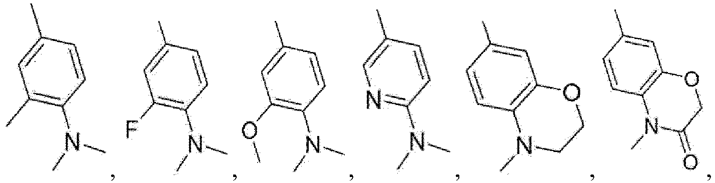

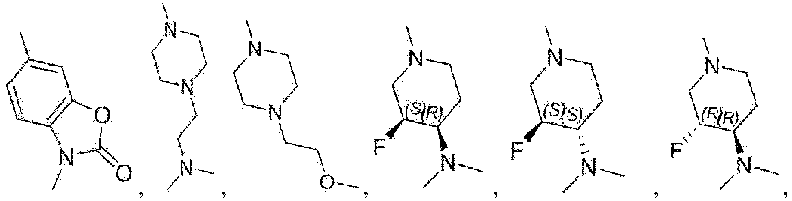

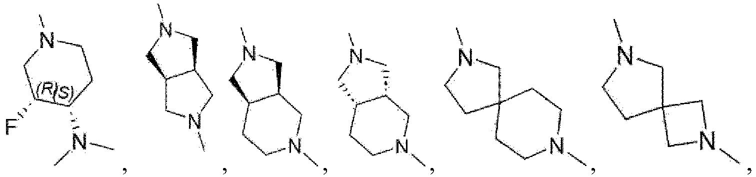

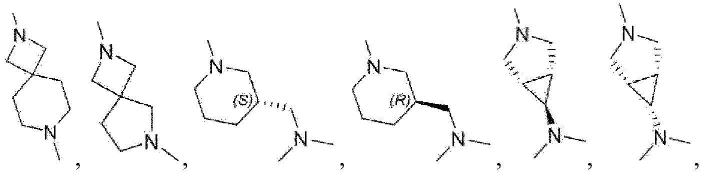

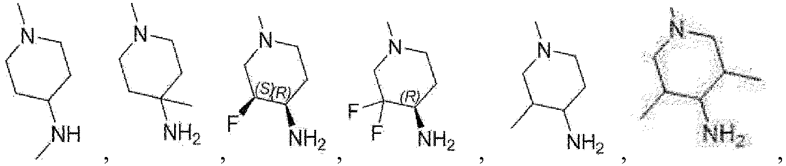

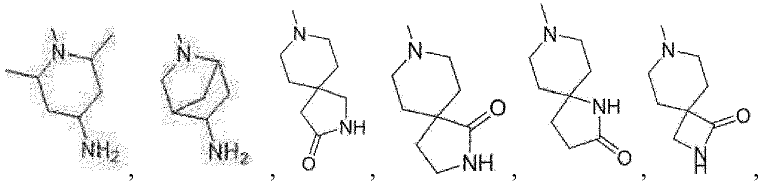

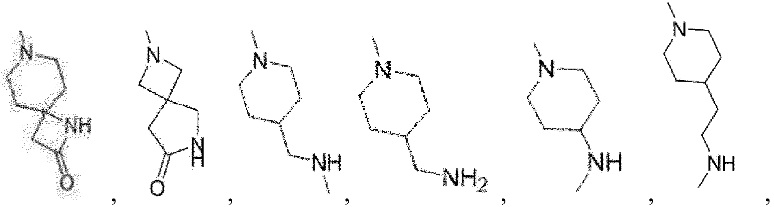

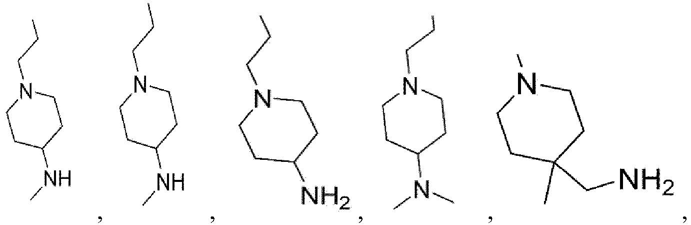

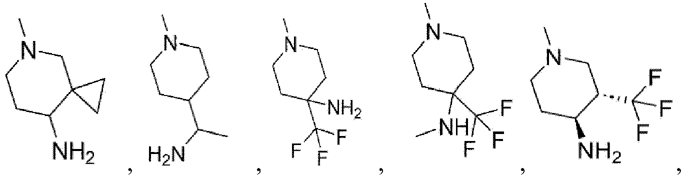

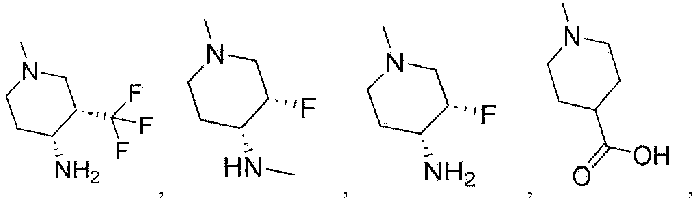

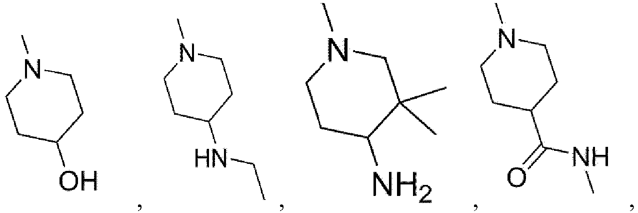

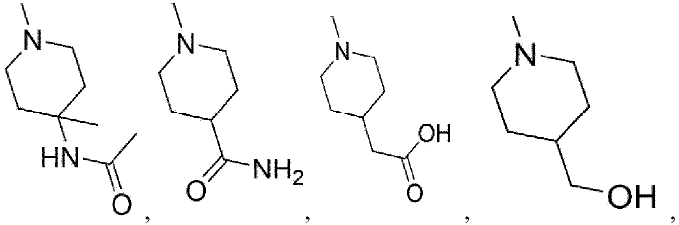

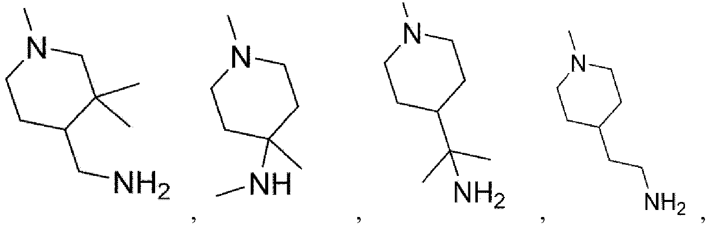

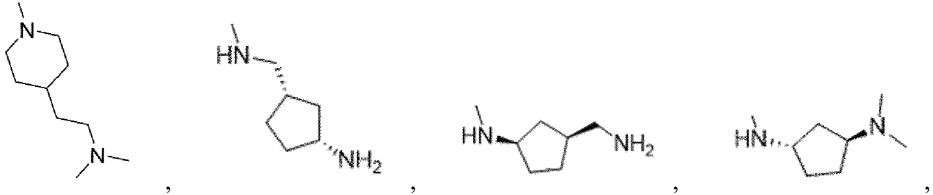

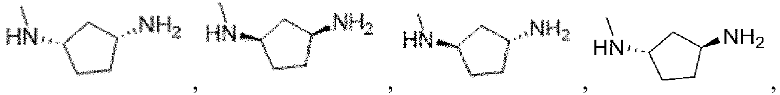

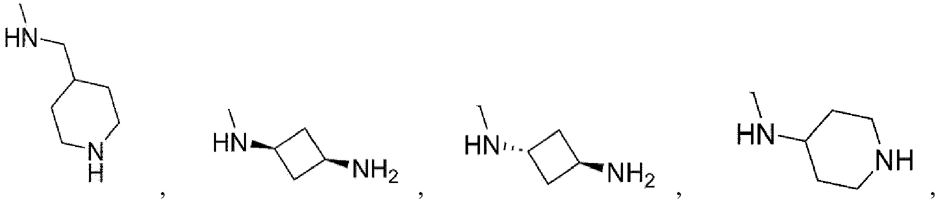

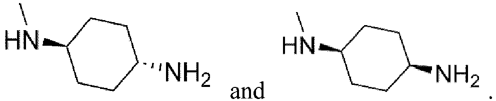

8. The compound of any one of claims 1 to 7, which is represented by Formula 1, or a pharmaceutically acceptable salt thereof :
R⁵ is hydrogen or halogen,
R⁶ is NR_{g}Rₕ,
R_{g} and Rₕ are each independently hydrogen or C₁₋₆ alkyl.

9. The compound of any one of claims 1 to 8, which is represented by Formula 1, or a pharmaceutically acceptable salt thereof : wherein 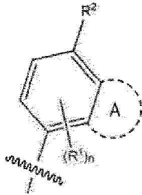 is selected from the group consisting of

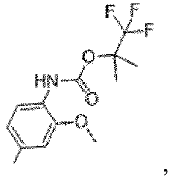

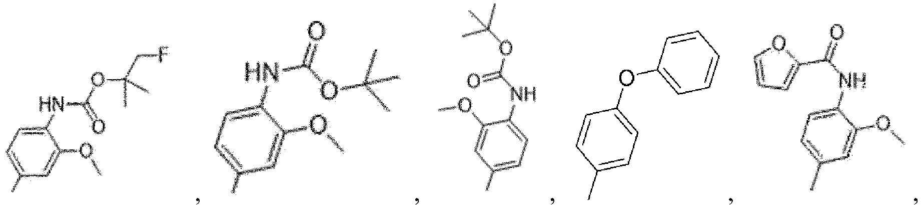

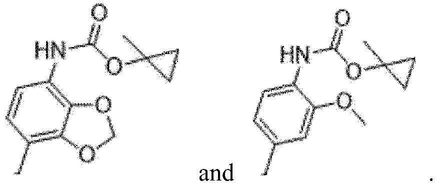

10. A compound selected from the group consisting of compounds 1 to 138 below, or a pharmaceutically acceptable salt thereof.
| No. | Compound name |
|---|---|
| 1 | tert-butyl (4-(4-amino-7-(4-(dimethylamino)phenyl)pyrrolo[2,1-f][1,2,4]triazin-5-yl)-2-methoxyphenyl)carbamate |
| 2 | tert-butyl (4-(4-amino-7-(4-(dimethylamino)-2-methylphenyl)pyrrolo[2,1-f][1,2,4]triazin-5-yl)-2-methoxyphenyl)carbamate |
| 3 | tert-butyl (4-(4-amino-7-(4-(dimethylamino)-3-methylphenyl)pyrrolo[2,1-f][1,2,4]triazin-5-yl)-2-methoxyphenyl)carbamate |
| 4 | tert-butyl (4-(4-amino-7-(4-(dimethylamino)-3-fluorophenyl)pyrrolo[2,1-f][1,2,4]triazin-5-yl)-2-methoxyphenyl)carbamate |
| 5 | tert-butyl (4-(4-amino-7-(4-(dimethylamino)-3-methoxyphenyl)pyrrolo[2,1-f][1,2,4]triazin-5-yl)-2-methoxyphenyl)carbamate |
| 6 | tert-butyl (4-(4-amino-7-(6-(dimethylamino)pyridin-3-yl)pyrrolo[2,1-f][1,2,4]triazin-5-yl)-2-methoxyphenyl)carbamate |
| 7 | tert-butyl (4-(4-amino-7-(4-methyl-3,4-dihydro-2H-benzo[b][1,4]oxazin-7-yl)pyrrolo[2,1-f][1,2,4]triazin-5-yl)-2-methoxyphenyl)carbamate |
| 8 | tert-butyl (4-(4-amino-7-(4-methyl-3-oxo-3,4-dihydro-2H-benzo[b][1,4]oxazin-7-yl)pyrrolo[2,1-f][1,2,4]triazin-5-yl)-2-methoxyphenyl)carbamate |
| 9 | tert-butyl (4-(4-amino-7-(3-methyl-2-oxo-2,3-dihydrobenzo[d]oxazol-6-yl)pyrrolo[2,1-f][1,2,4]triazin-5-yl)-2-methoxyphenyl)carbamate |
| 10 | tert-butyl (R)-(4-(4-amino-7-(3-(dimethylamino)pyrrolidin-1-yl)pyrrolo[2,1-f][1,2,4]triazin-5-yl)-2-methoxyphenyl)carbamate |
| 11 | tert-butyl (S)-(4-(4-amino-7-(3-(dimethylamino)pyrrolidin-1-yl)pyrrolo[2,1-f][1,2,4]triazin-5-yl)-2-methoxyphenyl)carbamate |
| 12 | tert-butyl (4-(4-amino-7-(4-aminopiperidin-1-yl)pyrrolo[2,1-f][1,2,4]triazin-5-yl)-2-methoxyphenyl)carbamate |
| 13 | tert-butyl (4-(4-amino-7-(4-(dimethylamino)-4-methylpiperidin-1-yl)pyrrolo[2,1-f][1,2,4]triazin-5-yl)-2-methoxyphenyl)carbamate |
| 14 | tert-butyl (4-(4-amino-7-(4-(diethylamino)piperidin-1-yl)pyrrolo[2,1-f][1,2,4]triazin-5-yl)-2-methoxyphenyl)carbamate |
| 15 | tert-butyl (4-(4-amino-7-(4-(dimethylamino)piperidin-1-yl)pyrrolo[2,1-f][1,2,4]triazin-5-yl)-2-methoxyphenyl)carbamate |
| 16 | tert-butyl (4-(4-amino-7-(4-(tert-butyl)piperazin-1-yl)pyrrolo[2,1-f][1,2,4]triazin-5-yl)-2-methoxyphenyl)carbamate |
| 17 | tert-butyl (4-(4-amino-7-(4-cyclopropylpiperazin-1-yl)pyrrolo[2,1-f][1,2,4]triazin-5-yl)-2-methoxyphenyl)carbamate |
| 18 | tert-butyl (4-(4-amino-7-(6-(dimethylamino)-2-azaspiro[3.3]heptan-2-yl)pyrrolo[2,1-f][1,2,4]triazin-5-yl)-2-methoxyphenyl)carbamate |
| 19 | tert-butyl (4-(4-amino-7-(4-((dimethylamino)methyl)piperidin-1-yl)pyrrolo[2,1-f][1,2,4]triazin-5-yl)-2-methoxyphenyl)carbamate |
| 20 | tert-butyl (4-(4-amino-7-(3-((dimethylamino)methyl)pyrrolidin-1-yl)pyrrolo[2,1-f][1,2,4]triazin-5-yl)-2-methoxyphenyl)carbamate |
| 21 | tert-butyl (4-(4-amino-7-(2-methyl-2,7-diazaspiro[3.5]nonan-7-yl)pyrrolo[2,1-f][1,2,4]triazin-5-yl)-2-methoxyphenyl)carbamate |
| 22 | tert-butyl (4-(4-amino-7-(2-methyl-2,8-diazaspiro[4.5]decan-8-yl)pyrrolo[2,1-f][1,2,4]triazin-5-yl)-2-methoxyphenyl)carbamate |
| 23 | tert-butyl (4-(4-amino-7-(1,8-diazaspiro[4.5]decan-8-yl)pyrrolo[2,1-f][1,2,4]triazin-5-yl)-2-methoxyphenyl)carbamate |
| 24 | tert-butyl (4-(4-amino-7-(1-methyl-1,8-diazaspiro[4.5]decan-8-yl)pyrrolo[2,1-f][1,2,4]triazin-5-yl)-2-methoxyphenyl)carbamate |
| 25 | tert-butyl (4-(4-amino-7-(octahydro-5H-pyrrolo[3,2-c]pyridin-5-yl)pyrrolo[2,1-f][1,2,4]triazin-5-yl)-2-methoxyphenyl)carbamate |
| 26 | tert-butyl (4-(4-amino-7-(1-methyloctahydro-5H-pyrrolo[3,2-c]pyridin-5-yl)pyrrolo[2,1-f][1,2,4]triazin-5-yl)-2-methoxyphenyl)carbamate |
| 27 | tert-butyl (4-(4-amino-7-(2-methyl-3-oxo-2,8-diazaspiro[4.5]decan-8-yl)pyrrolo[2,1-f][1,2,4]triazin-5-yl)-2-methoxyphenyl)carbamate |
| 28 | tert-butyl (4-(4-amino-7-(4-(2-(dimethylamino)ethyl)piperazin-1-yl)pyrrolo[2,1-f][1,2,4]triazin-5-yl)-2-methoxyphenyl)carbamate |
| 29 | tert-butyl (4-(4-amino-7-(4-(2-methoxyethyl)piperazin-1-yl)pyrrolo[2,1-f][1,2,4]triazin-5-yl)-2-methoxyphenyl)carbamate |
| 30 | tert-butyl (4-(4-amino-7-((3S,4R)-4-(dimethylamino)-3-fluoropiperidin-1-yl)pyrrolo[2,1-f][1,2,4]triazin-5-yl)-2-methoxyphenyl)carbamate |
| 31 | tert-butyl (4-(4-amino-7-((3S,4S)-4-(dimethylamino)-3-fluoropiperidin-1-yl)pyrrolo[2,1-f][1,2,4]triazin-5-yl)-2-methoxyphenyl)carbamate |
| 32 | tert-butyl (4-(4-amino-7-((3R,4R)-4-(dimethylamino)-3-fluoropiperidin-1-yl)pyrrolo[2,1-f][1,2,4]triazin-5-yl)-2-methoxyphenyl)carbamate |
| 33 | tert-butyl (4-(4-amino-7-((3R,4S)-4-(dimethylamino)-3-fluoropiperidin-1-yl)pyrrolo[2,1-f][1,2,4]triazin-5-yl)-2-methoxyphenyl)carbamate |
| 34 | tert-butyl (4-(4-amino-7-((3aR,6aS)-5-methylhexahydropyrrolo[3,4-c]pyrrol-2(1H)-yl)pyrrolo[2,1-f][1,2,4]triazin-5-yl)-2-methoxyphenyl)carbamate |
| 35A | tert-butyl (4-(4-amino-7-((3aR,7aR)-5-methyloctahydro-2H-pyrrolo[3,4-c]pyridin-2-yl)pyrrolo[2,1-f][1,2,4]triazin-5-yl)-2-methoxyphenyl)carbamate |
| 35B | tert-butyl (4-(4-amino-7-((3aS,7aS)-5-methyloctahydro-2H-pyrrolo[3,4-c]pyridin-2-yl)pyrrolo[2,1-f][1,2,4]triazin-5-yl)-2-methoxyphenyl)carbamate |
| 36 | tert-butyl (4-(4-amino-7-(8-methyl-2,8-diazaspiro[4.5]decan-2-yl)pyrrolo[2,1-f][1,2,4]triazin-5-yl)-2-methoxyphenyl)carbamate |
| 37 | tert-butyl (4-(4-amino-7-(2-methyl-2,6-diazaspiro[3.4]octan-6-yl)pyrrolo[2,1-f][1,2,4]triazin-5-yl)-2-methoxyphenyl)carbamate |
| 38 | tert-butyl (4-(4-amino-7-(7-methyl-2,7-diazaspiro[3.5]nonan-2-yl)pyrrolo[2,1-f][1,2,4]triazin-5-yl)-2-methoxyphenyl)carbamate |
| 39 | tert-butyl (4-(4-amino-7-(6-methyl-2,6-diazaspiro[3.4]octan-2-yl)pyrrolo[2,1-f][1,2,4]triazin-5-yl)-2-methoxyphenyl)carbamate |
| 40 | tert-butyl (S)-(4-(4-amino-7-(3-((dimethylamino)methyl)piperidin-1-yl)pyrrolo[2,1-f][1,2,4]triazin-5-yl)-2-methoxyphenyl)carbamate |
| 41 | tert-butyl (R)-(4-(4-amino-7-(3-((dimethylamino)methyl)piperidin-1-yl)pyrrolo[2,1-f][1,2,4]triazin-5-yl)-2-methoxyphenyl)carbamate |
| 42 | tert-butyl (4-(4-amino-7-((1R,5S,6S)-6-(dimethylamino)-3-azabicyclo[3.1.0]hexan-3-yl)pyrrolo[2,1-f][1,2,4]triazin-5-yl)-2-methoxyphenyl)carbamate |
| 44 | tert-butyl (4-(4-amino-7-(4-(methylamino)piperidin-1-yl)pyrrolo[2,1-f][1,2,4]triazin-5-yl)-2-methoxyphenyl)carbamate |
| 45 | tert-butyl (4-(4-amino-7-(4-amino-4-methylpiperidin-1-yl)pyrrolo[2,1-f][1,2,4]triazin-5-yl)-2-methoxyphenyl)carbamate |
| 46 | tert-butyl (4-(4-amino-7-((3S,4R)-4-amino-3-fluoropiperidin-1-yl)pyrrolo[2,1-f][1,2,4]triazin-5-yl)-2-methoxyphenyl)carbamate |
| 47 | tert-butyl (R)-(4-(4-amino-7-(4-amino-3,3-difluoropiperidin-1-yl)pyrrolo[2,1-f][1,2,4]triazin-5-yl)-2-methoxyphenyl)carbamate |
| 48 | tert-butyl (4-(4-amino-7-(4-amino-3-methylpiperidin-1-yl)pyrrolo[2,1-f][1,2,4]triazin-5-yl)-2-methoxyphenyl)carbamate |
| 49 | tert-butyl (4-(4-amino-7-(4-amino-3,5-dimethylpiperidin-1-yl)pyrrolo[2,1-f][1,2,4]triazin-5-yl)-2-methoxyphenyl)carbamate |
| 50 | tert-butyl (4-(4-amino-7-(4-amino-2,6-dimethylpiperidin-1-yl)pyrrolo[2,1-f][1,2,4]triazin-5-yl)-2-methoxyphenyl)carbamate |
| 51 | tert-butyl (4-(4-amino-7-(5-amino-2-azabicyclo[2.2.1]hepten-2-yl)pyrrolo[2,1-f][1,2,4]triazin-5-yl)-2-methoxyphenyl)carbamate |
| 52 | tert-butyl (4-(4-amino-7-(3-oxo-2,8-diazaspiro[4.5]decan-8-yl)pyrrolo[2,1-f][1,2,4]triazin-5-yl)-2-methoxyphenyl)carbamate |
| 53 | tert-butyl (4-(4-amino-7-(1-oxo-2,8-diazaspiro[4.5]decan-8-yl)pyrrolo[2,1-f][1,2,4]triazin-5-yl)-2-methoxyphenyl)carbamate |
| 54 | tert-butyl (4-(4-amino-7-(2-oxo-1,8-diazaspiro[4.5]decan-8-yl)pyrrolo[2,1-f][1,2,4]triazin-5-yl)-2-methoxyphenyl)carbamate |
| 55 | tert-butyl (4-(4-amino-7-(1-oxo-2,7-diazaspiro[3.5]nonan-7-yl)pyrrolo[2,1-f][1,2,4]triazin-5-yl)-2-methoxyphenyl)carbamate |
| 56 | tert-butyl (4-(4-amino-7-(2-oxo-1,7-diazaspiro[3.5]nonan-7-yl)pyrrolo[2,1-f][1,2,4]triazin-5-yl)-2-methoxyphenyl)carbamate |
| 57 | tert-butyl (4-(4-amino-7-(7-oxo-2,6-diazaspiro[3.4]octen-2-yl)pyrrolo[2,1-f][1,2,4]triazin-5-yl)-2-methoxyphenyl)carbamate |
| 58 | tert-butyl (4-(4-amino-7-(4-(methylamino)piperidin-1-yl)pyrrolo[2,1-f][1,2,4]triazin-5-yl)-2-methoxyphenyl)carbamate |
| 59 | tert-butyl (4-(4-amino-7-(4-(aminomethyl)piperidin-1-yl)pyrrolo[2,1-f][1,2,4]triazin-5-yl)-2-methoxyphenyl)carbamate |
| 60 | 1-fluoro-2-methylpropan-2-yl (4-(4-amino-7-(4-aminopiperidin-1-yl)-6-methylpyrrolo[2,1-f][1,2,4]triazin-5-yl)-2-methoxyphenyl)carbamate |
| 61 | 1-fluoro-2-methylpropan-2-yl (4-(4-amino-6-methyl-7-(4-(methylamino)piperidin-1-yl)pyrrolo[2,1-f][1,2,4]triazin-5-yl)-2-methoxyphenyl)carbamate |
| 62 | 1-fluoro-2-methylpropan-2-yl (4-(4-amino-7-(4-(dimethylamino)piperidin-1-yl)-6-methylpyrrolo[2,1-f][1,2,4]triazin-5-yl)-2-methylmethoxy)carbamate |
| 63 | 1-fluoro-2-methylpropan-2-yl (4-(4-amino-6-methyl-7-(4-(2-(methylamino)ethyl)piperidin-1-yl)pyrrolo[2,1-f][1,2,4]triazin-5-yl)-2-methoxyphenyl)carbamate |
| 64 | 1-fluoro-2-methylpropan-2-yl (4-(4-amino-6-methyl-7-(2-(4-(methylamino)piperidin-1-yl)ethyl)pyrrolo[2,1-f][1,2,4]triazin-5-yl)-2-methoxyphenyl)carbamate |
| 65 | 1-fluoro-2-methylpropan-2-yl (4-(4-amino-7-(4-aminopiperidin-1-yl)pyrrolo[2,1-f][1,2,4]triazin-5-yl)-2-methoxyphenyl)carbamate |
| 66 | 1-fluoro-2-methylpropan-2-yl (4-(4-amino-7-(4-(2-(methylamino)ethyl)piperidin-1-yl)pyrrolo[2,1-f][1,2,4]triazin-5-yl)-2-methoxyphenyl)carbamate |
| 67 | 1-fluoro-2-methylpropan-2-yl (4-(4-amino-7-(2-(4-(methylamino)piperidin-1-yl)ethyl)pyrrolo[2,1-f][1,2,4]triazin-5-yl)-2-methoxyphenyl)carbamate |
| 68 | 1-fluoro-2-methylpropan-2-yl (4-(4-amino-7-(2-(4-aminopiperidin-1-yl)ethyl)pyrrolo[2,1-f][1,2,4]triazin-5-yl)-2-methoxyphenyl)carbamate |
| 69 | 1-fluoro-2-methylpropan-2-yl (4-(4-amino-7-(2-(4-(dimethylamino)piperidin-1-yl)ethyl)pyrrolo[2,1-f][1,2,4]triazin-5-yl)-2-methoxyphenyl)carbamate |
| 70 | 1-methylcyclopropyl (7-(4-amino-7-(4-aminopiperidin-1-yl)pyrrolo[2,1-f][1,2,4]triazin-5-yl)benzo[d][1,3]dioxo-4-yl)carbamate |
| 71 | 1-methylcyclopropyl (4-(4-amino-7-(4-aminopiperidin-1-yl)pyrrolo[2,1-f][1,2,4]triazin-5-yl)-2-methoxyphenyl)carbamate |
| 72 | 1,1,1-trifluoro-2-methylpropan-2-yl (4-(4-amino-7-(4-aminopiperidin-1-yl)pyrrolo[2,1-f][1,2,4]triazin-5-yl)-2-methoxyphenyl)carbamate |
| 73 | tert-butyl (7-(4-amino-7-(4-aminopiperidin-1-yl)pyrrolo[2,1-f][1,2,4]triazin-5-yl)benzo[d][1,3]dioxo-4-yl)carbamate |
| 74 | tert-butyl (4-(4-amino-7-(4-aminopiperidin-1-yl)-6-methylpyrrolo[2,1-f][1,2,4]triazin-5-yl)-2-methoxyphenyl)carbamate |
| 75 | tert-butyl (4-(4-amino-6-methyl-7-(4-(methylamino)piperidin-1-yl)pyrrolo[2,1-f][1,2,4]triazin-5-yl)-2-methoxyphenyl)carbamate |
| 76 | tert-butyl (4-(4-amino-7-(4-(dimethylamino)piperidin-1-yl)-6-methylpyrrolo[2,1-f][1,2,4]triazin-5-yl)-2-methoxyphenyl)carbamate |
| 77 | tert-butyl (4-(4-amino-6-methyl-7-(4-(2-(methylamino)ethyl)piperidin-1-yl)pyrrolo[2,1-f][1,2,4]triazin-5-yl)-2-methoxyphenyl)carbamate |
| 78 | tert-butyl (4-(4-amino-7-(4-aminopiperidin-1-yl)-6-methylpyrrolo[2,1-f][1,2,4]triazin-5-yl)-2-methoxyphenyl)carbamate |
| 79 | tert-butyl (4-(4-amino-7-(4-(aminomethyl)-4-methylpiperidin-1-yl)pyrrolo[2,1-f][1,2,4]triazin-5-yl)-2-methoxyphenyl)carbamate |
| 80 | tert-butyl (4-(4-amino-7-(8-amino-5-azaspiro[2.5]octen-5-yl)pyrrolo[2,1-f][1,2,4]triazin-5-yl)-2-methoxyphenyl)carbamate |
| 81 | tert-butyl (4-(4-amino-7-(4-(1-aminoethyl)piperidin-1-yl)pyrrolo[2,1-f][1,2,4]triazin-5-yl)-2-methoxyphenyl)carbamate |
| 82 | tert-butyl (4-(4-amino-7-(4-amino-4-ethylpiperidin-1-yl)pyrrolo[2,1-f][1,2,4]triazin-5-yl)-2-methoxyphenyl)carbamate |
| 83 | tert-butyl (4-(4-amino-7-(4-amino-4-(trifluoromethyl)piperidin-1-yl)pyrrolo[2,1-f][1,2,4]triazin-5-yl)-2-methoxyphenyl)carbamate |
| 84 | tert-butyl (4-(4-amino-7-(4-(methylamino)-4-(trifluoromethyl)piperidin-1-yl)pyrrolo[2,1-f][1,2,4]triazin-5-yl)-2-methoxyphenyl)carbamate |
| 85 | tert-butyl (4-(4-amino-7-((3S,4S)-4-amino-3-(trifluoromethyl)piperidin-1-yl)pyrrolo[2,1-f][1,2,4]triazin-5-yl)-2-methoxyphenyl)carbamate |
| 86 | tert-butyl (4-(4-amino-7-((3S,4R)-4-amino-3-(trifluoromethyl)piperidin-1-yl)pyrrolo[2,1-f][1,2,4]triazin-5-yl)-2-methoxyphenyl)carbamate |
| 87 | tert-butyl (4-(4-amino-7-((3S,4R)-3-fluoro-4-(methylamino)piperidin-1-yl)pyrrolo[2,1-f][1,2,4]triazin-5-yl)-2-methoxyphenyl)carbamate |
| 89 | 1-(4-amino-5-(4-((tert-butoxycarbonyl)amino)-3-methoxyphenyl)pyrrolo[2,1-f][1,2,4]triazin-7-yl)piperidine-4-carboxylic acid |
| 90 | tert-butyl (4-(4-amino-7-(4-hydroxypiperidin-1-yl)pyrrolo[2,1-f][1,2,4]triazin-5-yl)-2-methoxyphenyl)carbamate |
| 91 | tert-butyl (4-(4-amino-7-(4-amino-3,5-dimethylpiperidin-1-yl)pyrrolo[2,1-f][1,2,4]triazin-5-yl)-2-methoxyphenyl)carbamate |
| 92 | tert-butyl (4-(4-amino-7-(4-(ethylamino)piperidin-1-yl)pyrrolo[2,1-f][1,2,4]triazin-5-yl)-2-methoxyphenyl)carbamate |
| 93 | tert-butyl (4-(4-amino-7-(6-amino-3-azabicyclo[3.1.1]hepten-3-yl)pyrrolo[2,1-f][1,2,4]triazin-5-yl)-2-methoxyphenyl)carbamate |
| 94 | tert-butyl (4-(4-amino-7-(4-amino-3,3-dimethylpiperidin-1-yl)pyrrolo[2,1-f][1,2,4]triazin-5-yl)-2-methoxyphenyl)carbamate |
| 95 | tert-butyl (4-(4-amino-7-(4-(methylcarbamoyl)piperidin-1-yl)pyrrolo[2,1-f][1,2,4]triazin-5-yl)-2-methoxyphenyl) |
| 96 | tert-butyl (4-(7-(4-acetamido-4-methylpiperidin-yl)-4-aminopyrrolo[2,1-f][1,2,4]triazin-5-yl)-2-methoxyphenyl)carbamate |
| 97 | tert-butyl (4-(4-amino-7-(4-carbamoylpiperidin-1-yl)pyrrolo[2,1-f][1,2,4]triazin-5-yl)-2-methoxyphenyl)carbamate |
| 98 | tert-butyl (4-(4-amino-7-(2-(4-(dimethylamino)piperidin-1-yl)ethyl)pyrrolo[2,1-f][1,2,4]triazin-5-yl)-2-methoxyphenyl)carbamate |
| 99 | tert-butyl (4-(4-amino-7-(2-(4-(methylamino)piperidin-1-yl)ethyl)pyrrolo[2,1-f][1,2,4]triazin-5-yl)-2-methoxyphenyl)carbamate |
| 100 | tert-butyl (4-(4-amino-7-(2-(4-aminopiperidin-1-yl)ethyl)pyrrolo[2,1-f][1,2,4]triazin-5-yl)-2-methoxyphenyl)carbamate |
| 101 | 2-(1-(4-amino-5-(4-((tert-butoxycarbonyl)amino)-3-methoxyphenyl)pyrrolo[2,1-f][1,2,4]triazin-7-yl)piperidin-4-yl)acetic acid |
| 102 | tert-butyl (4-(4-amino-7-(4-(hydroxymethyl)piperidin-1-yl)pyrrolo[2,1-f][1,2,4]triazin-5-yl)-2-methoxyphenyl)carbamate |
| 103 | tert-butyl (4-(7-(4-acetamidopiperidin-1-yl)-4-aminopyrrolo[2,1-f][1,2,4]triazin-5-yl)-2-methoxyphenyl)carbamate |
| 104 | tert-butyl (4-(4-amino-7-(4-(aminomethyl)-3,3-dimethylpiperidin-1-yl)pyrrolo[2,1-f][1,2,4]triazin-5-yl)-2-methoxyphenyl)carbamate |
| 105 | tert-butyl (4-(4-amino-7-(4-methyl-4-(methylamino)piperidin-1-yl)pyrrolo[2,1-f][1,2,4]triazin-5-yl)-2-methoxyphenyl)carbamate |
| 106 | tert-butyl (4-(4-amino-7-(4-(2-aminopropan-2-yl)piperidin-1-yl)pyrrolo[2,1-f][1,2,4]triazin-5-yl)-2-methoxyphenyl)carbamate |
| 107 | tert-butyl (4-(4-amino-7-(4-(2-aminoethyl)piperidin-1-yl)pyrrolo[2,1-f][1,2,4]triazin-5-yl)-2-methoxyphenyl)carbamate |
| 108 | tert-butyl (4-(4-amino-7-(4-(2-(methylamino)ethyl)piperidin-1-yl)pyrrolo[2,1-f][1,2,4]triazin-5-yl)-2-methoxyphenyl)carbamate |
| 109 | tert-butyl (4-(4-amino-7-(4-(2-(dimethylamino)ethyl)piperidin-1-yl)pyrrolo[2,1-f][1,2,4]triazin-5-yl)-2-methoxyphenyl)carbamate |
| 110 | tert-butyl (4-(4-amino-7-((((1S,3R)-3-aminocyclopentyl)methyl)amino)pyrrolo[2,1-f][1,2,4]triazin-5-yl)-2-methoxyphenyl)carbamate |
| 111 | tert-butyl (4-(4-amino-7-(((1R,3S)-3-(aminomethyl)cyclopentyl)amino)pyrrolo[2,1-f][1,2,4]triazin-5-yl)-2-methoxyphenyl)carbamate |
| 112 | tert-butyl (4-(4-amino-7-(((1S,3S)-3-(dimethylamino)cyclopentyl)amino)pyrrolo[2,1-f][1,2,4]triazin-5-yl)-2-methoxyphenyl)carbamate |
| 113 | tert-butyl (4-(4-amino-7-(((1S,3R)-3-aminocyclopentyl)amino)pyrrolo[2,1-f][1,2,4]triazin-5-yl)-2-methoxyphenyl)carbamate |
| 114 | tert-butyl (4-(4-amino-7-(((1R,3S)-3-aminocyclopentyl)amino)pyrrolo[2,1-f][1,2,4]triazin-5-yl)-2-methoxyphenyl)carbamate |
| 115 | tert-butyl (4-(4-amino-7-(((1R,3R)-3-aminocyclopentyl)amino)pyrrolo[2,1-f][1,2,4]triazin-5-yl)-2-methoxyphenyl)carbamate |
| 116 | tert-butyl (4-(4-amino-7-(((1S,3S)-3-aminocyclopentyl)amino)pyrrolo[2,1-f][1,2,4]triazin-5-yl)-2-methoxyphenyl)carbamate |
| 117 | tert-butyl (4-(4-amino-7-((piperidin-4-ylmethyl)amino)pyrrolo[2,1-f][1,2,4]triazin-5-yl)-2-methoxyphenyl)carbamate |
| 118 | tert-butyl (4-(4-amino-7-(((1S,3S)-3-aminocyclobutyl)amino)pyrrolo[2,1-f][1,2,4]triazin-5-yl)-2-methoxyphenyl)carbamate |
| 119 | tert-butyl (4-(4-amino-7-(((1R,3R)-3-aminocyclobutyl)amino)pyrrolo[2,1-f][1,2,4]triazin-5-yl)-2-methoxyphenyl)carbamate |
| 120 | tert-butyl (4-(4-amino-7-(piperidin-4-ylamino)pyrrolo[2,1-f][1,2,4]triazin-5-yl)-2-methoxyphenyl)carbamate |
| 121 | tert-butyl (4-(4-amino-7-(((1S,4S)-4-aminocyclohexyl)amino)pyrrolo[2,1-f][1,2,4]triazin-5-yl)-2-methoxyphenyl)carbamate |
| 122 | tert-butyl (4-(4-amino-7-(((1S,4S)-4-aminocyclohexyl)amino)pyrrolo[2,1-f][1,2,4]triazin-5-yl)-2-methoxyphenyl)carbamate |
| 123 | tert-butyl (4-(4-amino-7-(4-(aminomethyl)-4-methylpiperidin-1-yl)pyrrolo[2,1-f][1,2,4]triazin-5-yl)-2-methoxyphenyl)carbamate |
| 124 | tert-butyl (4-(4-amino-6-chloro-7-(4-(dimethylamino)piperidin-1-yl)pyrrolo[2,1-f][1,2,4]triazin-5-yl)-2-methoxyphenyl)carbamate |
| 125 | tert-butyl (4-(4-amino-7-(4-aminopiperidin-1-yl)-6-(trifluoromethyl)pyrrolo[2,1-f][1,2,4]triazin-5-yl)-2-methoxyphenyl)carbamate |
| 126 | tert-butyl (4-(4-amino-7-(4-aminopiperidin-1-yl)-6-iodopyrrolo[2,1-f][1,2,4]triazin-5-yl)-2-methoxyphenyl)carbamate |
| 127 | tert-butyl (4-(4-amino-7-(4-aminopiperidin-1-yl)-6-fluoropyrrolo[2,1-f][1,2,4]triazin-5-yl)-2-methoxyphenyl)carbamate |
| 128 | tert-butyl (4-(4-amino-7-(4-(dimethylamino)piperidin-1-yl)-6-fluoropyrrolo[2,1-f][1,2,4]triazin-5-yl)-2-methoxyphenyl)carbamate |
| 129 | tert-butyl (4-(4-amino-6-fluoro-7-(4-(methylamino)piperidin-1-yl)pyrrolo[2,1-f][1,2,4]triazin-5-yl)-2-methoxyphenyl)carbamate |
| 130 | tert-butyl (4-(4-amino-6-fluoro-7-(2-(4-(methylamino)piperidin-1-yl)ethyl)pyrrolo[2,1-f][1,2,4]triazin-5-yl)-2-methoxyphenyl)carbamate |
| 131 | tert-butyl (4-(4-amino-6-fluoro-7-(4-(2-(methylamino)ethyl)piperidin-1-yl)pyrrolo[2,1-f][1,2,4]triazin-5-yl)-2-methoxyphenyl)carbamate |
| 132 | tert-butyl (4-(1-amino-6-(4-aminopiperidin-1-yl)pyrrolo[1,2-a]pyrazin-8-yl)-2-methoxyphenyl)carbamate |
| 133 | tert-butyl (4-(1-amino-6-(4-(methylamino)piperidin-1-yl)pyrrolo[1,2-a]pyrazin-8-yl)-2-methoxyphenyl)carbamate |
| 134 | tert-butyl (4-(1-amino-6-(4-(dimethylamino)piperidin-1-yl)pyrrolo[1,2-a]pyrazin-8-yl)-2-methoxyphenyl)carbamate |
| 135 | 7-(4-aminopiperidin-1-yl)-5-(4-phenoxyphenyl)pyrrolo[2,1-f][1,2,4]triazin-4-amine |
| 136 | 7-(4-(dimethylamino)piperidin-1-yl)-5-(4-phenoxyphenyl)pyrrolo[2,1-f][1,2,4]triazin-4-amine |
| 137 | N-(4-(4-amino-7-(4-aminopiperidin-1-yl)pyrrolo[2,1-f][1,2,4]triazin-5-yl)-2-methoxyphenyl)furan-2-carboxamide |
| 138 | N-(4-(4-amino-7-(4-aminopiperidin-1-yl)pyrrolo[2,1-f][1,2,4]triazin-5-yl)-2-methoxyphenyl)furan-3-carboxamide |

11. A pharmaceutical composition for preventing or treating YES1-associated disease, comprising the compound or a pharmaceutically acceptable salt thereof of any one of claims 1 to 10.

12. A pharmaceutical composition for preventing or treating Transcriptional Enhanced Associated Domain (TEAD)-associated disease, comprising the compound or a pharmaceutically acceptable salt thereof of any one of claims 1 to 10.

13. A pharmaceutical composition for preventing or treating solid cancer, comprising the compound or a pharmaceutically acceptable salt thereof of any one of claims 1 to 10.

14. The pharmaceutical composition for preventing or treating solid cancer of claim 13, wherein solid cancer is selected from the group consisting of thyroid cancer, bladder cancer, breast cancer, colorectal cancer, small intestine cancer, endometrial cancer, cervical cancer, bone cancer, gastric cancer, lymphoma, bronchial cancer, laryngeal cancer, head and neck cancer, liver cancer, prostate cancer, pancreatic cancer, ovarian cancer, esophageal cancer, kidney cancer, lung cancer, oral cancer, brain cancer, brain tumor, glioma, gallbladder cancer, cholangiocarcinoma, skin cancer, skin melanoma, rectal cancer, colon cancer, uterine cancer, uterine sarcoma, and malignant mesothelioma.
